# EUROPEAN PATENT APPLICATION

(11) **EP 4 450 617 A1**
(43) Date of publication of application: **23.10.2024**
(21) Application number: 21967632.7
(22) Date of filing: 15.12.2021
(51) Int. Cl.: C12N 9/02, C12N 15/53, C12N 15/82, A01H 6/20, A01H 6/54, A01H 5/00, A01G 7/06, A01G 13/00, A01G 22/00

(54) **MUTATED HYDROXYPHENYLPYRUVATE DIOXYGENASE POLYPEPTIDE, AND CODING GENE AND USE THEREOF**

(71) Applicant: Beijing Dabeinong Biotechnology Co., Ltd., Beijing 100194 (CN)
(72) Inventor: XIAO, Xiang, Beijing 100193 (CN); SONG, Qingfang, Beijing 100193 (CN); TAO, Qing, Beijing 100193 (CN); YU, Caihong, Beijing 100193 (CN)
(74) Representative: Nederlandsch Octrooibureau
(86) International application number: PCT/CN2021/138425
(87) International publication number: WO 2023/108495

(57) **Abstract**

The present invention relates to a mutated hydroxyphenylpyruvate dioxygenase (HPPD) polypeptide, and a coding gene and use thereof. The mutated HPPD polypeptide retains the activity of catalyzing the conversion of 4-hydroxyphenylpyruvic acid into homogentisic acid or homogentisate, and has lower sensitive to an HPPD inhibitor herbicide than that to wild type HPPD. An amino acid sequence correspding to the amino acid sequence as shown in SEQ ID NO: 1 comprises amino acid mutation at the following sites: an F372 site is substituted by A, G or V, and an F383 site is substituted by W. The present invention discloses for the first time that the combination mutation at the 372 site and the 383 site of HPPD polypeptides from different species sources can endow plants with synergistic tolerance to HPPD inhibitor herbicides, and the application prospect in plants is wide.

## Description

### FIELD OF THE INVENTION

The present invention relates to a mutant hydroxyphenylpyruvate dioxygenase polypeptide, a coding gene and use thereof, and in particular, to a mutant hydroxyphenylpyruvate dioxygenase polypeptide which is tolerant to HPPD-inhibitor herbicides, a coding gene and use thereof.

### BACKGROUND

The hydroxyphenylpyruvate dioxygenases (abbreviated as HPPDs) are enzymes which, in the presence of iron ion (Fe²⁺) and oxygen, catalyze the reaction in which 4-hydroxyphenylpyruvic acid (abbreviated as HPP), a tyrosine degradation product, is transformed into homogentisic acid / homogentisate (abbreviated as HG), the precursor in plants of tocopherol and plastoquinone (abbreviated as PQ). Tocopherol acts as a membrane-associated antioxidant. PQ not only acts as an electron carrier between PSII and the cytochrome b6/f complex, but also an essential cofactor for the phytoene desaturase involved in the biosynthesis of carotenoids.

Herbicides that act by inhibiting HPPD mainly include three chemical families: triketones, isoxazoles, and pyrazolinates. Inhibition of HPPD blocks the biosynthesis of PQ from tyrosine in plants, thereby resulting in the depletion of PQ and deficiency in carotenoids. HPPD-inhibiting herbicides are plant phloem-mobile bleachers which cause the light-exposed new meristems and leaves to emerge white. Carotenoids are essential for photo-protection. In the absence of carotenoids, the synthesis and function of chlorophyll will be disrupted by UV-radiation and reactive oxygen intermediates, thereby leading to plant growth suppression or even death.

Methods for providing plants that are tolerant to HPPD-inhibitor herbicides have included: 1) overexpressing the HPPD enzyme so as to produce quantities of HPPD enzyme in the plant that are sufficient in relation to HPPD-inhibitor herbicides so as to have enough of the functional enzyme available despite the presence of its inhibitor; and 2) mutating the target HPPD enzyme into a functional HPPD that is less sensitive to herbicides or their active metabolites but retains the capability of transforming into HG. The prior art has reported several mutant HPPD polypeptides which have amino acid mutations at one or more positions relative to the origninal wild-type sequence from which they are derived, and exhibit enhanced tolerance to one or more HPPD-inhibitor herbicides. Moreover, the prior art has further reported that the mutant HPPD polypeptide obtained by combining two amino acid mutations shows higher tolerance to HPPD-inhibitor herbicides than the HPPD polypeptide having either of the two amino acid mutations alone. However, it has not been reported yet that HPPD polypeptides which are derived from a different species and comprise a combination of mutations at positions 372 and 383 of the *Avena sativa* wild-type HPPD polypeptides could enable the plants to exhibit synergistically enhanced tolerance to HPPD-inhibitor herbicides.

### SUMMARY OF THE INVENTION

The object of the present invention is to provide a new mutant hydroxyphenylpyruvate dioxygenase polypeptide, a coding gene and use thereof. The mutant hydroxyphenylpyruvate dioxygenase polypeptide not only has HPPD enzymatic activity, but also enables the plants transformed with genes encoding the mutant hydroxyphenylpyruvate dioxygenase polypeptide to show synergistically enhanced tolerance to HPPD-inhibitor herbicides.

To achieve the above object, the present invention provides a mutant hydroxyphenylpyruvate dioxygenase polypeptide, which retains the activity of catalyzing the reaction of transforming 4-hydroxyphenylpyruvic acid into homogentisic acid or homogentisate and is less sensitive to an HPPD-inhibitor herbicide than the wild-type HPPD, comprising amino acid mutations at the following positions of the amino acid sequence as set forth in SEQ ID NO: 1: substitutions of F with A, G or V at position 372, and with W at position 383.

Preferably, the mutant hydroxyphenylpyruvate dioxygenase polypeptide comprises amino acid mutations at the following positions of the amino acid sequence as set forth in SEQ ID NO: 1: substitution of F with A at position 372, and with W at position 383.

On the basis of the above technical solutions, the mutant hydroxyphenylpyruvate dioxygenase polypeptide can comprise a second mutation.

Preferably, the second mutation comprises at least one of the following amino acid mutation at the positions corresponding to the positions of the amino acid sequence as set forth in SEQ ID NO: 1: A106G, A107 deletion, A111 T, or K351N.

Particularly, the mutant hydroxyphenylpyruvate dioxygenase polypeptide comprises a polypeptide having an amino acid sequence as set forth in SEQ ID NO: 173, SEQ ID NO: 182, SEQ ID NO: 185, SEQ ID NO: 188, SEQ ID NO: 191, SEQ ID NO: 194, SEQ ID NO: 197, SEQ ID NO: 200, or SEQ ID NO: 203.

Further, the mutant hydroxyphenylpyruvate dioxygenase polypeptide is derived from wild-type HPPDs in plants or microorganisms.

Preferably, the plant comprises monocotyledonous plants and dicotyledonous plants; more preferably, the plant is oats (*Avena sativa*), wheat (*Triticum aestivum*), barley (*Hordeum vulgare*), millet (*Setaria italica*), corn (*Zea mays*)*,* sorghum (*Sorghum bicolor*), *Brachypodium distachyon,* rice (*Oryza sativa*), tobacco (*Nicotiana tabacum*), sunflower (*Helianthus annuus*), alfalfa (*Medicago sativa*), soybean (*Glycine max*), *cicer arietinum,* peanut (*Arachis hypogaea*), sugar beet (*Beta vulgaris*), cucumber (*Cucumis sativus*), cotton (*Gossypium hirsutum*), oilseed rape (*Brassica napus*), potato (*Solanum tuberosum*), tomato (*Solanum lycopersicum*) or *Arabidopsis thaliana;*

Preferably, the microorganism is *Pseudomonas fluorescens.*

To achieve the above object, the present invention further provides a polynucleotide encoding the mutant hydroxyphenylpyruvate dioxygenase polypeptide.

To achieve the above object, the present invention further provides an expression cassette or a recombinant vector, comprising the polynucleotide under the regulation of effectively-linked regulatory sequences.

To achieve the above object, the present invention further provides a method for expanding the scope of herbicides to which the plants are tolerant, comprising expressing the mutant hydroxyphenylpyruvate dioxygenase polypeptide together with at least one herbicide-tolerant protein other than the mutant hydroxyphenylpyruvate dioxygenase polypeptide.

Further, the herbicide-tolerant protein is 5-enolpyruvylshikimate-3-phosphate synthase (EPSPS), glyphosate oxidoreductase, glyphosate-N-acetyltransferase, glyphosate decarboxylase, glufosinate acetyltransferase, alpha-ketoglutarate-dependent dioxygenase, dicamba monooxygenase, acetolactate synthase, cytochrome-like protein and/or protoporphyrinogen oxidase.

To achieve the above object, the present invention further provides a method for selecting transformed plant cells, comprising transforming a plurality of plant cells with the polynucleotide, and cultivating the cells under a concentration of the HPPD-inhibitor herbicide that allows the growth of the transformed cells expressing the polynucleotide, while killing the untransformed cells or inhibiting the growth of the untransformed cells.

Preferably, the plant comprises monocotyledonous plants and dicotyledonous plants; more preferably, the plant is oats, wheat, barley, millet, sorghum, *Brachypodium distachyon,* rice, tobacco, sunflower, alfalfa, soybean, *Cicer arietinum,* peanuts, sugar beet, cucumber, cotton, oilseed rape, potato, tomato or *Arabidopsis thaliana;*

Preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of isoxazoles is isoxaflutole, and the HPPD-inhibitor herbicide of triketones is mesotrione; particular preferably, the method for selecting transformed soybean plant cells comprises transforming a plurality of soybean plant cells with the polynucleotide, and cultivating the cells at a concentration of the HPPD-inhibitor herbicide that allows the growth of the transformed cells that express the polynucleotide, and kills the untransformed cells or inhibits the growth of untransformed cells, wherein the HPPD-inhibitor herbicide is topramezone, mesotrione, or isoxaflutole.

To achieve the above object, the present invention further provides a method for controlling weeds, comprising applying an effective dose of the HPPD-inhibitor herbicide to a field planting with a target plant, wherein the target plant contains the polynucleotide;

Preferably, the target plant comprises monocotyledonous plants and dicotyledonous plants; more preferably, the target plant is oats, wheat, barley, millet, sorghum, *Brachypodium distachyon,* rice, tobacco, sunflower, alfalfa, soybean, *Cicer arietinum,* peanut, sugar beet, cucumber, cotton, oilseed rape, potato, tomato or *Arabidopsis thaliana;* further preferably, the target plant is glyphosate-tolerant plant, and the weeds are glyphosate-resistant weeds;

Preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of isoxazoles is isoxaflutole, and the HPPD-inhibitor herbicide of triketones is mesotrione; particular preferably, the method for controlling weeds comprises applying an effective dose of the HPPD-inhibitor herbicide to a field in which a soybean plant is grown, wherein the soybean plant comprises the polynucleotide; wherein the HPPD-inhibitor herbicide is topramezone, mesotrione, or isoxaflutole.

To achieve the above object, the present invention further provides a method for protecting a plant from damages caused by an HPPD-inhibitor herbicide or for conferring tolerance to HPPD-inhibitor herbicide upon a plant, comprising introducing the polynucleotide or the expression cassette or the recombinant vector into a plant to make the post-introduction plant produce a sufficient amount of the mutant hydroxyphenylpyruvate dioxygenase polypeptide to protect the plant from damages caused by the HPPD-inhibitor herbicide.

Preferably, the plant comprises monocotyledonous plants and dicotyledonous plants; more preferably, the plant is oats, wheat, barley, millet, sorghum, *Brachypodium distachyon,* rice, tobacco, sunflower, alfalfa, soybean, *Cicer arietinum,* peanut, sugar beet, cucumber, cotton, oilseed rape, potato, tomato or *Arabidopsis thaliana;*

Preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of isoxazoles is isoxaflutole, and the HPPD-inhibitor herbicide of triketones is mesotrione; particular preferably, the method for protecting a soybean plant from damages caused by an HPPD-inhibitor herbicide or for conferring tolerance to the HPPD-inhibitor herbicide upon a soybean plant comprises introducing the polynucleotide or the expression cassette or the recombinant vector into the soybean plant, resulting in an amount of the mutant hydroxyphenylpyruvate dioxygenase polypeptides that is sufficient to protect the soybean plant into which the polynucleotide or the expression cassette or the recombinant vector has been introduced from the damage of the HPPD-inhibitor herbicide; wherein the HPPD-inhibitor herbicide is topramezone, mesotrione, or isoxaflutole.

To achieve the above object, the present invention further provides a method for producing a plant which is tolerant to an HPPD-inhibitor herbicide, comprising introducing the polynucleotide into the genome of the plant.

Preferably, the introduction method comprises genetic transformation, genome editing or gene mutation methods.

Preferably, the plant comprises monocotyledonous plants and dicotyledonous plants; more preferably, the plant is oats, wheat, barley, millet, sorghum, *Brachypodium distachyon,* rice, tobacco, sunflower, alfalfa, soybean, *Cicer arietinum,* peanut, sugar beet, cucumber, cotton, oilseed rape, potato, tomato or *Arabidopsis thaliana;*

Preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of isoxazoles is isoxaflutole, and the HPPD-inhibitor herbicide of triketones is mesotrione; particular preferably, the method for generating soybean plants that are tolerant to the HPPD-inhibitor herbicide comprises introducing the polynucleotide into the genome of the soybean plants; wherein the HPPD-inhibitor herbicide is topramezone, mesotrione, or isoxaflutole.

To achieve the above object, the present invention further provides a method for cultivating a plant which is tolerant to an HPPD-inhibitor herbicide, comprising:
planting at least one plant propagule, the genome of which contains the polynucleotide;
growing the plant propagule into a plant; and
applying an effective dose of the HPPD-inhibitor herbicide to a plant growing environment comprising at least the plant, and harvesting the plant having a reduced plant damage and/or increased plant yield compared to other plants without the polynucleotide;

Preferably, the plant comprises monocotyledonous plants and dicotyledonous plants; more preferably, the plant is oats, wheat, barley, millet, sorghum, *Brachypodium distachyon,* rice, tobacco, sunflower, alfalfa, soybean, *Cicer arietinum,* peanut, sugar beet, cucumber, cotton, oilseed rape, potato, tomato or *Arabidopsis thaliana;*

Preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of isoxazoles is isoxaflutole, and the HPPD-inhibitor herbicide of triketones is mesotrione; particular preferably, the method for cultivating a soybean plant which is tolerant to the HPPD-inhibitor herbicide comprises planting at least one soybean plant seed, wherein the soybean plant seed comprises in its genome the polynucleotide; allowing the soybean plant seed to grow into a soybean plant; applying an effective dose of the HPPD-inhibitor herbicide to a plant growing environment comprising at least the soybean plant, and harvesting the soybean plant with reduced plant damage and/or increased plant yield compared to other soybean plants which do not comprise the polynucleotide; wherein the HPPD-inhibitor herbicide is topramezone, mesotrione, or isoxaflutole.

The present invention also provides a method for obtaining a processed agricultural product, comprising treating harvested product of the HPPD-inhibitor herbicide-tolerant plant obtained by the method to obtain the processed agricultural product.

To achieve the above object, the present invention further provides a planting system for controlling the growth of weeds, comprising an HPPD-inhibitor herbicide and a plant growing environment in which at least one target plant is present, wherein the target plant contains the polynucleotide.

Preferably, the target plant comprises monocotyledonous plants and dicotyledonous plants; more preferably, the target plant is oats, wheat, barley, millet, sorghum, *Brachypodium distachyon,* rice, tobacco, sunflower, alfalfa, soybean, *Cicer arietinum,* peanut, sugar beet, cucumber, cotton, oilseed rape, potato, tomato or *Arabidopsis thaliana;* further preferably, the target plant is a glyphosate-tolerant plant, and the weeds are glyphosate-resistant weeds.

Preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of isoxazoles is isoxaflutole, and the HPPD-inhibitor herbicide of triketones is mesotrione; particular preferably, the planting system for controlling the growth of weeds comprises an HPPD-inhibitor herbicide and a plant growing environment in which at least one soybean plant is present, wherein the soybean plant contains the polynucleotide, and the HPPD-inhibitor herbicide is topramezone, isoxaflutole or mesotrione.

To achieve the above object, the present invention further provides use of the mutant hydroxyphenylpyruvate dioxygenase polypeptide for conferring tolerance to an HPPD-inhibitor herbicide upon a plant.

Preferably, the plant comprises monocotyledonous plants and dicotyledonous plants; more preferably, the plant is oats, wheat, barley, millet, sorghum, *Brachypodium distachyon,* rice, tobacco, sunflower, alfalfa, soybean, *Cicer arietinum,* peanut, sugar beet, cucumber, cotton, oilseed rape, potato, tomato or *Arabidopsis thaliana;*

Preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of isoxazoles is isoxaflutole, and the HPPD-inhibitor herbicide of triketones is mesotrione; particular preferably, use of the mutant hydroxyphenylpyruvate dioxygenase polypeptide for conferring tolerance to an HPPD-inhibitor herbicide upon a soybean plant, wherein the HPPD-inhibitor herbicide is topramezone, isoxaflutole, or mesotrione.

The article "a" and "an" as used herein refers to one or more than one (i.e., to at least one). For example, "an element" means one or more elements (components). Furthermore, the term "comprise" or variants thereof such as "comprises" or "comprising" should be understood to imply the inclusion of a stated element, integer or step, or group of elements, integers or steps, but not the exclusion of any other element, integer or step, or group of elements, integers or steps.

Within the context of the present invention, the terms "hydroxy phenyl pyruvate dioxygenase (HPPD)", "4-hydroxy phenyl pyruvate dioxygenase (4-HPPD)" and "p-hydroxy phenyl pyruvate dioxygenase (p-HPPD)" are synonymous.

The term "HPPD-inhibitor herbicide" refers to herbicides that act either directly or indirectly to inhibit HPPD, where the herbicides are bleachers. Most commercially available HPPD-inhibitor herbicides belong to one of the three chemical families as listed below: (1) Triketones, e.g. sulcotrione (i.e. 2-[2-chloro-4-(methylsulfonyl)benzoyl]-1 ,3-cyclohexane- -dione), mesotrione (i.e. 2-[4-(methylsulfonyl)-2-nitrobenzoyl]-1 ,3-cyclohexanedione); tembotrione (i.e. 2-[2-chloro-4-(methylsulfonyl)-3-[(2,2,2-trifluoroethoxy)methyl]benzoyl]- -1 ,3-cyclohexanedione); (2) Isoxazoles, e.g. isoxaflutole (i.e. (5-cyclopropyl-4-isoxazolyl)[2--(methylsulfonyl)-4-(trifluoromethyl)phenyl]methanone); (3) Pyrazolinates, e.g. topramezone (i.e., [3-(4,5-dihydro-3-isoxazolyl)-2-methyl-4-(methylsulfonyl)phenyl] (5-hydroxy-1-methyl- -pyrazol-4-yl)methanone), pyrasulfotole (i.e., (5-hydroxy-1 ,3-dimethylpyrazol-4-yl)(2-methyl- - sulfonyl-4-(trifluoromethylphenyl)methanone).

As used herein, topramezone (also known as BAS-670H) refers to [3-(4,5-dihydro-3-isoxazolyl) -2-methyl-4-(methylsulfonyl)phenyl] (5-hydroxy-1-methyl-pyrazol-4-yl)methanone as a white crystalline solid. It is a systemic conduction-type HPPD-inhibitor herbicide of pyrazolinates for the post-emergence treatment of stems and leaves in a typical dosage form of 30% suspension concentrate. Commercial formulations of topramezone (such as Topramezone SC) can be used for the control of gramineous and broad-leaf weeds, at 5.6-6.7 g per acre. The weeds that can be effectively controlled include but are not limited to, Digitaria sanguinalis (Calathodes oxycarpa), Barnyard grass, Eleusine indica Gaertn, Eriochloa villosa, Setaria viridis (Giant foxtail), Chenopodium album, Polygonaceae, Abutilon avicennae, Abutilon theophrasti, Pigweeds, Portulaca oleracea, Xanthium strumarium, and Solanum nigrum. Topramezone SC combined with Atrazine can result in a significantly enhanced effect. Apart from the excellent efficacy on the aforementioned weeds, topramezone can also have good controlling effects on malignant broad-leaf weeds, such as Cephalanoplos segetum Kitam (Cirsium segestum), Sonchus arvensis, Acalypha australis, and Commelina communis (Asiatic dayflower), and in particular it can effectively control Setaria viridis, Digitaria sanguinalis, Eleusine indica Gaertn, and Eriochloa villosa which are difficult to be controlled by mesotrione.

As used herein, the "effective dose" of topramezone means being used at an amount ranging from 25 to 200 g ai/ha, including from 25 to 50 g ai/ha, from 50 to 100 g ai/ha, from 100 to 150 g ai/ha, or from 150 to 200 g ai/ha.

As used herein, isoxaflutole refers to 5-cyclopropyl-4-isoxazolyl)[2-(methylsulfonyl)-4-(trifluoromethyl)phenyl]methanone as a white to pale-yellow solid. It is a selective systemic pre-emergence HPPD-inhibitor herbicide of organic heterocyclic isoxazoles and predominantly works by absorption and translocation via the young weed roots. Isoxaflutole is mainly useful for controlling various annual broad-leaf weeds, such as Abutilon theophrasti, Chenopodium album, Kochia scoparia, Salsola arbuscula, Solanum nigrum, Amaranthus retroflexus, Polygonum bungeanum, Bidens pilosa, Portulaca oleracea, Chickweed, Elsholtzia, Xanthium strumarium, Acalypha australis, Amethystea caerulea, Polygonum Lapathifolium, and Veronica polita, in fields of dryland crops, and also has good controlling efficacy on some annually gramineous weeds, such as Digitaria sanguinalis, Barnyard grass, Eleusine indica Gaertn, Leptochloa chinensis, Setaria faberi, and Setaria viridis.

As used herein, the "effective dose" of isoxaflutole means being used at an amount ranging from 35 to 280 g ai/ha, including from 35 to 70 g ai/ha, from 70 to 140 g ai/ha, from 140 to 200 g ai/ha, or from 200 to 280 g ai/ha.

As used herein, the "mesotrione" refers to 2-[4-(methylsulfonyl)-2-nitrobenzoyl]-1,3-cyclohexanedione as a brown or light yellow solid. It is a selective systemic conduction-type HPPD-inhibitor herbicide of triketones that provides both pre- and post-emergence weed control in plants. It can be absorbed by plants through the leaves and roots, and be translocated downward from top to bottom parts, resulting in chlorosis (yellowing) symptom of the meristems followed by necrosis (dead tissue) 3 to 5 days after herbicide application, and further death of the entire plants. Mesotrione is useful for pre- and post-emergence control of annual broad-leaf weeds and gramineous weeds in plants; wherein the annual broad-leaf weeds that can be controlled mainly comprise Xanthium strumarium, Abutilon theophrasti, Chenopodium album, amaranth, Polygonaceae, Solanum nigrum, and Ambrosia trifida; and the gramineous weeds that can be controlled mainly comprise young barnyard grass, Digitaria sanguinalis, Setaria viridis, and Brachiaria decumbens.

As used herein, the "effective dose" of mesotrione means being used at an amount ranging from 52.5 to 420 g ai/ha, including from 52.5 to 105 g ai/ha, from 105 to 210 g ai/ha, from 210 to 300 g ai/ha, or from 300 to 420 g ai/ha.

As used herein, the term "resistance" is inheritable and allows a plant to grow and propagate under the circumstance where an effective treatment with an ordinary herbicide is performed on a given plant. As recognized by a person skilled in the art, even if there is certain degree of damage (such as small necrosis, dissolution, chlorosis or other damage) to the given plant treated with the herbicide, at least the yield is not significantly compromised and thus the plant can still be considered as "resistant". In other words, the given plant has increased ability to resist various degrees of damage induced by the herbicide, and in general, damage to a wild-type plant with the same genotype can be caused at the same dose of the herbicide. The term "tolerant" or "tolerance" in the present invention is more extensive than the term "resistance" and includes "resistance".

As used herein, the term "confer" refers to providing a characteristic or trait, such as herbicide tolerance and/or other desirable traits to a plant.

As used herein, the term "heterologous" means from another source. In the context of DNA, "heterologous" refers to any foreign "non-self" DNA including that from another plant of the same species. For example, in the present invention a soybean HPPD gene that can be expressed in a soybean plant by means of transgenic methods would still be considered as "heterologous" DNA.

As used herein, the term "nucleic acid" includes a deoxyribonucleotide or ribonucleotide polymer in either single- or double-stranded forms, and unless otherwise specified, encompasses known analogues (e.g., peptide nucleic acids) having the essential properties of wild-type nucleotides in that they hybridize to single-stranded nucleic acids in a manner similar to wild-type nucleotides.

As used herein, the term "encoding" or "encoded" when used in the context of a specified nucleic acid means that the nucleic acid comprises the requisite information to direct translation of the nucleotide sequence into a specified protein. The information by which a protein is encoded is specified by the use of codons. A nucleic acid encoding a protein may comprise non-translated sequences (e.g., introns) within translated regions of the nucleic acid or may lack such intervening non-translated sequences (e.g., as in cDNA).

The proteins of the invention may be altered in various ways including amino acid substitutions, deletions, truncations, and insertions. For example, amino acid sequence variants and fragments of the mutant HPPD proteins can be prepared by mutations in the DNA. Methods for the induction of polynucleotide mutations are well-known in the art. See, for example, Kunkel (1985) Proc. Natl. Acad. Sci. USA 82:488-492; Kunkel et al. (1987) Methods in Enzymol. 154:367-382; U.S. Pat. No. 4,873,192; Walker and Gaastra, eds. (1983) Techniques in Molecular Biology (MacMillan Publishing Company, New York) and the references cited therein. Guidance as to appropriate amino acid substitutions that often do not affect biological activity of the protein of interest may be found in the model of Dayhoff et al. (1978) Atlas of Protein Sequence and Structure (Natl. Biomed.Res. Found., Washington, D.C.). Conservative substitutions, such as exchanging one amino acid with another having similar properties, may be optimal.

As described herein, the mutant HPPD polypeptides or variants and fragments thereof possess HPPD enzymatic activity and confer tolerance to certain classes of HPPD-inhibitor herbicides upon plants. The mutant HPPD polypeptides have amino acid changes at one or more positions relative to the starting wild-type sequence from which they are derived, and exhibit enhanced tolerance to one or more HPPD-inhibitor herbicides. HPPD enzymes that exhibit enhanced tolerance to at least one HPPD-inhibitor herbicide may do so by virtue of exhibiting, relative to the like unmutated starting enzyme.

DNA sequences encoding such mutant HPPD polypeptides are used in the provision of plants, plant cells and seeds of the present invention that offer enhanced tolerance to one or more HPPD-inhibitor herbicides compared with like plants likewise expressing the unmutated starting enzyme.

Plant HPPD genes encoding such mutant HPPD polypeptides are useful for generating plants tolerant to HPPD-inhibitor herbicides. Plant HPPD genes so modified are particularly suitable for expression in plants to confer herbicide tolerance upon plants.

Many HPPD sequences are known in the art and can be used to generate mutant HPPD sequences by making substitutions, deletions, and/or additions in the corresponding amino acids. The positions 372 and 383 of the present invention are calculated using the amino acid position 372 or 383 of the *Avena sativa* wild-type HPPD amino acid sequence set forth in SEQ ID NO: 1 as the standard. The mutant HPPD polypeptide according to the present invention comprises a combinatorial mutation at the amino acids positions corresponding to positions 372 and 383 of SEQ ID NO: 1, wherein the combinatorial mutation comprises substitutions of F with A, G or V at position 372, and substitution of F with W at position 383; preferably, the mutation comprises substitution of F with A at position 372, and substitution of F with Wat position 383. Thus, a known or suspected HPPD sequence can be aligned with the amino acid sequence as set forth in SEQ ID NO: 1 using standard sequence alignment tools, and the substitutions or deletions in the corresponding amino acids with respect to the amino acid sequence as set forth in SEQ ID NO: 1 as described herein can be made in the known or suspected HPPD sequence.

The present invention comprise a mutant HPPD polypeptide which is derived from HPPDs in plants or microorganisms, has HPPD enzymatic activity, and comprises at least a combinatorial mutation at positions 372 and 383 of the amino acid sequence as set forth in SEQ ID NO: 1, optionally further in combination with a mutation at other position (the corresponding position present in the HPPD polypeptide), for example, in combination with one or more mutations at the following corresponding positions: A106G, A107 deletion, A111T, or K351N in the amino acid sequence of the HPPD from *Avena sativa.* In the various embodiments described above, the combinatorial mutation at positions 372 and 383 may be F372A+F383W, F372G+F383W or F372V+F383W. The combinatorial mutation of the combinatorial mutation at positions 372 and 383 and a mutation at other position comprises F372A+F383W+A107 deletion, F372A+F383W+ A111T, F372A+F383W+A106G, F372A+F383W+A106G+K351N, F372A+F383W+A107 deletion + K351N, or F372A+F383W+A111T + K351N. Further, the present invention comprise a mutant HPPD polypeptide which is derived from HPPDs (by substitutions, deletions and/or additions) in plants or microorganisms of different species or different ecotypes within the same species. Exemplary HPPDs from different ecotypes within the same species include, but are not limited to, HPPDs from the ecotypes within the following species: HPPDs from the different ecotypes of alfalfa with the Accession Number: XP_003617391.2, AAX59006.1, XP_003617384.1, XP_013466115.1, AET00342.2, or A0A396HWH5; HPPDs from the different ecotypes of cotton with the Accession Number: A0A0D2PWQ6, A0A2P5SI66, A0A0D2LWN1, or A0A0D2N7F6; HPPDs from the different ecotypes of oilseed rape with the Accession Number: VDC64417.1, CDY10210.1, AFB74208.1, XP_013695640.1, XP_013695641.1, RID40406.1, RID48932.1, XP_009118533.1, XP_009119049.1, XP_013723237.1, AFB74218.1, or AFB74207.1; HPPDs from different ecotypes of soybean with the Accession Number: A5Z1N7, I1M6Z4, A0A088MGH9, or I1 M6Z5; HPPDs from different ecotypes of tobacco with the Accession Number: XP_009770088.1, or XP_009587203.1; HPPDs from different ecotypes of rice with the Accession Number: A3A3J1, B8AIH6, or A0A0E0G1W2; HPPDs from different ecotypes of barley with the Accession Number: BAJ86732.1, BAJ95714.1, or F2E412 (these Accession Numbers are available at GenBank Database or UniProt Knowledgebase Database).

The term "position 372", "372 position", or "372 single position" not only in a narrow sense refers to the amino acid (phenylalanine) at position 372 of the amino acid sequence as set forth in SEQ ID NO: 1, but also in a broad sense comprises a position corresponding to the amino acid at position 372 of the amino acid sequence as set forth in SEQ ID NO: 1 obtained in a known or suspected HPPD amino acid sequence which can be aligned with the amino acid sequence as set forth in SEQ ID NO: 1 using standard sequence alignment tools (such as CLUSTAL software), which might not be the position 372 of the amino acid sequence of that HPPD.

The term "position 383", "383 position", or "383 single position" not only in a narrow sense refers to the amino acid (phenylalanine) at position 383 of the amino acid sequence as set forth in SEQ ID NO: 1, but also in a broad sense comprises a position corresponding to the amino acid at position 383 of the amino acid sequence as set forth in SEQ ID NO: 1 obtained in a known or suspected HPPD amino acid sequence which can be aligned with the amino acid sequence as set forth in SEQ ID NO: 1 using standard sequence alignment tools (such as CLUSTAL software), which might not be the position 383 of the amino acid sequence of that HPPD.

The term "position 415", "415 position", or "415 single position" not only in a narrow sense refers to the amino acid (phenylalanine) at position 415 of the amino acid sequence as set forth in SEQ ID NO: 1, but also in a broad sense comprises a position corresponding to the amino acid at position 415 of the amino acid sequence as set forth in SEQ ID NO: 1 obtained in a known or suspected HPPD amino acid sequence which can be aligned with the amino acid sequence as set forth in SEQ ID NO: 1 using standard sequence alignment tools (such as CLUSTAL software), which might not be the position 415 of the amino acid sequence of that HPPD.

Similarly, the above interpretation on "position 372" or "372 position" also applies to those on the other positions.

The term "corresponding to" refers to a position corresponding to an amino acid at a particular position of the amino acid sequence as set forth in SEQ ID NO: 1 obtained by aligning an HPPD amino acid sequence which is derived from a different species or different ecotype within the same species, with the amino acid sequence as set forth in SEQ ID NO: 1 using standard sequence alignment tools, such as a position corresponding to the amino acid at position 372 or 383 of the amino acid sequence as set forth in SEQ ID NO: 1.

The terms "polypeptide", "peptide", and "protein" are used interchangeably herein and refer to a polymer of amino acid residues. The terms apply to amino acid polymers in which one or more amino acid residues are an artificial chemical analogue of a corresponding wild-type amino acid, as well as to wild-type amino acid polymers. Polypeptides of the invention can be produced either from a nucleic acid disclosed herein, or by means of standard molecular biology techniques. For example, a truncated protein of the invention can be produced by expression of a recombinant nucleic acid of the invention in an appropriate host cell, or alternatively by a combination of ex *vivo* procedures, such as protease digestion and purification.

Accordingly, the present invention also provides nucleic acid molecules comprising polynucleotide sequences that encode mutant HPPD polypeptides that have enzymatic activity of HPPD and that confer tolerance in plants to certain classes of herbicides that inhibit HPPD, and variants and fragments thereof. In general, the invention includes any polynucleotide sequence that encodes any of the mutant HPPD polypeptides described herein, as well as any polynucleotide sequence that encodes HPPD polypeptides having one or more conservative amino acid substitutions relative to the mutant HHPD polypeptides described herein. Conservative substitutions providing functionally similar amino acids are well-known in the art. The following five groups each contain amino acids that are conservative substitutions for one another: Aliphatic: Glycine (G), Alanine (A), Valine (V), Leucine (L), Isoleucine (I); Aromatic: Phenylalanine (F), Tyrosine (Y), Tryptophan (W); Sulfur-containing: Methionine (M), Cysteine (C); Basic: Arginine (I), Lysine (K), Histidine (H); Acidic: Aspartic acid (D), Glutamic acid (E), Asparagine (N), Glutamine (Q).

In one embodiment, the present invention provides a polynucleotide sequence encoding an amino acid sequence derived from HPPDs in plants or microorganisms, where the polypeptide has HPPD enzymatic activity and comprises at least a combinatorial mutation at the positions corresponding to the amino acids at positions 372 and 383 of SEQ ID NO: 1.

Accordingly, sequences which have tolerance activity to HPPD-inhibitor herbicides and hybridize to genes encoding the mutant HPPD polypeptides of the invention are included within the present invention. Exemplary sequences comprise at least about 75%, 80%, 85%, 90%, 91%, 92%, 93%, 94%, 95%, 96%, 97%, 98%, 99% or more sequence identity to SEQ ID NO: 11-12, SEQ ID NO: 33-34, SEQ ID NO: 45-46, SEQ ID NO: 57-58, SEQ ID NO: 69-70, SEQ ID NO: 81-82, SEQ ID NO: 93-94, SEQ ID NO: 105-106, SEQ ID NO: 117-118, SEQ ID NO: 129-130, SEQ ID NO: 141-142, SEQ ID NO: 153-154, SEQ ID NO: 159-160, SEQ ID NO: 165-166, SEQ ID NO: 174-175, SEQ ID NO: 183-184, SEQ ID NO: 186-187, SEQ ID NO: 189-190, SEQ ID NO: 192-193, SEQ ID NO: 195-196, SEQ ID NO: 198-199, SEQ ID NO: 201-202, and SEQ ID NO: 204-205 of the invention.

Any conventional nucleic acid hybridization or amplification method can be used to identify the presence of the mutant HPPD gene of the present invention. A nucleic acid molecule or a fragment thereof is capable of specifically hybridizing to other nucleic acid molecules under certain circumstances. In the present invention, if two nucleic acid molecules can form an anti-parallel double-stranded nucleic acid structure, then it can be considered that these two nucleic acid molecules can be specifically hybridized to each other. If two nucleic acid molecules exhibit a complete complementarity, then one nucleic acid molecule of the two is said to be the "complement" of the other nucleic acid molecule. In the present invention, when each nucleotide of a nucleic acid molecule is complementary to the corresponding nucleotide of another nucleic acid molecule, then these two nucleic acid molecules are said to exhibit a "complete complementarity". If two nucleic acid molecules can be hybridized to each other with a sufficient stability to allow them to anneal and bind with each other at least under conventional "low stringency" conditions, then these two nucleic acid molecules are said to be "minimally complementary". Similarly, if two nucleic acid molecules can be hybridized to each other with a sufficient stability to allow them to anneal and bind with each other under conventional "high stringency" conditions, then these two nucleic acid molecules are said to be "complementary". Deviation from a complete complementarity is permissible, as long as this deviation does not completely prevent two molecules from forming a double-stranded structure. In order to enable a nucleic acid molecule to act as a primer or probe, it is only guaranteed that the molecule has a sufficient complementarity in its sequence to allow a stable double-stranded structure to be formed under the conditions of particular solvent and salt concentration.

In the present invention, a substantially homologous sequence is a nucleic acid molecule that can be specifically hybridized to the complementary strand of a matched nucleic acid molecule under high stringency conditions. Suitable stringent conditions that promote DNA hybridization are well-known to a person skilled in the art; for example, the suitable stringent conditions can be achieved by treating with 6.0× sodium chloride/sodium citrate (SSC) under conditions of approximately 45 °C, and then washing with 2.0×SSC under conditions of 50 °C. For example, the salt concentration in the washing step can be selected from the low stringency condition of about 2.0×SSC and 50 °C to the high stringency condition of about 0.2×SSC and 50 °C. In addition, the temperature condition in the washing step can rise from the low stringency condition of room temperature (about 22 °C) to the high stringency condition of about 65 °C. The temperature condition and the salt concentration can both vary, and it is also possible that one of the two remains unchanged, while the other varies. Preferably, the stringent conditions in the present invention can be achieved by specifically hybridizing to the mutant HPPD gene of the present invention in a 6×SSC, 0.5% SDS solution at 65 °C, and then washing the membrane each once with 2×SSC, 0.1% SDS and 1×SSC, 0.1% SDS.

As used herein, the term "hybridizing" or "hybridizing specifically" refers to the binding, duplexing, or hybridizing of a molecule only to a particular nucleotide sequence under stringent conditions when that sequence is present in a complex mixture (e.g., total cellular) DNA or RNA.

Because of the degeneracy of the genetic codon, a variety of different DNA sequences may encode the same amino acid sequence. It is within the skill of a person skilled in the art to produce these alternative DNA sequences encoding the same or substantially the same protein. These different DNA sequences are included in the scope of the present invention. The "substantially the same" sequence refers to a sequence with an amino acid substitution, deletion, addition or insertion that does not substantively affect the herbicide tolerance activity, and includes a fragment retaining the herbicide tolerance activity.

The term "functional activity" or "activity" in the present invention means that the protein/enzyme used in the present invention (alone or in combination with other proteins) has the ability to degrade an herbicide or diminish the herbicide activity. A plant producing the protein of the present invention preferably produces an "effective amount" of the protein, so that when treating the plant with an herbicide, the protein expression level is sufficient to confer the plant a complete or partial tolerance to the herbicide (unless otherwise specified, in a general amount). The herbicide can be used in an amount which would usually kill a target plant or in a normal field amount and concentration. Preferably, the plant cell and plant of the present invention are protected from growth suppression or damage caused by treatment with the herbicide. The transformed plant and plant cell of the present invention are preferably tolerant to HPPD-inhibitor herbicides, that is, the transformed plant and plant cell can grow in the presence of an effective dose of HPPD-inhibitor herbicides.

The gene and protein in the present invention not only comprise a specific exemplary sequence, but also comprise a portion and/or a fragment (including an internal deletion and/or terminal deletion compared to the full-length protein), a variant, a mutant, a variant protein, a substitute (a protein having substituted amino acids), a chimera and a fusion protein which retain the HPPD-inhibitor herbicide tolerance activity characteristic of the specific exemplary protein.

The term "variant" in the present invention is intended to mean substantially similar sequences. For polynucleotides, a variant comprises a deletion and/or addition of one or more nucleotides at one or more internal sites within the reference polynucleotide and/or a substitution of one or more nucleotides at one or more sites in the mutant HPPD polynucleotide. As used herein, the term "reference polynucleotide or polypeptide" comprises a mutant HPPD nucleotide sequence or amino acid sequence, respectively. As used herein, the term "wild-type polynucleotide or polypeptide" comprises a wild-type nucleotide sequence or amino acid sequence, respectively. For polynucleotides, conservative variants include those sequences that, because of the degeneracy of the genetic code, encode the amino acid sequence of one of the mutant HPPD polypeptides of the invention. Wild-type allelic variants such as these can be identified with the use of well-known molecular biology techniques, for example, with polymerase chain reaction (PCR) and hybridization techniques as outlined below. Variant polynucleotides also include synthetically derived polynucleotide, such as those generated, for example, by using site-directed mutagenesis but which still encode a mutant HPPD protein of the invention. Generally, variants of a particular polynucleotide of the invention will have at least about 75%, 80%, 85%, 90%, 91%, 92%, 93%, 94%, 95%, 96%, 97%, 98%, 99% or more sequence identity to that particular polynucleotide as determined by sequence alignment programs and parameters.

"Variant protein" in the present invention is intended to mean a protein derived from the reference protein by deletion or addition of one or more amino acids at one or more internal sites in the mutant HPPD protein and/or substitution of one or more amino acids at one or more sites in the mutant HPPD protein. Variant proteins encompassed by the present invention are biologically active, that is they continue to possess the desired biological activity of the mutant HPPD protein, that is, HPPD enzymatic activity and/or herbicide tolerance as described herein. Such variants may result from, for example, genetic polymorphism or from human manipulation. Biologically active variants of a mutant HPPD protein of the invention will have at least about 85%, 90%, 91%, 92%, 93%, 94%, 95%, 96%, 97%, 98%, 99% or more sequence identity across the entirety of the amino acid sequence for the mutant HPPD protein as determined by sequence alignment programs and parameters. A biologically active variant of a protein of the invention may differ from that protein by as few as 1-15 amino acid residues, as few as 1-10, such as 6-10, as few as 5, as few as 4, 3, 2, or even 1, amino acid residue.

Methods of alignment of sequences are well-known in the art and can be accomplished using mathematical algorithms such as the algorithm of Myers and Miller (1988) CABIOS 4:11-17; the local alignment algorithm of Smith et al. (1981) Adv. Appl. Math. 2:482; the global alignment algorithm of Needleman and Wunsch (1970) J. Mol. Biol. 48:443-453; and the algorithm of Karlin and Altschul (1990) Proc. Natl. Acad. Sci. USA 872264, modified as in Karlin and Altschul (1993) Proc. Natl. Acad. Sci. USA 90:5873-5877. Computer implementations of these mathematical algorithms can be utilized for comparison of sequences to determine sequence identity. Such implementations include, but are not limited to: CLUSTAL in the PC/Gene program (available from Intelligenetics, Mountain View, California); the ALIGN program (Version 2.0) and GAP, BESTFIT, BLAST, FASTA, and TFASTA in the GCG Wisconsin Genetics Software Package, Version 10 (available from Accelrys Inc., 9685 Scranton Road, San Diego, California, USA).

In certain examples, the amino acids encoding mutant HPPD polypeptides or variants thereof that retain HPPD enzymatic activity can be stacked with any combination of polynucleotide sequences of interest in order to create plants with a desired trait. The term "trait" refers to the phenotype derived from a particular sequence or groups of sequences. For example, the amino acids/polynucleotides encoding a mutant HPPD polypeptide or variant thereof that retains HPPD enzymatic activity may be stacked with any other polynucleotides encoding polypeptides that confer a desirable trait, including but not limited to resistance to diseases, insects, and herbicides, tolerance to heat and drought, reduced time to crop maturity, improved industrial processing, such as for the conversion of starch or biomass to fermentable sugars, and improved agronomic quality, such as high oil content and high protein content.

It is well-known for a person skilled in the art that the benefits of a combination of two or more modes of action in improving the spectrum of weeds controlled and/or the control of wild-type more tolerant species or resistant weed species, can also be extended to chemicals for which herbicide tolerance was enabled in crops through artificial methods (either transgenically or non-transgenically) beyond HPPD tolerant crops. In fact, the traits encoding the following resistances can be superposed alone or in multiple combinations to provide the ability to effectively control or prevent weed shifts to herbicides: glyphosate resistance (such as EPSPS, GOX, and GAT from resistant plants or bacteria), glufosinate resistance (such as PAT and Bar), herbicide resistance to acetolactate synthase (ALS) inhibitors (such as imidazolinones, sulfonyl urea, triazole pyrimidines, sulfonated aniline, pyrimidinyl thiobenzoic acids and other chemicals resistant genes, e.g., AHAS, CsrI, and SurA), phenoxyauxin herbicide resistance (such as aryloxyalkanoate dioxygenase-12 (AAD-12)), dicamba herbicide resistance (such as dicamba monooxygenase (DMO)), bromoxynil resistance (such as Bxn), phytoene desaturase (PDS) inhibitor resistance, herbicide resistance to photosystem II inhibitors (such as psbA), herbicide resistance to photosystem I inhibitors, herbicide resistance to protoporphyrinogen oxidase IX (PPO) inhibitors (such as PPO-1), phenylurea herbicide resistance (such as CYP76B1), and dichloromethoxybenzoic acid degrading enzymes.

Glyphosate is widely used, as it controls a very broad spectrum of broad-leaf and gramineous weed species. However, repeat use of glyphosate in glyphosate-tolerant crop and non-crop applications has (and will continue to) selected for weed shifts to wild-type more tolerant species or glyphosate resistant biotypes. Most herbicide resistance management programs suggest using an effective dose of tank-mixed herbicide partners as a means for delaying the emergence of resistant weeds, wherein the herbicide partners provide control for the same species, but have different modes of action. Superposing the gene encoding the mutant HPPD polypeptide of the present invention with a glyphosate tolerance trait (and/or other herbicide tolerance traits) can achieve the control of glyphosate resistant weed species (broad-leaf weed species controlled by one or more HPPD-inhibitor herbicides) in glyphosate tolerant crops by enabling the selective use of glyphosate and HPPD-inhibitor herbicides (such as topramezone, mesotrione, or isoxaflutole) in the same crop. The applications of these herbicides can be performed simultaneously in a tank mixture containing two or more herbicides with different modes of action, or can be performed alone in a single herbicide composition in sequential applications (e.g., before planting, or before or after emergence) (with the interval time of applications ranging from 2 hours to 3 months); or alternatively, the applications of these herbicides can be performed by using a combination of any number of herbicides representative of each applicable compound category at any time (from 7 months after planting a crop to the time when the crop is harvested (or the pre-harvest interval for a single herbicide, wherein the shortest is taken)).

The flexibility in controlling broad-leaf weeds is very important, in terms of the application time, application amount of single herbicide, and abilities to control the stubborn or resistant weeds. The application range of glyphosate superposed with a glyphosate resistant gene/mutant HPPD gene in crops can be from 250 to 2500 g ae/ha. The application range of HPPD-inhibitor herbicides (one or more) can be from 25 to 500 g ai/ha. The optimal combination of time for these applications depends on the specific conditions, species and environments.

An herbicide formulation (e.g., an ester, acid or salt-formulation, or soluble concentrate, emulsifiable concentrate or soluble liquid) and a tank mix additive (e.g., an adjuvant or compatilizer) can significantly affect the weed control of a given herbicide or a combination of one or more herbicides. Any chemical combination of any of the foregoing herbicides is within the scope of the present invention.

In addition, the gene encoding the mutant HPPD polypeptide of the present invention alone or being stacked with other characteristics of herbicide resistant crops can be stacked with one or more other input traits (for example, insect resistance, fungal resistance or stress tolerance, etc.) or output traits (for example, increased yield, improved oil amount, increased fiber quality, etc.). Therefore, the present invention can be used to provide complete agricultural solutions for improving the qualities of crops with the abilities for flexibly and economically controlling any number of agriculture pests.

These stacked combinations can be created by any method including, but not limited to, cross-breeding plants by any conventional or TopCross methodology, or genetic transformation. If the sequences are stacked by genetically transforming the plants, the polynucleotide sequences of interest can be combined at any time and in any order. For example, a transgenic plant comprising one or more desired traits can be used as the target to introduce further traits by subsequent transformation. The traits can be introduced simultaneously in a co-transformation protocol with the polynucleotides of interest provided by any combination of transformation cassettes. For example, if two sequences will be introduced, the two sequences can be contained in separate transformation cassettes (trans) or contained on the same transformation cassette (cis). Expression of the sequences can be driven by the same promoter or by different promoters. In certain cases, it may be desirable to introduce a transformation cassette that will suppress the expression of the polynucleotide of interest. This may be combined with any combination of other suppression cassettes or overexpression cassettes to generate the desired combination of traits in the plant. It is further recognized that polynucleotide sequences can be stacked at a desired genomic location using a site-specific recombination system.

The gene encoding the mutant HPPD polypeptide according to the present invention has higher tolerance to HPPD-inhibitor herbicides, which is an important basis for herbicide tolerant crops and selectable marker trait opportunities.

The term "expression cassette" as used herein means a nucleic acid molecule capable of directing expression of a particular nucleotide sequence in an appropriate host cell, comprising a promoter effectively linked to the nucleotide sequence of interest (i.e., a polynucleotide encoding a mutant HPPD polypeptide or variant thereof that retains HPPD enzymatic activity, alone or in combination with one or more additional nucleic acid molecules encoding polypeptides that confer desirable traits) which is effectively linked to termination signals. The coding region usually codes for a protein of interest but may also code for a functional RNA of interest, for example antisense RNA or a non-translated RNA, in the sense or antisense direction. The expression cassette comprising the nucleotide sequence of interest may be chimeric, meaning that at least one of its components is heterologous with respect to at least one of its other components. The expression cassette may also be one that is wild-type but has been obtained in a recombinant form useful for heterologous expression. Typically, however, the expression cassette is heterologous with respect to the host, i.e., the particular DNA sequence of the expression cassette does not occur wild-type in the host cell and must have been introduced into new host cell by a transformation event. The expression of the nucleotide sequence in the expression cassette may be under the control of a constitutive promoter or of an inducible promoter that initiates transcription only when the host cell is exposed to some particular external stimulus. Additionally, the promoter can also be specific to a particular tissue or organ or stage of development.

The present invention encompasses the transformation of plants with expression cassettes capable of expressing a polynucleotide of interest (i.e., a polynucleotide encoding a mutant HPPD polypeptide or variant thereof that retains HPPD enzymatic activity, alone or in combination with one or more additional nucleic acid molecules encoding polypeptides that confer desirable traits). The expression cassette will include in the 5'-3' direction of transcription, a transcriptional and translational initiation region (i.e., a promoter) and a polynucleotide open reading frame. The expression cassette may optionally comprise a transcriptional and translational termination region (i.e., termination region) functional in plants. In some embodiments, the expression cassette comprises a selectable marker gene to allow for selection for stable transformants. Expression constructs of the invention may also comprise a leader sequence and/or a sequence allowing for inducible expression of the polynucleotide of interest.

The regulatory sequences of the expression construct are effectively linked to the polynucleotide of interest. The regulatory sequence in the present invention includes, but is not limited to, a promoter, a transit peptide, a terminator, an enhancer, a leader sequence, an intron and other regulatory sequences operably linked to the herbicide-tolerant gene encoding the mutant HPPD polypeptide.

The promoter is a plant expressible promoter. The "plant expressible promoter" refers to a promoter that ensures the expression of the coding sequence linked thereto in a plant cell. The plant expressible promoter can be a constitutive promoter. Examples of the promoters directing the constitutive expression in plants include, but are not limited to, a 35S promoter derived from a cauliflower mosaic virus, maize Ubi promoters, rice GOS2 gene promoters, and the like. Alternatively, the plant expressible promoter can be a tissue specific promoter, i.e. the promoter directs the expression of an coding sequence in several tissues, such as green tissues, at a level higher than in other tissues of the plant (which can be measured through conventional RNA trials), such as a PEP carboxylase promoter. Alternatively, the plant expressible promoter can be a wound-inducible promoter. The wound-inducible promoter or a promoter directing a wound-induced expression pattern means that when a plant suffers from a wound caused by a mechanical factor or the gnawing of insects, the expression of the coding sequence under the regulation of the promoter is significantly improved compared to normal growth conditions. Examples of the wound-inducible promoters include, but are not limited to, promoters of potato and tomato protease inhibitor genes (pin I and pin II) and a maize protease inhibitor gene (MPI).

The transit peptide (also known as secretion signal sequence or targeting sequence) directs a transgenic product to a specific organelle or cell compartment. For a receptor protein, the transit peptide may be heterologous, for example, targeting the chloroplast using a sequence encoding the chloroplast transit peptide, or targeting the endoplasmic reticulum using a 'KDEL' retention sequence, or targeting the vacuole using CTPP of a barley phytolectin gene.

The leader sequence includes, but is not limited to, a small RNA virus leader sequence, such as an EMCV leader sequence (a 5' non-coding region of encephlomyocarditis virus); a potato virus Y group leader sequence, such as a MDMV (Maize Dwarf Mosaic Virus) leader sequence; human immunoglobulin heavy chain binding protein (BiP); an untranslated leader sequence of the coat protein mRNA of alfalfa mosaic virus (AMV RNA4); and a tobacco mosaic virus (TMV) leader sequence.

The enhancer includes, but is not limited to, a cauliflower mosaic virus (CaMV) enhancer, figwort mosaic virus (FMV) enhancer, carnation etched ring virus (CERV) enhancer, cassava vein mosaic virus (CsVMV) enhancer, mirabilis mosaic virus (MMV) enhancer, cestrum yellow leaf curling virus (CmYLCV) enhancer, cotton leaf curl Multan virus (CLCuMV) enhancer, commelina yellow mottle virus (CoYMV) enhancer and peanut chlorotic streak caulimovirus (PCLSV) enhancer.

For use in a monocotyledonous plant, the intron includes, but is not limited to, a maize hsp70 intron, maize ubiquitin intron, Adh intron 1, sucrose synthase intron or rice Act1 intron. For use in a dicotyledonous plant, the intron includes, but is not limited to, a CAT-1 intron, pKANNIBAL intron, PIV2 intron and "super ubiquitin" intron.

The terminator can be a suitable polyadenylation signal sequence that functions in a plant, including, but not limited to, a polyadenylation signal sequence derived from the Agrobacterium tumefaciens nopaline synthetase (NOS) gene, a polyadenylation signal sequence derived from the protease inhibitor II (pinII) gene, a polyadenylation signal sequence derived from the pea ssRUBISCO E9 gene and a polyadenylation signal sequence derived from the α-tubulin gene.

The "effectively linking" in the present invention indicates the binding of nucleic acid sequences that enables one of the sequences to provide a function required for the sequence linked thereto. The "effectively linking" in the present invention can link a promoter to a sequence of interest, so that the transcription of the sequence of interest is controlled and regulated by the promoter. When a sequence of interest encodes a protein and the expression of the protein is desired, "effectively linking" means that: a promoter is linked to the sequence in such a manner that the resulting transcript is efficiently translated. If the linking of a promoter to a coding sequence is a transcript fusion and expression of the encoded protein is to be achieved, such linking is created that the first translation initiation codon in the resulting transcript is the initiation codon in the coding sequence. Alternatively, if the linking of a promoter to a coding sequence is a translation fusion and expression of the encoded protein is to be achieved, such a linking is created that the first translation initiation codon contained in the 5' untranslated sequence is linked to the promoter in such a manner that the relationship of the resulting translation product with the translation open reading frame encoding the desired protein is an in-frame. Nucleic acid sequences that can be "effectively linked" include, but are not limited to: sequences providing gene expression functions (i.e., gene expression elements, such as promoters, 5' untranslated regions, introns, protein coding regions, 3' untranslated regions, polyadenylation sites and/or transcription terminators), sequences providing DNA transfer and/or integration functions (i.e., T-DNA boundary sequences, site-specific recombinase recognition sites and integrase recognition sites), sequences providing selective functions (i.e., antibiotic resistance markers and biosynthesis genes), sequences providing marker scoring functions, sequences assisting in sequence manipulation *in vitro* or *in vivo* (i.e., polylinker sequences and site-specific recombination sequences), and sequences providing replication functions (i.e., the bacterial origins of replication, autonomously replicating sequences and centromeric sequences).

The genome of a plant, plant tissue or plant cell in the present invention refers to any genetic material within the plant, plant tissue or plant cell, and includes nuclear, plastid and mitochondrial genomes.

As used herein, the term "plant part" or "plant tissue" includes plant cells, plant protoplasts, plant cell tissue cultures from which plants can be regenerated, plant calli, plant clumps, and plant cells that are intact in plants or parts of plants such as embryos, pollen, ovules, seeds, leaves, flowers, branches, fruit, kernels, ears, cobs, husks, stalks, roots, root tips, anthers, and the like.

The mutant HPPD polypeptide of the present invention can be applied to various types of plants. The dicotyledonous plant includes, but is not limited to, alfalfa, beans, cauliflowers, cabbages, carrots, celery, cotton, cucumbers, eggplants, lettuces, melon, peas, peppers, zucchinis, radishes, oilseed rape, spinach, soybeans, pumpkins, tomatoes, *Arabidopsis thaliana,* peanuts or watermelons; preferably, the dicotyledonous plant refers to cucumbers, soybeans, *Arabidopsis thaliana,* tobacco, cotton, peanuts or oilseed rape. The monocotyledonous plant includes, but is not limited to, rice, sorghum, wheat, barley, rye, millet, sugar cane, oats or turfgrass. Preferably, the monocotyledonous plant refers to rice, sorghum, wheat, barley, millet, sugar cane or oats.

As used herein, the term "plant transformation" means that once an herbicide resistant or tolerant mutant HPPD polynucleotide, alone or in combination with one or more additional nucleic acid molecules encoding polypeptides that confer desirable traits, has been cloned into an expression system, it is transformed into a plant cell. The receptors and target expression cassettes of the present invention can be introduced into the plant cell in a number of art-recognized ways. The term "introducing" in the context of a polynucleotide, for example, a nucleotide construct of interest, is intended to mean presenting to the plant the polynucleotide in such a manner that the polynucleotide gains access to the interior of a cell of the plant. Where more than one polynucleotide is to be introduced, these polynucleotides can be assembled as part of a single nucleotide construct, or as separate nucleotide constructs, and can be located on the same or different transformation vectors. Accordingly, these polynucleotides can be introduced into the host cell of interest in a single transformation event, in separate transformation events, or, for example, in plants, as part of a breeding protocol. The methods of the invention do not depend on a particular method for introducing one or more polynucleotides into a plant, only that the polynucleotide(s) gains access to the interior of at least one cell of the plant. Methods for introducing one or more polynucleotides into plants are known in the art including, but not limited to, transient transformation methods, stable transformation methods, and virus-mediated methods or genome-editing techniques.

The term "stable transformation" means that an exogenous gene is introduced into the genome of the plant and stably integrates into the plant or its genome of any successive generations, so that the exogenous gene is stably inherited.

The term "transient transformation" means that a nucleic acid molecule or protein is introduced into the plant cell to execute the function, but does not integrate into the genome of the plant, so that an exogenous gene cannot be stably inherited.

The term "genome-editing technique" refers to the techniques used to modify the genome that are capable of precisely manipulating the genomic sequences to realize operations such as site-directed gene mutations, insertions and deletions. At present, the genome-editing techniques mainly include homing endonucleases (HEs), Zinc-finger nucleases (ZFNs), transcription activator-like effector nucleases (TALENs), and Clustered regulatory interspaced short palindromic repeat (CRISPR) technologies.

Numerous transformation vectors available for plant transformation are known to those of ordinary skills in the art, and the genes pertinent to this invention can be used in conjunction with any such vectors. The selection of vector will depend upon the preferred transformation technique and the target species for transformation. For certain target species, different antibiotic or herbicide selection markers may be preferred. Selection markers used routinely in transformation include the nptll gene (which was published by Bevan et al., Nature 304:184-187 (1983)), which confers resistance to kanamycin and related antibiotics or herbicides; the pat and bar genes, which confer resistance to the herbicide glufosinate (also called phosphinothricin; see White et al., Nucl. Acids Res 18: 1062 (1990), Spencer et al. Theon. Appl. Genet. 79: 625-631 (1990) and U.S. Pat. Nos. 5,561,236 and 5,276,268); the hph gene, which confers resistance to the antibiotic hygromycin (Blochinger & Diggelmann, Mol. Cell. Biol. 4: 2929-2931); the dhfr gene, which confers resistance to methatrexate (Bourouis et al., EMBO J. 2(7): 1099-1104 (1983)); the EPSPS gene, which confers resistance to glyphosate (U.S. Pat. Nos. 4,940,935 and 5,188,642); the glyphosate N-acetyltransferase (GAT) gene, which also confers resistance to glyphosate (Castle et al. (2004) Science, 304:1151-1154; U.S. Patent App. Pub. Nos. 20070004912, 20050246798, and 20050060767); and the mannose-6-phosphate isomerase gene, which provides the ability to metabolize mannose (U.S. Pat. Nos. 5,767,378 and 5,994,629).

Methods for regeneration of plants are also well-known in the art. For example, Ti plasmid vectors have been utilized for the delivery of foreign DNA, as well as direct DNA uptake, liposomes, electroporation, microinjection, and microprojectiles.

The planting system in the present invention comprises a combination of a transgenic plant having tolerance to one or more herbicides, and/or an herbicide treatment available in different developmental stages of the plant. When the herbicide is applied, the planting system could effectively control weed growth and produce a plant with higher yield and/or less damage.

In the present invention, weeds refer to plants competing with the cultivated target plants in the plant growth environment.

The term "control" and/or "prevention" in the present invention refers to at least a direct application of (e.g., by spraying) an effective dose of an HPPD-inhibitor herbicide to the plant growth environment, so as to minimize weed development and/or stop weed growth. At the same time, the cultivated target plants should be morphologically normal and can be cultivated under conventional methods for product consumption and/or generation; and preferably, compared to non-transgenic wild-type plants, the cultivated plants have reduced plant damage and/or an increased plant yield. The reduced plant damage includes, but is not limited to, an improved stem resistance and/or an increased grain weight, etc. The "control" and/or "prevention" effect of the mutant HPPD polypeptide on weeds can exist independently, and will not be diminished and/or lost due to the presence of other substances that can "control" and/or "prevent" the weeds. Specifically, if any tissue of a transgenic plant (containing the gene encoding the mutant HPPD polypeptide) has and/or produces the mutant HPPD polypeptide and/or another substance that can control weeds simultaneously and/or separately, then the presence of the another substance will neither affect the "control" and/or "prevention" effect of the mutant HPPD polypeptide on the weeds, nor result in the "control" and/or "prevention" effect being completely and/or partially achieved by the another substance, regardless of the mutant HPPD polypeptide.

The "plant propagule" in the present invention includes, but is not limited to, plant sexual propagules and plant vegetative propagules. The plant sexual propagules include, but are not limited to, plant seeds; and the plant vegetative propagules refer to vegetative organs or a specific tissue of a plant which can generate a new plant under ex *vivo* conditions. The vegetative organs or the specific tissue include, but are not limited to, roots, stems and leaves, for example: plants with roots as the vegetative propagules including strawberries, sweet potatoes and the like; plants with stems as the vegetative propagules including sugar cane, potatoes (tubers) and the like; and plants with leaves as the vegetative propagules including aloe, begonias and the like.

The present invention may confer a new herbicide resistance trait to a plant, and no adverse effects on the phenotypes (including yields) are observed. The plant in the present invention can tolerate, e.g., 0.5×, 1×, 2×, 3×, 4×, or 8× the general application level of at least one herbicide tested. The improvement of these levels of tolerance is within the scope of the present invention. For example, foreseeable optimization and further development can be performed on various techniques known in the art, in order to increase the expression of a given gene.

The present invention provides a mutant HPPD polypeptide, a coding gene and use thereof, having the following advantages:
1. The present invention discloses for the first time that a combinatorial mutation at positions 372 + 383 of hydroxyphenyl pyruvate dioxygenase polypeptides from different species can confer a synergistically enhanced tolerance to HPPD-inhibitor herbicides of pyrazolinates, triketones and isoxazoles upon plants, and in particular can confer transgenic soybean plants with the tolerance to topramezone, isoxaflutole and mesotrione at four-fold field concentration. Therefore, the present invention has a broad application prospect in plants.
2. in the hydroxyphenylpyruvate dioxygenase polypeptide of the present invention, a combination of a combinatorial mutation at positions 372+383 and a mutation at other position would not affect the synergistically enhanced tolerance to HPPD-inhibitor herbicides produced by the combinatorial mutation ta positions 372+383 alone, demonstrating the importance and stability of the tolerance to HPPD-inhibitor herbicides of the plants conferred by the combinatorial mutation at positions 372+383 of the HPPD polypeptide.
3. On the basis of the combinatorial mutation at positions 372+383 of the hydroxyphenylpyruvate dioxygenase polypeptide of the present invention, optimizing the C-terminal of the HPPD amino acid sequence is beneficial for improving the tolerance of plants to isoxaflutole.

The technical solution of the present invention is further illustrated in details through the drawings and examples below.

### BRIEF DESCRIPTION OF THE DRAWINGS

FIG.1 is a schematic structural diagram of a recombinant expression vector DBN11726 containing the AsHPPDm-F372A-F383W-02 nucleotide sequence for *Arabidopsis thaliana*according to the present invention;
FIG.2 is a schematic structural diagram of a control recombinant expression vector DBN11726N according to the present invention;
FIG.3 is a phylogenetic tree of HPPDs from different species according to the present invention.

### DETAILED DESCRIPTION OF THE INVENTION

The embodiments of the mutant hydroxyphenylpyruvate dioxygenase polypeptide, the coding gene and use thereof according to the present invention will be further illustrated in specific examples.

### Example 1: Selection of positions 372 and 383 of AsHPPD for the combinatorial mutation (F372A+F383W) and verification of the mutation effect

### 1. Acquisition of AsHPPD and AsHPPDm-F372A-F383W genes

The amino acid sequence of the *Avena sativa* wild-type HPPD (AsHPPD) is set forth as SEQ ID NO: 1 in the SEQUENCE LISTING; the AsHPPD-01 nucleotide sequence encoding the AsHPPD is set forth as SEQ ID NO: 2 in the SEQUENCE LISTING; and the AsHPPD-02 nucleotide sequence encoding the AsHPPD, which was obtained based on the *Arabidopsis thaliana*/soybean common codon usage bias, is set forth as SEQ ID NO: 3 in the SEQUENCE LISTING.

The amino acid at position 372 of the AsHPPD amino acid sequence was mutated from the original phenylalanine (F) to alanine (A), to obtain the AsHPPDm-F372A amino acid sequence as set forth in SEQ ID NO: 4 in the SEQUENCE LISTING; the AsHPPDm-F372A-01 nucleotide sequence encoding the AsHPPDm-F372A amino acid sequence is set forth as SEQ ID NO: 5 in the SEQUENCE LISTING; and the AsHPPDm-F372A-02 nucleotide sequence encoding the AsHPPDm-F372A amino acid sequence, which was obtained based on the *Arabidopsis thaliana*/soybean common codon usage bias, is set forth as SEQ ID NO: 6 in the SEQUENCE LISTING.

The amino acid at position 383 of the AsHPPD amino acid sequence was mutated from the original phenylalanine (F) to tryptophan (W), to obtain the AsHPPDm-F383W amino acid sequence as set forth in SEQ ID NO: 7 in the SEQUENCE LISTING; the AsHPPDm-F383W-01 nucleotide sequence encoding the AsHPPDm-F383W amino acid sequence is set forth as SEQ ID NO: 8 in the SEQUENCE LISTING; and the AsHPPDm-F383W-02 nucleotide sequence encoding the AsHPPDm-F383W amino acid sequence, which was obtained based on the *Arabidopsis thaliana*/soybean common codon usage bias, is set forth as SEQ ID NO: 9 in the SEQUENCE LISTING.

The amino acid at position 372 of the AsHPPD amino acid sequence was mutated from the original phenylalanine (F) to alanine (A) and the amino acid at position 383 was mutated from the original phenylalanine (F) to tryptophan (W), to obtain the AsHPPDm-F372A-F383W amino acid sequence as set forth in SEQ ID NO: 10 in the SEQUENCE LISTING; the AsHPPDm-F372A-F383W-01 nucleotide sequence encoding the AsHPPDm-F372A-F383W amino acid sequence is set forth as SEQ ID NO: 11 in the SEQUENCE LISTING; and the AsHPPDm-F372A-F383W-02 nucleotide sequence encoding the AsHPPDm-F372A-F383W amino acid sequence, which was obtained based on the *Arabidopsis thaliana*/soybean common codon usage bias, is set forth as SEQ ID NO: 12 in the SEQUENCE LISTING.

### 2. Synthesis of the aforementioned nucleotide sequences

The 5' and 3' ends of the synthesized AsHPPD-02 nucleotide sequence (SEQ ID NO: 3), AsHPPDm-F372A-02 nucleotide sequence (SEQ ID NO: 6), AsHPPDm-F383W-02 nucleotide sequence (SEQ ID NO: 9), and AsHPPDm-F372A-F383W-02 nucleotide sequence (SEQ ID NO: 12) were respectively linked to a universal adapter primer 1:
Universal adapter primer 1 for the 5' end: 5'-agtttttctgattaacagactagt-3', as set forth in SEQ ID NO: 230 in the SEQUENCE LISTING; and
Universal adapter primer 1 for the 3' end: 5'-caaatgtttgaacgatcggcgcgcc-3', as set forth in SEQ ID NO: 231 in the SEQUENCE LISTING.
3. Construction of recombinant expression vectors containing *Avena sativa* HPPD genes (F372A-F383W) for *Arabidopsis thaliana*

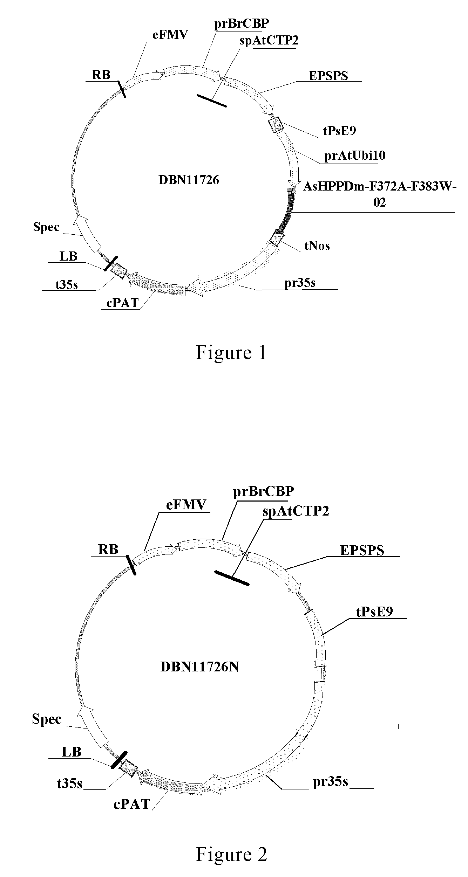

A plant expression vector DBNBC-01 was subjected to double digestion using restriction enzymes Spe I and *Asc* I to linearize the plant expression vector. The digestion product was purified to obtain the linearized DBNBC-01 expression vector backbone (vector backbone: pCAMBIA2301 (which is available from CAMBIA)) which then underwent a recombination reaction with the AsHPPDm-F372A-F383W-02 nucleotide sequence linked to the universal adapter primer 1, according to the procedure of Takara InFusion products seamless connection kit (Clontech, CA, USA, CAT: 121416) instructions, to construct a recombinant expression vector DBN11726 with the schematic structure as shown in FIG. 1 (Spec: spectinomycin gene; RB: right border; eFMV: 34S enhancer of Figwort mosaic virus (SEQ ID NO: 13); prBrCBP: promoter of oilseed rape eukaryotic elongation factor gene 1α (Tsf1) (SEQ ID NO: 14); spAtCTP2: *Arabidopsis* thalianachloroplast transit peptide (SEQ ID NO: 15); EPSPS: 5-enolpyruvylshikimate-3-phosphate synthase gene (SEQ ID NO: 16); tPsE9: terminator of a pea RbcS gene (SEQ ID NO: 17); prAtUbi10: promoter of an *Arabidopsis thaliana*Ubiquitin 10 gene (SEQ ID NO: 18); AsHPPDm-F372A-F383W-02: AsHPPDm-F372A-F383W-02 nucleotide sequence (SEQ ID NO: 12); tNos: terminator of a nopaline synthase gene (SEQ ID NO: 19); pr35S-01: the cauliflower mosaic virus 35S promoter (SEQ ID NO: 20); PAT: phosphinothricin acetyltransferase gene (SEQ ID NO: 21);
t35S: cauliflower mosaic virus 35S terminator (SEQ ID NO: 22); LB: left border).

*Escherichia coli* T1 competent cells were transformed with the recombinant expression vector DBN11726 by using a heat shock method under the following heat shock conditions: 50 µL of *Escherichia coli* T1 competent cells and 10 µL of plasmid DNA (recombinant expression vector DBN11726) were water-bathed at 42°C for 30 seconds, shake cultured at 37°C for 1 hour (using a shaker at a rotation speed of 100 rpm for shaking), and then cultured under the condition of a temperature of 37°C on the LB solid plate containing 50 mg/L of spectinomycin for 12 hours; white bacterial colonies were picked out, and cultured under the condition of a temperature of 37°C overnight in an LB liquid culture medium (10 g/L of tryptone, 5 g/L of yeast extract, 10 g/L of NaCl, and 50 mg/L of spectinomycin; adjusted to a pH of 7.5 with NaOH). The plasmids in the cells were extracted through an alkaline method: the bacteria solution was centrifuged at a rotation speed of 12,000 rpm for 1 min, the supernatant was removed, and the precipitated thalli were suspended with 100 µL of ice pre-cooled solution I (25 mM Tris-HCl, 10 mM EDTA (ethylenediaminetetraacetic acid), and 50 mM glucose, with a pH of 8.0); 200 µL of newly prepared solution II (0.2M NaOH, 1% SDS (sodium dodecyl sulfate)) was added, mixed by inverting the tube 4 times, and placed on ice for 3-5 min; 150 µL of ice-cold solution III (3 M potassium acetate, 5 M acetic acid) was added, mixed uniformly immediately and placed on ice for 5-10 min; the mixture was centrifuged under the conditions of a temperature of 4°C and a rotation speed of 12,000 rpm for 5 min, 2-fold volumes of anhydrous ethanol was added to the supernatant, mixed uniformly and placed at room temperature for 5 min; the mixture was centrifuged under the conditions of a temperature of 4°C and a rotation speed of 12,000 rpm for 5 min, the supernatant was discarded, and the precipitate was washed with ethanol at a concentration of 70% (V/V) and then was air dried; 30 µL of TE (10 mM Tris-HCl, and 1 mM EDTA, with a pH of 8.0) containing RNase (20 µg/mL) was added to dissolve the precipitate; the obtained product was water bathed at a temperature of 37°C for 30 min to digest the RNA; and stored at a temperature of -20°C for use. The extracted plasmids were identified by sequencing. The results showed that the nucleotide sequence between the *Spe* I and *Asc* I sites in the recombinant expression vector DBN11726 was the one as set forth in SEQ ID NO: 12 in the SEQUENCE LISTING, i.e., the AsHPPDm-F372A-F383W-02 nucleotide sequence.

According to the above method for constructing recombinant expression vector DBN11726, the AsHPPD-02 nucleotide sequence, AsHPPDm-F372A-02 nucleotide sequence and AsHPPDm-F383W-02 nucleotide sequence which were linked to the universal adapter primer 1 were respectively subjected to a recombination reaction with the linearized DBNBC-01 expression vector backbone, to construct the recombinant expression vectors DBN11727, DBN11728 and DBN11729 in sequence. Respective sequencing verified that the nucleotide sequences in the recombinant expression vectors DBN11727, DBN11728 and DBN11729 respectively comprise the nucleotide sequence as set forth in SEQ ID NO: 3, the nucleotide sequence as set forth in SEQ ID NO: 6 and the nucleotide sequence as set forth in SEQ ID NO: 9 in the SEQUENCE LISTING; i.e., AsHPPD-02 nucleotide sequence, AsHPPDm-F372A-02 nucleotide sequence and AsHPPDm-F383W-02 nucleotide sequence were inserted correctly.

The control recombinant expression vector DBN11726N was constructed, of which the structure was shown in FIG. 2 (Spec: the spectinomycin gene; RB: right border; eFMV: 34S enhancer of Figwort mosaic virus (SEQ ID NO: 13); prBrCBP: promoter of oilseed rape eukaryotic elongation factor gene 1α (Tsf1) (SEQ ID NO: 14); spAtCTP2: *Arabidopsis* thalianachloroplast transit peptide (SEQ ID NO: 15); EPSPS: 5-enolpyruvylshikimate-3-phosphate synthase gene (SEQ ID NO: 16); tPsE9: terminator of a pea RbcS gene (SEQ ID NO: 17); pr35S-01: the cauliflower mosaic virus 35S promoter (SEQ ID NO: 20); PAT: phosphinothricin acetyltransferase gene (SEQ ID NO: 21); t35S: cauliflower mosaic virus 35S terminator (SEQ ID NO: 22); LB: left border).

### 4. Transformation of Agrobacterium with the recombinant expression vectors for Arabidopsis thaliana

The recombinant expression vectors DBN11726, DBN11727, DBN11728, DBN11729, and DBN11726N which had been constructed correctly were respectively transformed into *Agrobacterium* GV3101 using a liquid nitrogen method, under the following transformation conditions: 100 µL of *Agrobacterium* GV3101 and 3 µL of plasmid DNA (recombinant expression vector) were placed in liquid nitrogen for 10 minutes, and bathed in warm water at 37°C for 10 min; the transformed *Agrobacterium* GV3101 was inoculated into an LB tube, cultured under the conditions of a temperature of 28°C and a rotation speed of 200 rpm for 2 hours, and spread on the LB solid plate containing 50 mg/L of rifampicin and 50 mg/L of spectinomycin until positive single clones were grown, and single clones were picked out for culturing and the plasmids thereof were extracted. The extracted plasmids were identified by sequencing. The results showed that the structures of the recombinant expression vectors DBN11726 to DBN11729 and DBN11726N were completely correct.

### 5. Acquisition of transgenic Arabidopsis thaliana plants

Seeds of wild-type *Arabidopsis thalianawere* suspended in a 0.1% (w/v) agarose solution. The suspended seeds were stored at 4°C for 2 days to fulfill the need for dormancy, in order to ensure synchronous seed germination. Vermiculite was mixed with horse manure soil, the mixture was sub-irrigated with water to wet, and the soil mixture was allowed to drain the water away for 24 hours. The pretreated seeds were sowed in the soil mixture and covered with a moisturizing cover for 7 days. The seeds were germinated and the plants were cultivated in a greenhouse under long sunlight conditions (16-hour light/8-hour dark) at a constant temperature (22°C) and a constant humidity (40-50%), with a light intensity of 120-150 µmol/m²s⁻¹. The plants were initially irrigated with Hoagland's nutrient solution and then with deionized water, thus keeping the soil moist, but not water penetrated.

*Arabidopsis thalianawas* transformed using the flower soaking method. One or more 15-30 mL pre-cultures of a LB culture solution (10 g/L of tryptone, 5 g/L of yeast extract, and 10 g/L of NaCl; adjusted to a pH of 7.5 with NaOH) containing spectinomycin (50 mg/L) and rifampicin (10 mg/L) were inoculated with the picked Agrobacterium colonies. The pre-cultures were incubated at a temperature of 28° C and a rotation speed of 220 rpm with shaking at a constant speed overnight. Each pre-culture was used to inoculate two 500 mL cultures of the LB culture solution containing spectinomycin (50 mg/L) and rifampicin (10 mg/L), and the cultures were incubated at 28°C with continuous shaking overnight. Centrifugation at a rotation speed of about 4,000 rpm was carried out at room temperature for 20 minutes to precipitate cells, and the resulting supernatant was discarded. The cell precipitate was gently re-suspended in 500 mL of an osmotic medium which contained 1/2×MS salt/B5 vitamin, 10% (w/v) sucrose, 0.044 µM of benzylaminopurine (10 µL/L (1 mg/mL stock solution in DMSO)) and 300 µL/L of Silvet L-77. About 1-month-old *Arabidopsis thaliana* plants were soaked in an osmotic culture medium which contained re-suspended cells for 15 seconds to ensure immersion of the latest inflorescence. Then, the *Arabidopsis thaliana* plants were reclined laterally and covered and they were kept wet in dark for 24 hours. The *Arabidopsis thaliana* plants were normally cultivated with a photoperiod of 16 hours of light/8 hours of darkness at 22°C. Seeds were harvested after about 4 weeks.

The newly harvested (AsHPPD-02 nucleotide sequence, AsHPPDm-F372A-02 nucleotide sequence, AsHPPDm-F383W-02 nucleotide sequence, AsHPPDm-F372A-F383W-02 nucleotide sequence, and the control recombinant expression vector DBN11726N) T₁ seeds were dried at room temperature for 7 days. The seeds were sowed in 26.5 cm ×51 cm germination disks, and 200 mg of T₁ seeds (about 10,000 seeds) were accepted per disk, wherein the seeds had been previously suspended in distilled water and stored at 4°C for 2 days to fulfill the need for dormancy, in order to ensure synchronous seed germination.

Vermiculite was mixed with horse manure soil, the mixture was sub-irrigated with water to wet, and water was drained by gravity. The pretreated seeds were sowed evenly in the soil mixture using a pipette, and covered with a moisturizing cover for 4-5 days. The cover was removed 1 day before the post-emergence spraying application of glufosinate (used to select the co-transformed PAT gene) for the selection of initial transformant.

The T1 plants were sprayed with a 0.2% solution of a Liberty herbicide (200 g ai/L of glufosinate) by a DeVilbiss compressed air nozzle at a spray volume of 10 mL/disk (703 L/ha) 7 days after planting (DAP) and 11 DAP (the cotyledon stage and 2-4 leaf stage, respectively) to provide an effective amount of glufosinate of 280 g ai/ha per application. Surviving plants (actively growing plants) were identified 4-7 days after the final spraying, and transplanted to 7 cm×7 cm square pots prepared from horse manure soil and vermiculite (3-5 plants/disk). The transplanted plants were covered with a moisturizing cover for 3-4 days, and placed in a 22°C culture chamber or directly transferred into a greenhouse as described above. Then, the cover was removed, and at least 1 day before testing the ability of the mutant HPPD gene to provide HPPD-inhibitor herbicide tolerance, the plants were planted in a greenhouse (22±5°C, 50±30% RH, 14 hours of light: 10 hours of darkness, a minimum of 500 µE/m²s⁻¹ wild-type + supplemental light).

### 6. Detection of the herbicide tolerance of the transgenic Arabidopsis thaliana plants containing the AsHPPDm-F372A-F383W-02 nucleotide sequence.

T₁ transformants were initially selected from the untransformed seeds using a glufosinate selection scheme. The *Arabidopsis thaliana* T1 plants (AsHPPD-02) into which the AsHPPD-02 nucleotide sequence was introduced, the *Arabidopsis thaliana* T1 plants (AsHPPDm-F372A-02) into which the AsHPPDm-F372A-02 nucleotide sequence was introduced, the *Arabidopsis thaliana* T1 plants (AsHPPDm-F383W-02) into which the AsHPPDm-F383W-02 nucleotide sequence was introduced, the *Arabidopsis thaliana* T1 plants (AsHPPDm-F372A-F383W-02) into which the AsHPPDm-F372A-F383W-02 nucleotide sequence was introduced, the *Arabidopsis thaliana* T1 plants (DBN11726N) into which the control recombinant expression vector DBN11726N was introduced, and the wild-type *Arabidopsis thaliana* plants (CK) (18 days after sowing) were sprayed respectively with topramezone at three concentrations (100 g ai/ha (four-fold field concentration, 4×), 200 g ai/ha (eight-fold field concentration, 8×), and 0 g ai/ha (water, 0×)), isoxaflutole at three concentrations (140 g ai/ha (two-fold field concentration, 2×), 280 g ai/ha (four-fold field concentration, 4×), and 0 g ai/ha (water, 0×)), and mesotrione at three concentrations (210 g ai/ha (two-fold field concentration, 1×), 420 g ai/ha (four-fold field concentration, 4×), and 0 g ai/ha (water, 0×)), to determine the tolerance of *Arabidopsis thaliana* to the herbicides. The degree of damage caused by the herbicide was measured for each plant according to the proportion of bleached leaf area (the proportion of bleached leaf area=bleached leaf area/total leaf area×100%) 7 days after spraying (7 DAT): the case where there is basically no bleached phenotype is defined as grade 0, the case where the proportion of bleached leaf area is less than 50% is defined as grade 1 ,the case where the proportion of bleached leaf area is more than 50% is defined as grade 2, and the case where the proportion of bleached leaf area is 100% grade is defined as grade 3.

According to the formula X=[Σ(N×S)/(T×M)]×100, the performance of resistance of the transformation event of each recombinant expression vector was scored (X-the score for pesticide damage, N-the number of plants with the same grade of damage, S- the pesticide damage grade, T-the total number of plants, M-the maximum grade of pesticide damage) and the resistance is evaluated based on the scores: highly resistant plants (scores 0-15), moderately resistant plants (scores 16-33), poorly resistant plants (scores 34-67) and non-resistant plants (scores 68-100). The results were shown in TABLE 1.

**TABLE 1 Topramezone tolerance of transgenic Arabidopsis thaliana T1 plants**

| *Arabidopsis thaliana* genotypes | Concentration (g ai/ha) | Classification and statistics of the grade of pesticide damage | | | | Scores | Resistance evaluation |
|---|---|---|---|---|---|---|---|
| | | Grade 0 | Grade 1 | Grade 2 | Grade 3 | | |
| CK | 0 | 16 | 0 | 0 | 0 | 0 | |
| | 100 | 0 | 0 | 0 | 16 | 100 | non-resistant |
| | 200 | 0 | 0 | 0 | 16 | 100 | non-resistant |
| DBN11726N | 0 | 16 | 0 | 0 | 0 | 0 | |
| | 100 | 0 | 0 | 0 | 16 | 100 | non-resistant |
| | 200 | 0 | 0 | 0 | 16 | 100 | non-resistant |
| AsHPPD-02 | 0 | 16 | 0 | 0 | 0 | 0 | |
| | 100 | 0 | 0 | 4 | 12 | 92 | non-resistant |
| | 200 | 0 | 0 | 0 | 16 | 100 | non-resistant |
| AsHPPDm-F372A-02 | 0 | 16 | 0 | 0 | 0 | 0 | |
| | 100 | 16 | 0 | 0 | 0 | 0 | highly resistant |
| | 200 | 16 | 0 | 0 | 0 | 0 | highly resistant |
| AsHPPDm-F383W-02 | 0 | 16 | 0 | 0 | 0 | 0 | |
| | 100 | 16 | 0 | 0 | 0 | 0 | highly resistant |
| | 200 | 16 | 0 | 0 | 0 | 0 | highly resistant |
| AsHPPDm-F372A-F383W-02 | 0 | 16 | 0 | 0 | 0 | 0 | |
| | 100 | 16 | 0 | 0 | 0 | 0 | highly resistant |
| | 200 | 16 | 0 | 0 | 0 | 0 | highly resistant |

The results of Table 1 show that as compared with CK, the *Arabidopsis thaliana* genotypes AsHPPDm-F372A-02, AsHPPDm-F383W-02 and AsHPPDm-F372A-F383W-02 all exhibited highly-resistant tolerance to topramezone at four-fold or eight-fold field concentration, while AsHPPD-02 and DBN11726N both exhibited no tolerance to topramezone.

**TABLE 2 Isoxaflutole tolerance of transgenic Arabidopsis thaliana T1 plants**

| *Arabidopsis thaliana* genotypes | Concentration (g ai/ha) | Classification and statistics of the grade of pesticide damage | | | | Scores | Resistance evaluation |
|---|---|---|---|---|---|---|---|
| | | Grade 0 | Grade 1 | Grade 2 | Grade 3 | | |
| CK | 0 | 16 | 0 | 0 | 0 | 0 | |
| | 140 | 0 | 0 | 0 | 16 | 100 | non-resistant |
| | 280 | 0 | 0 | 0 | 16 | 100 | non-resistant |
| DBN11726N | 0 | 16 | 0 | 0 | 0 | 0 | |
| | 140 | 0 | 0 | 0 | 16 | 100 | non-resistant |
| | 280 | 0 | 0 | 0 | 16 | 100 | non-resistant |
| AsHPPD-02 | 0 | 16 | 0 | 0 | 0 | 0 | |
| | 140 | 0 | 1 | 4 | 11 | 88 | non-resistant |
| | 280 | 0 | 0 | 0 | 16 | 100 | non-resistant |
| AsHPPDm-F372A-02 | 0 | 16 | 0 | 0 | 0 | 0 | |
| | 140 | 5 | 6 | 5 | 0 | 33 | moderately resistant |
| | 280 | 0 | 0 | 16 | 0 | 67 | poorly resistant |
| AsHPPDm-F383W-02 | 0 | 16 | 0 | 0 | 0 | 0 | |
| | 140 | 6 | 10 | 0 | 0 | 21 | moderately resistant |
| | 280 | 4 | 4 | 8 | 0 | 42 | poorly resistant |
| AsHPPDm-F372A-F383W-02 | 0 | 16 | 0 | 0 | 0 | 0 | |
| | 140 | 16 | 0 | 0 | 0 | 0 | highly resistant |
| | 280 | 12 | 4 | 0 | 0 | 8 | highly resistant |

The results of TABLE 2 show that (1) as compared with CK, the *Arabidopsis thaliana* genotypes AsHPPDm-F372A-02, AsHPPDm-F383W-02 and AsHPPDm-F372A-F383W-02 exhibited different degrees of tolerance to isoxaflutole at different concentrations, while AsHPPD-02 and DBN11726N both exhibited no tolerance to isoxaflutole; (2) the *Arabidopsis thaliana* genotypes AsHPPDm-F372A-02, AsHPPDm-F383W-02 and AsHPPDm-F372A-F383W-02 respectively exhibited moderately-resistant tolerance, moderately-resistant tolerance, and highly-resistant tolerance to isoxaflutole at two-fold field concentration, showing that the combinatorial mutation (F372A+F383W) at positions 372 and 383 of the wild-type HPPD amino acid sequence achieved better effect than the single position mutation F372A or F383W; and (3) the *Arabidopsis thaliana* genotypes AsHPPDm-F372A-02, AsHPPDm-F383W-02 and AsHPPDm-F372A-F383W-02 respectively exhibited poorly-resistant tolerance, poorly-resistant tolerance, and highly-resistant tolerance to isoxaflutole at four-fold field concentration, showing that the combinatorial mutation (F372A+F383W) at positions 372 and 383 of the wild-type HPPD amino acid sequence achieved better effect than the single position mutation F372A or F383W, and further achieved a synergistically enhanced effect of herbicide tolerance.

**TABLE 3 Mesotrione tolerance of transgenic Arabidopsis thaliana T1 plants**

| *Arabidopsis thaliana* genotypes | Concentration (g ai/ha) | Classification and statistics of the grade of pesticide damage | | | | Scores | Resistance evaluation |
|---|---|---|---|---|---|---|---|
| | | Grade 0 | Grade 1 | Grade 2 | Grade 3 | | |
| CK | 0 | 16 | 0 | 0 | 0 | 0 | |
| | 210 | 0 | 0 | 0 | 16 | 100 | non-resistant |
| | 420 | 0 | 0 | 0 | 16 | 100 | non-resistant |
| DBN11726N | 0 | 16 | 0 | 0 | 0 | 0 | |
| | 210 | 0 | 0 | 0 | 16 | 100 | non-resistant |
| | 420 | 0 | 0 | 0 | 16 | 100 | non-resistant |
| AsHPPD-02 | 0 | 16 | 0 | 0 | 0 | 0 | |
| | 210 | 0 | 0 | 0 | 16 | 100 | non-resistant |
| | 420 | 0 | 0 | 0 | 16 | 100 | non-resistant |
| AsHPPDm-F372A-02 | 0 | 16 | 0 | 0 | 0 | 0 | |
| | 210 | 5 | 7 | 4 | 0 | 31 | moderately resistant |
| | 420 | 0 | 8 | 8 | 0 | 50 | poorly resistant |
| AsHPPDm-F383W-02 | 0 | 16 | 0 | 0 | 0 | 0 | |
| | 210 | 4 | 10 | 1 | 1 | 31 | moderately resistant |
| | 420 | 0 | 2 | 14 | 0 | 63 | poorly resistant |
| AsHPPDm-F372A-F383W-02 | 0 | 16 | 0 | 0 | 0 | 0 | |
| | 210 | 16 | 0 | 0 | 0 | 0 | highly resistant |
| | 420 | 15 | 1 | 0 | 0 | 2 | highly resistant |

The results of TABLE 3 show that (1) as compared with CK, the *Arabidopsis thaliana* genotypes AsHPPDm-F372A-02, AsHPPDm-F383W-02 and AsHPPDm-F372A-F383W-02 exhibited different degrees of tolerance to mesotrione at different concentrations, while AsHPPD-02 and DBN11726N both exhibited no tolerance to mesotrione; (2) the *Arabidopsis thaliana* genotypes AsHPPDm-F372A-02, AsHPPDm-F383W-02 and AsHPPDm-F372A-F383W-02 respectively exhibited moderately-resistant tolerance, moderately-resistant tolerance, and highly-resistant tolerance to mesotrione at two-fold field concentration, showing that the combinatorial mutation (F372A+F383W) at positions 372 and 383 of the wild-type HPPD amino acid sequence achieved better effect than the single position mutation F372A or F383W; and (3) the *Arabidopsis thaliana* genotypes AsHPPDm-F372A-02, AsHPPDm-F383W-02 and AsHPPDm-F372A-F383W-02 respectively exhibited poorly-resistant tolerance, poorly-resistant tolerance, and highly-resistant tolerance to mesotrione at four-fold field concentration, showing that the combinatorial mutation (F372A+F383W) at positions 372 and 383 of the wild-type HPPD amino acid sequence achieved better effect than the single position mutation F372A or F383W, and further achieved a synergistically enhanced effect of herbicide tolerance.

The above TABLES 2 and 3 adequately show that the combinatorial mutation (F372A+F383W) at positions 372 and 383 of the wild-type HPPD amino acid sequence had a synergistically enhanced effect of HPPD-inhibitor herbicide tolerance.

### Example 2: Combinatorail mutation at positions 372 and 383 (F372A+F383W) of the HPPD amino acid sequences from different species and verification of the mutation effect

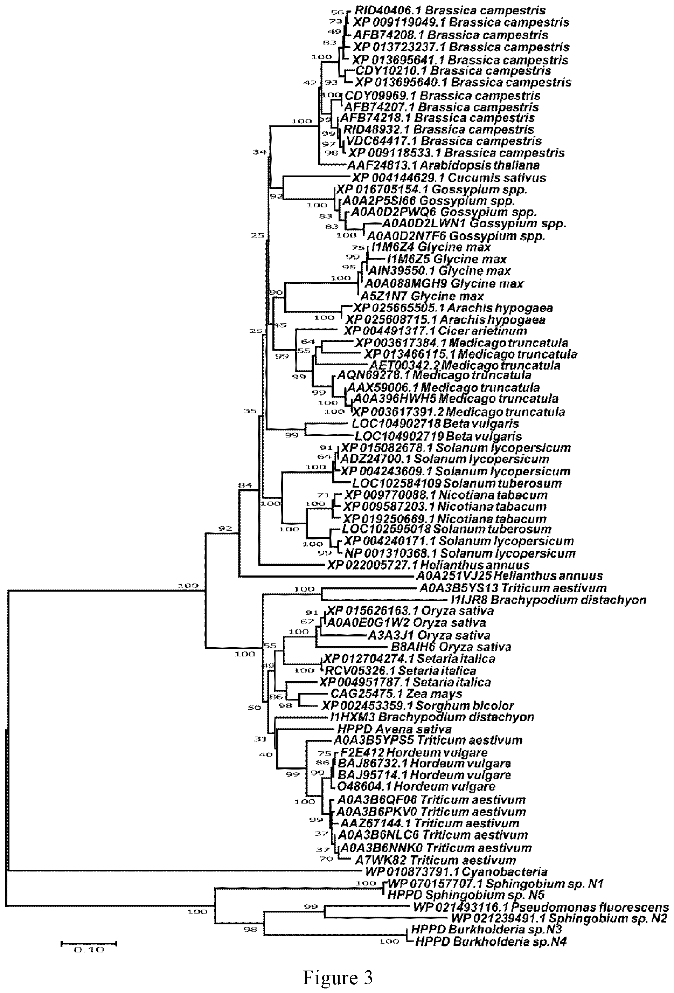

In order to further verify the synergistical effect of the combinatorial mutation at positions 372 and 383 of the HPPD amino acid sequence, a phylogenetic tree of HPPDs from different species (as shown in FIG. 3) was analyzed. HPPDs from representative species on different branches were selected, and the amino acids at positions 372 and 383 of the amino acid sequence were mutated (F372A+F383W) so as to verify the mutation effect.

### 1. Acquisition of HPPDs from different species and mutant HPPDs (F372A+F383W)

### (1) Acquisition of the mutant HPPDs (F372A+F383W) from Arabidopsis thaliana

The amino acid sequence of wild-type *Arabidopsis thaliana* HPPD (AtHPPD) is set forth in SEQ ID NO: 23 in the SEQUENCE LISTING; the AtHPPD-01 nucleotide sequence encoding the AtHPPD is set forth as SEQ ID NO: 24 in the SEQUENCE LISTING; and the AtHPPD-02 nucleotide sequence encoding the AtHPPD, which was obtained based on the *Arabidopsis thaliana*/soybean common codon usage bias, is set forth as SEQ ID NO: 25 in the SEQUENCE LISTING.

The amino acid at position 372 of the AtHPPD amino acid sequence was mutated from the original phenylalanine (F) to alanine (A), to obtain the AtHPPDm-F372A amino acid sequence as set forth in SEQ ID NO: 26 in the SEQUENCE LISTING; the AtHPPDm-F372A-01 nucleotide sequence encoding the AtHPPDm-F372A amino acid sequence is set forth as SEQ ID NO: 27 in the SEQUENCE LISTING; and the AtHPPDm-F372A-02 nucleotide sequence encoding the AtHPPDm-F372A amino acid sequence, which was obtained based on the *Arabidopsis thaliana*/soybean common codon usage bias, is set forth as SEQ ID NO: 28 in the SEQUENCE LISTING.

The amino acid at position 383 of the AtHPPD amino acid sequence was mutated from the original phenylalanine (F) to tryptophan (W), to obtain the AtHPPDm-F383W amino acid sequence as set forth in SEQ ID NO: 29 in the SEQUENCE LISTING; the AtHPPDm-F383W-01 nucleotide sequence encoding the AtHPPDm-F383W amino acid sequence is set forth as SEQ ID NO: 30 in the SEQUENCE LISTING; and the AtHPPDm-F383W-02 nucleotide sequence encoding the AtHPPDm-F383W amino acid sequence, which was obtained based on the *Arabidopsis thaliana*/soybean common codon usage bias, is set forth as SEQ ID NO: 31 in the SEQUENCE LISTING.

The amino acid at position 372 of the AtHPPD amino acid sequence was mutated from the original phenylalanine (F) to alanine (A) and the amino acid at position 383 was mutated from the original phenylalanine (F) to tryptophan (W), to obtain the AtHPPDm-F372A-F383W amino acid sequence as set forth in SEQ ID NO: 32 in the SEQUENCE LISTING; the AtHPPDm-F372A-F383W-01 nucleotide sequence encoding the AtHPPDm-F372A-F383W amino acid sequence is set forth as SEQ ID NO: 33 in the SEQUENCE LISTING; and the AtHPPDm-F372A-F383W-02 nucleotide sequence encoding the AtHPPDm-F372A-F383W amino acid sequence, which was obtained based on the *Arabidopsis thaliana*/soybean common codon usage bias, is set forth as SEQ ID NO: 34 in the SEQUENCE LISTING.

### (2) Acquisition of mutant HPPDs (F372A and F383W) from Medicago sativa

The amino acid sequence of wild-type *Medicago sativa* HPPD (MsHPPD) is set forth as SEQ ID NO: 35 in the SEQUENCE LISTING; the MsHPPD-01 nucleotide sequence encoding the MsHPPD is set forth as SEQ ID NO: 36 in the SEQUENCE LISTING; and the MsHPPD-02 nucleotide sequence encoding the MsHPPD, which was obtained based on the *Arabidopsis thaliana*/soybean common codon usage bias, is set forth as SEQ ID NO: 37 in the SEQUENCE LISTING.

The amino acid at position 372 of the MsHPPD amino acid sequence was mutated from the original phenylalanine (F) to alanine (A), to obtain the MsHPPDm-F372A amino acid sequence as set forth in SEQ ID NO: 38 in the SEQUENCE LISTING; the MsHPPDm-F372A-01 nucleotide sequence encoding the MsHPPDm-F372A amino acid sequence is set forth as SEQ ID NO: 39 in the SEQUENCE LISTING; and the MsHPPDm-F372A-02 nucleotide sequence encoding the MsHPPDm-F372A amino acid sequence, which was obtained based on the *Arabidopsis thaliana*/soybean common codon usage bias, is set forth as SEQ ID NO: 40 in the SEQUENCE LISTING.

The amino acid at position 383 of the MsHPPD amino acid sequence was mutated from the original phenylalanine (F) to tryptophan (W), to obtain the MsHPPDm-F383W amino acid sequence as set forth in SEQ ID NO: 41 in the SEQUENCE LISTING; the MsHPPDm-F383W-01 nucleotide sequence encoding the MsHPPDm-F383W amino acid sequence is set forth as SEQ ID NO: 42 in the SEQUENCE LISTING; and the MsHPPDm-F383W-02 nucleotide sequence encoding the MsHPPDm-F383W amino acid squence, which was obtained based on the *Arabidopsis* thaliana/soybean common codon usage bias, is set forth as SEQ ID NO: 43 in the SEQUENCE LISTING.

The amino acid at position 372 of the MsHPPD amino acid sequence was mutated from the original phenylalanine (F) to alanine (A) and the amino acid at position 383 was mutated from the original phenylalanine (F) to tryptophan (W), to obtain the MsHPPDm-F372A-F383W amino acid sequence as set forth in SEQ ID NO: 44 in the SEQUENCE LISTING; the MsHPPDm-F372A-F383W-01 nucleotide sequence encoding the MsHPPDm-F372A-F383W amino acid sequence is set forth as SEQ ID NO: 45 in the SEQUENCE LISTING; and the MsHPPDm-F372A-F383W-02 nucleotide sequence encoding the MsHPPDm-F372A-F383W amino acid sequence, which was obtained based on the *Arabidopsis thaliana*/soybean common codon usage bias, is set forth as SEQ ID NO: 46 in the SEQUENCE LISTING.

### (3) Acquisition of mutant HPPDs (F372A and F383W) from Gossypium hirsutum

The amino acid sequence of wild-type *Gossypium hirsutum* HPPD (GsHPPD) is set forth as SEQ ID NO: 47 in the SEQUENCE LISTING; the GsHPPD-01 nucleotide sequence encoding the GsHPPD is set forth as SEQ ID NO: 48 in the SEQUENCE LISTING; and the GsHPPD-02 nucleotide sequence encoding the GsHPPD, which was obtained based on the *Arabidopsis thaliana*/soybean common codon usage bias, is set forth as SEQ ID NO: 49 in the SEQUENCE LISTING.

The amino acid at position 372 of the GsHPPD amino acid sequence was mutated from the original phenylalanine (F) to alanine (A), to obtain the GsHPPDm-F372A amino acid sequence as set forth in SEQ ID NO: 50 in the SEQUENCE LISTING; the GsHPPDm-F372A-01 nucleotide sequence encoding the GsHPPDm-F372A amino acid sequence is set forth as SEQ ID NO: 51 in the SEQUENCE LISTING; and the GsHPPDm-F372A-02 nucleotide sequence encoding the GsHPPDm-F372A amino acid sequence, which was obtained based on the *Arabidopsis thaliana*/soybean common codon usage bias, is set forth as SEQ ID NO: 52 in the SEQUENCE LISTING.

The amino acid at position 383 of the GsHPPD amino acid sequence was mutated from the original phenylalanine (F) to tryptophan (W), to obtain the GsHPPDm-F383W amino acid sequence as set forth in SEQ ID NO: 53 in the SEQUENCE LISTING; the GsHPPDm-F383W-01 nucleotide sequence encoding the GsHPPDm-F383W amino acid sequence is set forth as SEQ ID NO: 54 in the SEQUENCE LISTING; and the GsHPPDm-F383W-02 nucleotide sequence encoding the GsHPPDm-F383W amino acid sequence, which was obtained based on the *Arabidopsis thaliana*/soybean common codon usage bias, is set forth as SEQ ID NO: 55 in the SEQUENCE LISTING.

The amino acid at position 372 of the GsHPPD amino acid sequence was mutated from the original phenylalanine (F) to alanine (A) and the amino acid at position 383 was mutated from the original phenylalanine (F) to tryptophan (W), to obtain the GsHPPDm-F372A-F383W amino acid sequence as set forth in SEQ ID NO: 56 in the SEQUENCE LISTING; the GsHPPDm-F372A-F383W-01 nucleotide sequence encoding the GsHPPDm-F372A-F383W amino acid sequence is set forth as SEQ ID NO: 57 in the SEQUENCE LISTING; and the GsHPPDm-F372A-F383W-02 nucleotide sequence encoding the GsHPPDm-F372A-F383W amino acid sequence, which was obtained based on the Arabidopsis thaliana/soybean common codon usage bias, is set forth as SEQ ID NO: 58 in the SEQUENCE LISTING.

### (4) Acquisition of mutant HPPDs (F372A and F383W) from Brassica napus

The amino acid sequence of the wild-type *Brassica napus* HPPD (BnHPPD) is set forth as SEQ ID NO: 59 in the SEQUENCE LISTING; the BnHPPD-01 nucleotide sequence encoding the BnHPPD is set forth as SEQ ID NO: 60 in the SEQUENCE LISTING; and the BnHPPD-02 nucleotide sequence encoding the BnHPPD, which was obtained based on the *Arabidopsis* thaliana/soybean common codon usage bias, is set forth as SEQ ID NO: 61 in the SEQUENCE LISTING.

The amino acid at position 372 of the BnHPPD amino acid sequence was mutated from the original phenylalanine (F) to alanine (A), to obtain the BnHPPDm-F372A amino acid sequence as set forth in SEQ ID NO: 62 in the SEQUENCE LISTING; the BnHPPDm-F372A-01 nucleotide sequence encoding the BnHPPDm-F372A amino acid sequence is set forth as SEQ ID NO: 63 in the SEQUENCE LISTING; and the BnHPPDm-F372A-02 nucleotide sequence encoding the BnHPPDm-F372A amino acid sequence, which was obtained based on the *Arabidopsis thaliana*/soybean common codon usage bias, is set forth as SEQ ID NO: 64 in the SEQUENCE LISTING.

The amino acid at position 383 of the BnHPPD amino acid sequence was mutated from the original phenylalanine (F) to tryptophan (W), to obtain the BnHPPDm-F383W amino acid sequence as set forth in SEQ ID NO: 65 in the SEQUENCE LISTING; the BnHPPDm-F383W-01 nucleotide sequence encoding the BnHPPDm-F383W amino acid sequence is set forth as SEQ ID NO: 66 in the SEQUENCE LISTING; and the BnHPPDm-F383W-02 nucleotide sequence encoding the BnHPPDm-F383W amino acid sequence, which was obtained based on the *Arabidopsis thaliana*/soybean common codon usage bias, is set forth as SEQ ID NO: 67 in the SEQUENCE LISTING.

The amino acid at position 372 of the BnHPPD amino acid sequence was mutated from the original phenylalanine (F) to alanine (A) and the amino acid at position 383 was mutated from the original phenylalanine (F) to tryptophan (W), to obtain the BnHPPDm-F372A-F383W amino acid sequence as set forth in SEQ ID NO: 68 in the SEQUENCE LISTING; the BnHPPDm-F372A-F383W-01 nucleotide sequence encoding the BnHPPDm-F372A-F383W amino acid sequence is set forth as SEQ ID NO: 69 in the SEQUENCE LISTING; and the BnHPPDm-F372A-F383W-02 nucleotide sequence encoding the BnHPPDm-F372A-F383W amino acid sequence, which was obtained based on the *Arabidopsis thaliana*/soybean common codon usage bias, is set forth as SEQ ID NO: 70 in the SEQUENCE LISTING.

### (5) Acquisition of mutant HPPDs (F372A and F383W) from Glycine max

The amino acid sequence of the wild-type *Glycine max* HPPD (GmHPPD) is set forth as SEQ ID NO: 71 in the SEQUENCE LISTING; the GmHPPD-01 nucleotide sequence encoding the GmHPPD is set forth as SEQ ID NO: 72 in the SEQUENCE LISTING; and the GmHPPD-02 nucleotide sequence encoding the GmHPPD, which was obtained based on the *Arabidopsis thaliana*/soybean common codon usage bias, is set forth as SEQ ID NO: 73 in the SEQUENCE LISTING.

The amino acid at position 372 of the GmHPPD amino acid sequence was mutated from the original phenylalanine (F) to alanine (A), to obtain the GmHPPDm-F372A amino acid sequence as set forth in SEQ ID NO: 74 in the SEQUENCE LISTING; the GmHPPDm-F372A-01 nucleotide sequence encoding the GmHPPDm-F372A amino acid sequence is set forth as SEQ ID NO: 75 in the SEQUENCE LISTING; and the GmHPPDm-F372A-02 nucleotide sequence encoding the GmHPPDm-F372A amino acid sequence, which was obtained based on the *Arabidopsis thaliana*/soybean common codon usage bias, is set forth as SEQ ID NO: 76 in the SEQUENCE LISTING.

The amino acid at position 383 of the GmHPPD amino acid sequence was mutated from the original original phenylalanine (F) to tryptophan (W), to obtain the GmHPPDm-F383W amino acid sequence as set forth in SEQ ID NO: 77 in the SEQUENCE LISTING; the GmHPPDm-F383W-01 nucleotide sequence encoding the GmHPPDm-F383W amino acid sequence is set forth as SEQ ID NO: 78 in the SEQUENCE LISTING; and the GmHPPDm-F383W-02 nucleotide sequence encoding the GmHPPDm-F383W amino acid sequence, which was obtained based on the *Arabidopsis thaliana*/soybean common codon usage bias, is set forth as SEQ ID NO: 79 in the SEQUENCE LISTING.

The amino acid at position 372 of the GmHPPD amino acid sequence was mutated from the original phenylalanine (F) to alanine (A) and the amino acid at position 383 was mutated from the original phenylalanine (F) to tryptophan (W), to obtain the GmHPPDm-F372A-F383W amino acid sequence as set forth in SEQ ID NO: 80 in the SEQUENCE LISTING; the GmHPPDm-F372A-F383W-01 nucleotide sequence encoding the GmHPPDm-F372A-F383W amino acid sequence is set forth as SEQ ID NO: 81 in the SEQUENCE LISTING; and the GmHPPDm-F372A-F383W-02 nucleotide sequence encoding the GmHPPDm-F372A-F383W amino acid sequence, which was obtained based on the *Arabidopsis thaliana*/soybean common codon usage bias, is set forth as SEQ ID NO: 82 in the SEQUENCE LISTING.

### (6) Acquisition of mutant HPPDs (F372A + F383W) from Nicotiana tabacum

The amino acid sequence of the wild-type *Nicotiana tabacum* HPPD (NtHPPD) is set forth as SEQ ID NO: 83 in the SEQUENCE LISTING; the NtHPPD-01 nucleotide sequence encoding the NtHPPD is set forth as SEQ ID NO: 84 in the SEQUENCE LISTING; and the NtHPPD-02 nucleotide sequence encoding the NtHPPD, which was obtained based on the *Arabidopsis thaliana*/soybean common codon usage bias, is set forth as SEQ ID NO: 85 in the SEQUENCE LISTING.

The amino acid at position 372 of the NtHPPD amino acid sequence was mutated from the original phenylalanine (F) to alanine (A), to obtain the NtHPPDm-F372A amino acid sequence as set forth in SEQ ID NO: 86 in the SEQUENCE LISTING; the NtHPPDm-F372A-01 nucleotide sequence encoding the NtHPPDm-F372A amino acid sequence is set forth as SEQ ID NO: 87 in the SEQUENCE LISTING; and the NtHPPDm-F372A-02 nucleotide sequence encoding the NtHPPDm-F372A amino acid sequence, which was obtained based on the *Arabidopsis thaliana*/soybean common codon usage bias, is set forth as SEQ ID NO: 88 in the SEQUENCE LISTING.

The amino acid at position 383 of the NtHPPD amino acid sequence was mutated from the original phenylalanine (F) to tryptophan (W), to obtain the NtHPPDm-F383W amino acid sequence as set forth in SEQ ID NO: 89 in the SEQUENCE LISTING; the NtHPPDm-F383W-01 nucleotide sequence encoding the NtHPPDm-F383W amino acid sequence is set forth as SEQ ID NO: 90 in the SEQUENCE LISTING; and the NtHPPDm-F383W-02 nucleotide sequence encoding the NtHPPDm-F383W amino acid sequence, which was obtained based on the *Arabidopsis thaliana*/soybean common codon usage bias, is set forth as SEQ ID NO: 91 in the SEQUENCE LISTING.

The amino acid at position 372 of the NtHPPD amino acid sequence was mutated from the original phenylalanine (F) to alanine (A) and the amino acid at position 383 was mutated from the original phenylalanine (F) to tryptophan (W), to obtain the NtHPPDm-F372A-F383W amino acid sequence as set forth in SEQ ID NO: 92 in the SEQUENCE LISTING; the NtHPPDm-F372A-F383W-01 nucleotide sequence encoding the NtHPPDm-F372A-F383W amino acid sequence is set forth as SEQ ID NO: 93 in the SEQUENCE LISTING; and the NtHPPDm-F372A-F383W-02 nucleotide sequence encoding the NtHPPDm-F372A-F383W amino acid sequence, which was obtained based on the *Arabidopsis thaliana*/soybean common codon usage bias, is set forth as SEQ ID NO: 94 in the SEQUENCE LISTING.

### (7) Acquisition of mutant HPPDs (F372A + F383W) from Oryza sativa

The amino acid sequence of the wild-type *Oryza sativa* HPPD (OsHPPD) is set forth as SEQ ID NO: 95 in the SEQUENCE LISTING; the OsHPPD-01 nucleotide sequence encoding the OsHPPD is set forth as SEQ ID NO: 96 in the SEQUENCE LISTING; and the OsHPPD-02 nucleotide sequence encoding the OsHPPD, which was obtained based on the *Arabidopsis thaliana*/soybean common codon usage bias, is set forth as SEQ ID NO: 97 in the SEQUENCE LISTING.

The amino acid at position 372 of the OsHPPD amino acid sequence was mutated from the original phenylalanine (F) to alanine (A), to obtain the OsHPPDm-F372A amino acid sequence as set forth in SEQ ID NO: 98 in the SEQUENCE LISTING; and the OsHPPDm-F372A-01 nucleotide sequence encoding the OsHPPDm-F372A amino acid sequence is set forth as SEQ ID NO: 99 in the SEQUENCE LISTING; and the OsHPPDm-F372A-02 nucleotide sequence encoding the OsHPPDm-F372A amino acid sequence, which was obtained based on the *Arabidopsis thaliana*/soybean common codon usage bias, is set forth as SEQ ID NO: 100 in the SEQUENCE LISTING.

The amino acid at position 383 of the OsHPPD amino acid sequence was mutated from the original phenylalanine (F) to tryptophan (W), to obtain the OsHPPDm-F383W amino acid sequence as set forth in SEQ ID NO: 101 in the SEQUENCE LISTING; the OsHPPDm-F383W-01 nucleotide sequence encoding the OsHPPDm-F383W amino acid sequence is set forth as SEQ ID NO: 102 in the SEQUENCE LISTING; and the OsHPPDm-F383W-02 nucleotide sequence encoding the OsHPPDm-F383W amino acid sequence, which was obtained based on the *Arabidopsis thaliana*/soybean common codon usage bias, is set forth as SEQ ID NO: 103 in the SEQUENCE LISTING.

The amino acid at position 372 of the OsHPPD amino acid sequence was mutated from the original phenylalanine (F) to alanine (A) and the amino acid at position 383 was mutated from the original phenylalanine (F) to tryptophan (W), to obtain the OsHPPDm-F372A-F383W amino acid sequence as set forth in SEQ ID NO: 104 in the SEQUENCE LISTING. The OsHPPDm-F372A-F383W-01 nucleotide sequence encoding the OsHPPDm-F372A-F383W amino acid sequence is set forth as SEQ ID NO: 105 in the SEQUENCE LISTING. The OsHPPDm-F372A-F383W-02 nucleotide sequence encoding the OsHPPDm-F372A-F383W amino acid sequence, which was obtained based on the *Arabidopsis thaliana*/soybean common codon usage bias, is set forth as SEQ ID NO: 106 in the SEQUENCE LISTING.

### (8) Acquisition of mutant HPPDs (F372A + F383W) from Sorghum bicolor

The amino acid sequence of the wild-type *Sorghum bicolorHPPD* (SbHPPD) is set forth as SEQ ID NO: 107 in the SEQUENCE LISTING; the SbHPPD-01 nucleotide sequence encoding the SbHPPD is set forth as SEQ ID NO: 108 in the SEQUENCE LISTING; and the SbHPPD-02 nucleotide sequence encoding the SbHPPD, which was obtained based on the *Arabidopsis thaliana*/soybean common codon usage bias, is set forth as SEQ ID NO: 109 in the SEQUENCE LISTING.

The amino acid at position 372 of the SbHPPD amino acid sequence was mutated from the original phenylalanine (F) to alanine (A), to obtain the SbHPPDm-F372A amino acid sequence as set forth in SEQ ID NO: 110 in the SEQUENCE LISTING. The SbHPPDm-F372A-01 nucleotide sequence encoding the SbHPPDm-F372A amino acid sequence is set forth as SEQ ID NO: 111 in the SEQUENCE LISTING. The SbHPPDm-F372A-02 nucleotide sequence encoding the SbHPPDm-F372A amino acid sequence, which was obtained based on the *Arabidopsis thaliana*/soybean common codon usage bias, is set forth as SEQ ID NO: 112 in the SEQUENCE LISTING.

The amino acid at position 383 of the SbHPPD amino acid sequence was mutated from the original phenylalanine (F) to tryptophan (W), to obtain the SbHPPDm-F383W amino acid sequence as set forth in SEQ ID NO: 113 in the SEQUENCE LISTING. The SbHPPDm-F383W-01 nucleotide sequence encoding the SbHPPDm-F383W amino acid sequence is set forth as SEQ ID NO: 114 in the SEQUENCE LISTING. The SbHPPDm-F383W-02 nucleotide sequence encoding the SbHPPDm-F383W amino acid sequence, which was obtained based on the *Arabidopsis thaliana*/soybean common codon usage bias, is set forth as SEQ ID NO: 115 in the SEQUENCE LISTING.

The amino acid at position 372 of the SbHPPD amino acid sequence was mutated from the original phenylalanine (F) to alanine (A) and the amino acid at position 383 was mutated from the original phenylalanine (F) to tryptophan (W), to obtain the SbHPPDm-F372A-F383W amino acid sequence as set forth in SEQ ID NO: 116 in the SEQUENCE LISTING. The SbHPPDm-F372A-F383W-01 ucleotide sequence encoding the SbHPPDm-F372A-F383W amino acid sequence is set forth as SEQ ID NO: 117 in the SEQUENCE LISTING. The SbHPPDm-F372A-F383W-02 nucleotide sequence encoding the SbHPPDm-F372A-F383W amino acid sequence, which was obtained based on the *Arabidopsis thaliana*/soybean common codon usage bias, is set forth as SEQ ID NO: 118 in the SEQUENCE LISTING.

### (9) Acquisition of mutant HPPDs (F372A + F383W) from Hordeum vulgare

The amino acid sequence of the wild-type *Hordeum vulgare*HPPD (HvHPPD) is set forth as SEQ ID NO: 119 in the SEQUENCE LISTING; the HvHPPD-01 nucleotide sequence encoding the HvHPPD is set forth as SEQ ID NO: 120 in the SEQUENCE LISTING; and the HvHPPD-02 nucleotide sequence encoding the HvHPPD, which was obtained based on the *Arabidopsis thaliana*/soybean common codon usage bias, is set forth as SEQ ID NO: 121 in the SEQUENCE LISTING.

The amino acid at position 372 of the HvHPPD amino acid sequence was mutated from the original phenylalanine (F) to alanine (A), to obtain the HvHPPDm-F372A amino acid sequence as set forth in SEQ ID NO: 122 in the SEQUENCE LISTING; the HvHPPDm-F372A-01 nucleotide sequence encoding the HvHPPDm-F372A amino acid sequence is set forth as SEQ ID NO: 123 in the SEQUENCE LISTING; and the HvHPPDm-F372A-02 nucleotide sequence encoding the HvHPPDm-F372A amino acid sequence, which was obtained based on the *Arabidopsis* thaliana/soybean common codon usage bias, is set forth as SEQ ID NO: 124 in the SEQUENCE LISTING.

The amino acid at position 383 of the HvHPPD amino acid sequence was mutated from the original phenylalanine (F) to tryptophan (W), to obtain the HvHPPDm-F383W amino acid sequence as set forth in SEQ ID NO: 125 in the SEQUENCE LISTING; the HvHPPDm-F383W-01 nucleotide sequence encoding the HvHPPDm-F383W amino acid sequence is set forth as SEQ ID NO: 126 in the SEQUENCE LISTING; and the HvHPPDm-F383W-02 nucleotide sequence encoding the HvHPPDm-F383W amino acid sequence, which was obtained based on the *Arabidopsis thaliana*/soybean common codon usage bias, is set forth as SEQ ID NO: 127 in the SEQUENCE LISTING.

The amino acid at position 372 of the HvHPPD amino acid sequence was mutated from the original phenylalanine (F) to alanine (A) and the amino acid at position 383 was mutated from the original phenylalanine (F) to tryptophan (W), to obtain the HvHPPDm-F372A-F383W amino acid sequence as set forth in SEQ ID NO: 128 in the SEQUENCE LISTING; the HvHPPDm-F372A-F383W-01 nucleotide sequence encoding the HvHPPDm-F372A-F383W amino acid sequence is set forth as SEQ ID NO: 129 in the SEQUENCE LISTING; and the HvHPPDm-F372A-F383W-02 nucleotide sequence encoding the HvHPPDm-F372A-F383W amino acid sequence, which was obtained based on the *Arabidopsis thaliana*/soybean common codon usage bias, is set forth as SEQ ID NO: 130 in the SEQUENCE LISTING.

### (10) Acquisition of mutant HPPDs (F372A + F383W) from Zea mays

The amino acid sequence of the wild-type *Zea mays* HPPD (ZmHPPD) is set forth as SEQ ID NO: 131 in the SEQUENCE LISTING; the ZmHPPD-01 nucleotide sequence encoding the ZmHPPD is set forth as SEQ ID NO: 132 in the SEQUENCE LISTING; and the ZmHPPD-02 nucleotide sequence encoding the ZmHPPD, which was obtained based on the *Arabidopsis thaliana*/soybean common codon usage bias, is set forth as SEQ ID NO: 133 in the SEQUENCE LISTING.

The amino acid at position 372 of the ZmHPPD amino acid sequence was mutated from the original phenylalanine (F) to alanine (A), to obtain the ZmHPPDm-F372A amino acid sequence as set forth in SEQ ID NO: 134 in the SEQUENCE LISTING; the ZmHPPDm-F372A-01 nucleotide sequence encoding the ZmHPPDm-F372A amino acid sequence is set forth as SEQ ID NO: 135 in the SEQUENCE LISTING; and the ZmHPPDm-F372A-02 nucleotide sequence encoding the ZmHPPDm-F372A amino acid sequence, which was obtained based on the *Arabidopsis thaliana*/soybean common codon usage bias, is set forth as SEQ ID NO: 136 in the SEQUENCE LISTING.

The amino acid at position 383 of the ZmHPPD amino acid sequence was mutated from the original phenylalanine (F) to tryptophan (W), to obtain the ZmHPPDm-F383W amino acid sequence as set forth in SEQ ID NO: 137 in the SEQUENCE LISTING; the ZmHPPDm-F383W-01 nucleotide sequence encoding the ZmHPPDm-F383W amino acid sequence is set forth as SEQ ID NO: 138 in the SEQUENCE LISTING; and the ZmHPPDm-F383W-02 nucleotide sequence encoding the ZmHPPDm-F383W amino acid sequence, which was obtained based on the *Arabidopsis thaliana*/soybean common codon usage bias, is set forth as SEQ ID NO: 139 in the SEQUENCE LISTING.

The amino acid at position 372 of the ZmHPPD amino acid sequence was mutated from the original phenylalanine (F) to alanine (A) and the amino acid at position 383 was mutated from the original phenylalanine (F) to tryptophan (W), to obtain the ZmHPPDm-F372A-F383W amino acid sequence as set forth in SEQ ID NO: 140 in the SEQUENCE LISTING; the ZmHPPDm-F372A-F383W-01 nucleotide sequence encoding the ZmHPPDm-F372A-F383W amino acid sequence is set forth as SEQ ID NO: 141 in the SEQUENCE LISTING; and the ZmHPPDm-F372A-F383W-02 nucleotide sequence encoding the ZmHPPDm-F372A-F383W amino acid sequence, which was obtained based on the *Arabidopsis thaliana*/soybean common codon usage bias, is set forth as SEQ ID NO: 142 in the SEQUENCE LISTING.

### (11) Acquisition of mutant HPPDs (F372A+F383W) from Pseudomonas fluorescens

The amino acid sequence of the wild-type *Pseudomonas fluorescens* HPPD (PfHPPD) is set forth as SEQ ID NO: 143 in the SEQUENCE LISTING; the PfHPPD-01 nucleotide sequence encoding the Pfl-IPPD is set forth as SEQ ID NO: 144 in the SEQUENCE LISTING; and the PfHPPD-02 nucleotide sequence encoding the PfHPPD, which was obtained based on the *Arabidopsis thaliana*/soybean common codon usage bias, is set forth as SEQ ID NO: 145 in the SEQUENCE LISTING.

The amino acid at position 372 of the PfHPPD amino acid sequence was mutated from the original phenylalanine (F) to alanine (A), to obtain the PfHPPDm-F372A amino acid sequence as set forth in SEQ ID NO: 146 in the SEQUENCE LISTING; the PfHPPDm-F372A-01 nucleotide sequence encoding the PfHPPDm-F372A amino acid sequence is set forth as SEQ ID NO: 147 in the SEQUENCE LISTING; and the PfHPPDm-F372A-02 nucleotide sequence encoding the PfHPPDm-F372A amino acid sequence, which was obtained based on the *Arabidopsis thaliana*/soybean common codon usage bias, is set forth as SEQ ID NO: 148 in the SEQUENCE LISTING.

The amino acid at position 383 of the PfHPPD amino acid sequence was mutated from the original phenylalanine (F) to tryptophan (W), to obtain the PfHPPDm-F383W amino acid sequence as set forth in SEQ ID NO: 149 in the SEQUENCE LISTING; the PfHPPDm-F383W-01 nucleotide sequence encoding the PfHPPDm-F383W amino acid sequence is set forth as SEQ ID NO: 150 in the SEQUENCE LISTING; and the PfHPPDm-F383W-02 nucleotide sequence encoding the PfHPPDm-F383W amino acid sequence, which was obtained based on the *Arabidopsis thaliana*/soybean common codon usage bias, is set forth as SEQ ID NO: 151 in the SEQUENCE LISTING.

The amino acid at position 372 of the PfHPPD amino acid sequence was mutated from the original phenylalanine (F) to alanine (A) and the amino acid at position 383 was mutated from the original phenylalanine (F) to tryptophan (W), to obtain the PfHPPDm-F372A-F383W amino acid sequence as set forth in SEQ ID NO: 152 in the SEQUENCE LISTING; the PfHPPDm-F372A-F383W-01 nucleotide sequence encoding the PfHPPDm-F372A-F383W amino acid sequence is set forth as SEQ ID NO: 153 in the SEQUENCE LISTING; and the PfHPPDm-F372A-F383W-02 nucleotide sequence encoding the PfHPPDm-F372A-F383W amino acid sequence, which was obtained based on the *Arabidopsis thaliana*/soybean common codon usage bias, is set forth as SEQ ID NO: 154 in the SEQUENCE LISTING.

### 2. Construction of recombinant expression vectors containing mutant HPPDs (with a combinatotial mutation of F372A+F383W, a single position mutation F372A or a single position mutation F383W) from different species for Arabidopsis thaliana

According to the method of constructing the recombinant expression vector DBN11726 containing the AsHPPDm-F372A-F383W-02 nucleotide sequence as described above in point 3 of Example 1, the AtHPPD-02 nucleotide sequence, AtHPPDm-F372A-02 nucleotide sequence, AtHPPDm-F383W-02 nucleotide sequence, AtHPPDm-F372A-F383W-02 nucleotide sequence, MsHPPD-02 nucleotide sequence, MsHPPDm-F372A-02 nucleotide sequence, MsHPPDm-F383W-02 nucleotide sequence, MsHPPDm-F372A-F383W-02 nucleotide sequence, GsHPPD-02 nucleotide sequence, GsHPPDm-F372A-02 nucleotide sequence, GsHPPDm-F383W-02 nucleotide sequence, GsHPPDm-F372A-F383W-02 nucleotide sequence, BnHPPD-02 nucleotide sequence, BnHPPDm-F372A-02 nucleotide sequence, BnHPPDm-F383W-02 nucleotide sequence, BnHPPDm-F372A-F383W-02 nucleotide sequence, GmHPPD-02 nucleotide sequence, GmHPPDm-F372A-02 nucleotide sequence, GmHPPDm-F383W-02 nucleotide sequence, GmHPPDm-F372A-F383W-02 nucleotide sequence, NtHPPD-02 nucleotide sequence, NtHPPDm-F372A-02 nucleotide sequence, NtHPPDm-F383W-02 nucleotide sequence, NtHPPDm-F372A-F383W-02 nucleotide sequence, OsHPPD-02 nucleotide sequence, OsHPPDm-F372A-02 nucleotide sequence, OsHPPDm-F383W-02 nucleotide sequence, OsHPPDm-F372A-F383W-02 nucleotide sequence, SbHPPD-02 nucleotide sequence, SbHPPDm-F372A-02 nucleotide sequence, SbHPPDm-F383W-02 nucleotide sequence, SbHPPDm-F372A-F383W-02 nucleotide sequence, HvHPPD-02 nucleotide sequence, HvHPPDm-F372A-02 nucleotide sequence, HvHPPDm-F383W-02 nucleotide sequence, HvHPPDm-F372A-F383W-02 nucleotide sequence, ZmHPPD-02 nucleotide sequence, ZmHPPDm-F372A-02 nucleotide sequence, ZmHPPDm-F383W-02 nucleotide sequence, ZmHPPDm-F372A-F383W-02 nucleotide sequence, PfHPPD-02 nucleotide sequence, PfHPPDm-F372A-02 nucleotide sequence, PfHPPDm-F383W-02 nucleotide sequence, and PfHPPDm-F372A-F383W-02 nucleotide sequence which were linked to the universal adapter primer 1 was respectively subjected to a recombination reaction with the linearized DBNBC-01 expression vector backbone to obtain the recombinant expression vectors DBN11730 to DBN11773 in sequence. Sequencing verified that the aforementioned nucleotide sequences were inserted correctly in the recombinant expression vectors DBN11730 to DBN11773.

### 3. Transformation of Agrobacterium with the recombinant expression vectors for Arabidopsis thaliana

According to the method of transformation of *Agrobacterium* with the recombinant expression vectors for *Arabidopsis thalianaas* described above in point 4 of Example 1, the recombinant expression vectors DBN11730 to DBN11773 which had been correctly constructed, and the control recombinant expression vector DBN11726N which had been constructed in point 3 of Example 1, were transformed into the *Agrobacterium* GV3101 respectively using a liquid nitrogen method, and the results were verified by sequencing, showing that the structures of the recombinant expression vectors DBN11730 to DBN11773 and DBN11726N were completely correct.

### 4. Detection of the herbicide tolerance of the Arabidopsis thaliana plants into which the mutant HPPDs (with a combinatotial mutation of F372A+F383W, a single position mutation F372A or a single position mutation F383W) from different species were introduced

According to the method as described above in point 5 of Example 1, *Arabidopsis thaliana* inflorescences were immersed in the *Agrobacterium* solution as described in Example 3 so as to introduce the T-DNA in the recombinant expression vectors DBN11730 to DBN11773 constructed in this Example 2 and the control recombinant expression vector DBN11726N constructed in point 3 of Example 1 into the *Arabidopsis thaliana* chromosomes, thereby obtaining the corresponding transgenic *Arabidopsis thaliana* plants, i.e., *Arabidopsis thaliana* T1 plants (AtHPPD-02) into which the AtHPPD-02 nucleotide sequence was introduced, *Arabidopsis thaliana* T1 plants (AtHPPDm-F372A-02) into which the AtHPPDm-F372A-02 nucleotide sequence was introduced, *Arabidopsis thaliana* T1 plants (AtHPPDm-F383W-02) into which the AtHPPDm-F383W-02 nucleotide sequence was introduced, *Arabidopsis thaliana* T1 plants (AtHPPDm-F372A-F383W-02) into which the AtHPPDm-F372A-F383W-02 nucleotide sequence was introduced, *Arabidopsis thaliana* T1 plants (MsHPPD-02) into which the MsHPPD-02 nucleotide sequence was introduced, *Arabidopsis thaliana* T1 plants (MsHPPDm-F372A-02) into which the MsHPPDm-F372A-02 nucleotide sequence was introduced, *Arabidopsis thaliana* T1 plants (MsHPPDm-F383W-02) into which the MsHPPDm-F383W-02 nucleotide sequence was introduced, *Arabidopsis thaliana* T1 plants (MsHPPDm-F372A-F383W-02) into which the MsHPPDm-F372A-F383W-02 nucleotide sequence was introduced, *Arabidopsis thaliana* T1 plants (GsHPPD-02) into which the GsHPPD-02 nucleotide sequence was introduced, *Arabidopsis thaliana* T1 plants (GsHPPDm-F372A-02) into which the GsHPPDm-F372A-02 nucleotide sequence was introduced, *Arabidopsis thaliana* T1 plants (GsHPPDm-F383W-02) into which the GsHPPDm-F383W-02 nucleotide sequence was introduced, *Arabidopsis thaliana* T1 plants (GsHPPDm-F372A-F383W-02) into which the GsHPPDm-F372A-F383W-02 nucleotide sequence was introduced, *Arabidopsis thaliana* T1 plants (BnHPPD-02) into which the BnHPPD-02 nucleotide sequence was introduced, *Arabidopsis thaliana* T1 plants (BnHPPDm-F372A-02) into which the BnHPPDm-F372A-02 nucleotide sequence was introduced, *Arabidopsis thaliana* T1 plants (BnHPPDm-F383W-02) into which the BnHPPDm-F383W-02 nucleotide sequence was introduced, *Arabidopsis thaliana* T1 plants (BnHPPDm-F372A-F383W-02) into which the BnHPPDm-F372A-F383W-02 nucleotide sequence was introduced, *Arabidopsis thaliana* T1 plants (GmHPPD-02) into which the GmHPPD-02 nucleotide sequence was introduced, *Arabidopsis thaliana* T1 plants (GmHPPDm-F372A-02) into which the GmHPPDm-F372A-02 nucleotide sequence was introduced, *Arabidopsis thaliana* T1 plants (GmHPPDm-F383W-02) into which the GmHPPDm-F383W-02 nucleotide sequence was introduced, *Arabidopsis thaliana* T1 plants (GmHPPDm-F372A-F383W-02) into which the GmHPPDm-F372A-F383W-02 nucleotide sequence was introduced, *Arabidopsis thaliana* T1 plants (NtHPPD-02) into which the NtHPPD-02 nucleotide sequence was introduced, *Arabidopsis thaliana* T1 plants (NtHPPDm-F372A-02) into which the NtHPPDm-F372A-02 nucleotide sequence was introduced, *Arabidopsis thaliana* T1 plants (NtHPPDm-F383W-02) into which the NtHPPDm-F383W-02 nucleotide sequence was introduced, *Arabidopsis thaliana* T1 plants (NtHPPDm-F372A-F383W-02) into which the NtHPPDm-F372A-F383W-02 nucleotide sequence was introduced, *Arabidopsis thaliana* T1 plants (OsHPPD-02) into which the OsHPPD-02 nucleotide sequence was introduced, *Arabidopsis thaliana* T1 plants (OsHPPDm-F372A-02) into which the OsHPPDm-F372A-02 nucleotide sequence was introduced, *Arabidopsis thaliana* T1 plants (OsHPPDm-F383W-02) into which the OsHPPDm-F383W-02 nucleotide sequence was introduced, *Arabidopsis thaliana* T1 plants (OsHPPDm-F372A-F383W-02) into which the OsHPPDm-F372A-F383W-02 nucleotide sequence was introduced, *Arabidopsis thaliana* T1 plants (SbHPPD-02) into which the SbHPPD-02 nucleotide sequence was introduced, *Arabidopsis thaliana* T1 plants (SbHPPDm-F372A-02) into which the SbHPPDm-F372A-02 nucleotide sequence was introduced, *Arabidopsis thaliana* T1 plants (SbHPPDm-F383W-02) into which the SbHPPDm-F383W-02 nucleotide sequence was introduced, *Arabidopsis thaliana* T1 plants (SbHPPDm-F372A-F383W-02) into which the SbHPPDm-F372A-F383W-02 nucleotide sequence was introduced, *Arabidopsis thaliana* T1 plants (HvHPPD-02) into which the HvHPPD-02 nucleotide sequence was introduced, *Arabidopsis thaliana* T1 plants (HvHPPDm-F372A-02) into which the HvHPPDm-F372A-02 nucleotide sequence was introduced, *Arabidopsis thaliana* T1 plants (HvHPPDm-F383W-02) into which the HvHPPDm-F383W-02 nucleotide sequence was introduced, *Arabidopsis thaliana* T1 plants (HvHPPDm-F372A-F383W-02) into which the HvHPPDm-F372A-F383W-02 nucleotide sequence was introduced, *Arabidopsis thaliana* T1 plants (ZmHPPD-02) into which the ZmHPPD-02 nucleotide sequence was introduced, *Arabidopsis thaliana* T1 plants (ZmHPPDm-F372A-02) into which the ZmHPPDm-F372A-02 nucleotide sequence was introduced, *Arabidopsis thaliana* T1 plants (ZmHPPDm-F383W-02) into which the ZmHPPDm-F383W-02 nucleotide sequence was introduced, *Arabidopsis thaliana* T1 plants (ZmHPPDm-F372A-F383W-02) into which the ZmHPPDm-F372A-F383W-02 nucleotide sequence was introduced, *Arabidopsis thaliana* T1 plants (PfHPPD-02) into which the PfHPPD-02 nucleotide sequence was introduced, *Arabidopsis thaliana* T1 plants (Pfl-IPPDm-F372A-02) into which the PfHPPDm-F372A-02 nucleotide sequence was introduced, *Arabidopsis thaliana* T1 plants (PfHPPDm-F383W-02) into which the PfHPPDm-F383W-02 nucleotide sequence was introduced, *Arabidopsis thaliana* T1 plants (PfHPPDm-F372A-F383W-02) into which the PfHPPDm-F372A-F383W-02 nucleotide sequence was introduced, and *Arabidopsis thaliana* T1 plants (DBN11726N) into which the control recombinant expression vector DBN11726N was introduced.

According to the method as described above in point 6 of Example 1, the aforementioned *Arabidopsis thaliana* T1 plants and wild-type *Arabidopsis thaliana* plants (CK) (18 days after sowing) were sprayed respectively with topramezone at three concentrations (25 g ai/ha (one-fold field concentration, 1×), 100 g ai/ha (four-fold field concentration, 4×), and 0 g ai/ha (water, 0×)), isoxaflutole at three concentrations (35 g ai/ha (half-fold field concentration, 0.5×), 70 g ai/ha (one-fold field concentration, 1×), and 0 g ai/ha (water, 0×)), and mesotrione at three concentrations (52.5 g ai/ha (half-fold field concentration, 0.5×), 105 g ai/ha (one-fold field concentration, 1×), and 0 g ai/ha (water, 0×)) to detect the tolerance of *Arabidopsis thaliana* to the herbicides. The experimental results are shown in TABLES 4 to 6.

**TABLE 4 Topramezone tolerance of Arabidopsis thaliana T₁ plants into which the mutant HPPDs from different species were introduced**

| Source of the gene | *Arabidopsis thaliana* genotypes | Concentrati on (g ai/ha) | Classification and statistics of the grade of pesticide damage | | | | Scores | Resistance evaluation |
|---|---|---|---|---|---|---|---|---|
| | | | Grade 0 | Grade 1 | Grade 2 | Grade 3 | | |
| | CK | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | DBN11726N | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| *Arabidopsis thaliana* | AtHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | AtHPPDm-F372A-F383W-02 | 0 | 16 | 0 | 0 | 0 | 0 | |
| | | 25 | 9 | 7 | 0 | 0 | 15 | highly resistant |
| | | 100 | 6 | 7 | 1 | 2 | 31 | moderately resistant |
| | AtHPPDm-F372A-02 | 0 | 16 | 0 | 0 | 0 | 0 | |
| | | 25 | 4 | 4 | 8 | 0 | 42 | poorly resistant |
| | | 100 | 0 | 0 | 4 | 12 | 92 | non-resistant |
| | AtHPPDm-F383W-02 | 0 | 16 | 0 | 0 | 0 | 0 | |
| | | 25 | 0 | 2 | 5 | 9 | 81 | non-resistant |
| | | 100 | 0 | 0 | 2 | 14 | 96 | non-resistant |
| *Medicago sativa* | MsHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | MsHPPDm-F372A-F383W-02 | 0 | 16 | 0 | 0 | 0 | 0 | |
| | | 25 | 6 | 9 | 1 | 0 | 23 | moderately resistant |
| | | 100 | 0 | 8 | 6 | 2 | 54 | poorly resistant |
| | MsHPPDm-F372A-02 | 0 | 16 | 0 | 0 | 0 | 0 | |
| | | 25 | 5 | 7 | 4 | 0 | 31 | moderately resistant |
| | | 100 | 0 | 4 | 9 | 3 | 65 | poorly resistant |
| | MsHPPDm-F383W-02 | 0 | 16 | 0 | 0 | 0 | 0 | |
| | | 25 | 0 | 0 | 4 | 12 | 92 | non-resistant |
| | | 100 | 0 | 0 | 0 | 16 | 100 | non-resistant |
| *Gossypium hirsutum* | GsHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | GsHPPDm-F372A-F383W-02 | 0 | 16 | 0 | 0 | 0 | 0 | |
| | | 25 | 16 | 0 | 0 | 0 | 0 | highly resistant |
| | | 100 | 5 | 7 | 2 | 2 | 35 | poorly resistant |
| | GsHPPDm-F372A-02 | 0 | 16 | 0 | 0 | 0 | 0 | |
| | | 25 | 16 | 0 | 0 | 0 | 0 | highly resistant |
| | | 100 | 0 | 0 | 0 | 16 | 100 | non-resistant |
| | GsHPPDm-F383W-02 | 0 | 16 | 0 | 0 | 0 | 0 | |
| | | 25 | 0 | 3 | 9 | 4 | 69 | non-resistant |
| | | 100 | 0 | 0 | 0 | 16 | 100 | non-resistant |
| *Brassica napus* | BnHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | BnHPPDm-F372A-F383W-02 | 0 | 16 | 0 | 0 | 0 | 0 | |
| | | 25 | 6 | 6 | 4 | 0 | 29 | moderately resistant |
| | | 100 | 3 | 8 | 5 | 0 | 38 | poorly resistant |
| | BnHPPDm-F372A-02 | 0 | 16 | 0 | 0 | 0 | 0 | |
| | | 25 | 0 | 4 | 12 | 0 | 58 | poorly resistant |
| | | 100 | 0 | 0 | 0 | 16 | 100 | non-resistant |
| | BnHPPDm-F383W-02 | 0 | 16 | 0 | 0 | 0 | 0 | |
| | | 25 | 0 | 3 | 9 | 4 | 69 | non-resistant |
| | | 100 | 0 | 0 | 0 | 16 | 100 | non-resistant |
| *Glycine max* | GmHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | GmHPPDm-F372A-F383W-02 | 0 | 16 | 0 | 0 | 0 | 0 | |
| | | 25 | 9 | 7 | 0 | 0 | 15 | highly resistant |
| | | 100 | 5 | 6 | 4 | 1 | 35 | poorly resistant |
| | GmHPPDm-F372A-02 | 0 | 16 | 0 | 0 | 0 | 0 | |
| | | 25 | 0 | 12 | 4 | 0 | 42 | poorly resistant |
| | | 100 | 0 | 0 | 0 | 16 | 100 | non-resistant |
| | GmHPPDm-F383W-02 | 0 | 16 | 0 | 0 | 0 | 0 | |
| | | 25 | 0 | 3 | 9 | 4 | 69 | non-resistant |
| | | 100 | 0 | 0 | 0 | 16 | 100 | non-resistant |
| *Nicotiana tabacum* | NtHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | NtHPPDm-F372A-F383W-02 | 0 | 16 | 0 | 0 | 0 | 0 | |
| | | 25 | 2 | 13 | 1 | 0 | 31 | moderately resistant |
| | | 100 | 0 | 1 | 13 | 2 | 69 | non-resistant |
| | NtHPPDm-F372A-02 | 0 | 16 | 0 | 0 | 0 | 0 | |
| | | 25 | 0 | 6 | 10 | 0 | 54 | poorly resistant |
| | | 100 | 0 | 0 | 0 | 16 | 100 | non-resistant |
| | NtHPPDm-F383W-02 | 0 | 16 | 0 | 0 | 0 | 0 | |
| | | 25 | 0 | 0 | 10 | 6 | 79 | non-resistant |
| | | 100 | 0 | 0 | 0 | 16 | 100 | non-resistant |
| *Oryza sativa* | OsHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | OsHPPDm-F372A-F383W-02 | 0 | 16 | 0 | 0 | 0 | 0 | |
| | | 25 | 10 | 6 | 0 | 0 | 13 | highly resistant |
| | | 100 | 2 | 13 | 1 | 0 | 31 | moderately resistant |
| | OsHPPDm-F372A-02 | 0 | 16 | 0 | 0 | 0 | 0 | |
| | | 25 | 8 | 8 | 0 | 0 | 17 | moderately resistant |
| | | 100 | 0 | 0 | 0 | 16 | 100 | non-resistant |
| | OsHPPDm-F383W-02 | 0 | 16 | 0 | 0 | 0 | 0 | |
| | | 25 | 0 | 3 | 9 | 4 | 69 | non-resistant |
| | | 100 | 0 | 0 | 0 | 16 | 100 | non-resistant |
| *Sorghum bicolor* | SbHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | SbHPPDm-F372A-F383W-02 | 0 | 16 | 0 | 0 | 0 | 0 | |
| | | 25 | 16 | 0 | 0 | 0 | 0 | highly resistant |
| | | 100 | 9 | 7 | 0 | 0 | 15 | highly resistant |
| | SbHPPDm-F372A-02 | 0 | 16 | 0 | 0 | 0 | 0 | |
| | | 25 | 16 | 0 | 0 | 0 | 0 | highly resistant |
| | | 100 | 4 | 4 | 8 | 0 | 42 | poorly resistant |
| | SbHPPDm-F383W-02 | 0 | 16 | 0 | 0 | 0 | 0 | |
| | | 25 | 7 | 6 | 3 | 0 | 25 | moderately resistant |
| | | 100 | 0 | 0 | 0 | 16 | 100 | non-resistant |
| *Hordeum vulgare* | HvHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | HvHPPDm-F372A-F383W-02 | 0 | 16 | 0 | 0 | 0 | 0 | |
| | | 25 | 16 | 0 | 0 | 0 | 0 | highly resistant |
| | | 100 | 10 | 6 | 0 | 0 | 13 | highly resistant |
| | HvHPPDm-F372A-02 | 0 | 16 | 0 | 0 | 0 | 0 | |
| | | 25 | 15 | 1 | 0 | 0 | 2 | highly resistant |
| | | 100 | 8 | 8 | 0 | 0 | 17 | moderately resistant |
| | HvHPPDm-F383W-02 | 0 | 16 | 0 | 0 | 0 | 0 | |
| | | 25 | 4 | 6 | 5 | 1 | 40 | poorly resistant |
| | | 100 | 0 | 0 | 0 | 16 | 100 | non-resistant |
| *Zea mays* | ZmHPPD-02 | 0 | 16 | 0 | 0 | 0 | 0 | |
| | | 25 | 0 | 0 | 0 | 16 | 100 | non-resistant |
| | | 100 | 0 | 0 | 0 | 16 | 100 | non-resistant |
| | ZmHPPDm-F372A-F383W-02 | 0 | 16 | 0 | 0 | 0 | 0 | |
| | | 25 | 16 | 0 | 0 | 0 | 0 | highly resistant |
| | | 100 | 10 | 5 | 1 | 0 | 15 | highly resistant |
| | ZmHPPDm-F372A-02 | 0 | 16 | 0 | 0 | 0 | 0 | |
| | | 25 | 16 | 0 | 0 | 0 | 0 | highly resistant |
| | | 100 | 4 | 12 | 0 | 0 | 25 | moderately resistant |
| | ZmHPPDm-F383W-02 | 0 | 16 | 0 | 0 | 0 | 0 | |
| | | 25 | 2 | 9 | 5 | 0 | 40 | poorly resistant |
| | | 100 | 0 | 4 | 7 | 5 | 69 | non-resistant |

The results of TABLE 4 show that (1) as compared with the *Arabidopsis thaliana* plants into which unmutated HPPD genes were introduced, the *Arabidopsis thaliana* plants into which the HPPD genes with the combinatorial mutation at positions 372 and 383 (F372A+F383W) from different species and HPPD genes with the single mutation at position 372 (F372A) from different species were introduced, had different degrees of tolerance to topramezone, and only the HPPD genes with the single mutation at position 383 (F383W) from some species (*Sorghum bicolor, Hordeum vulgare* and *Zea mays*) could confer tolerance to topramezone upon the *Arabidopsis thaliana* plants, while the CK plants and control vector DBN11726N plants had no tolerance to topramezone.
(2) From the perspective of resistance evaluation, as to topramezone, the *Arabidopsis thaliana* plants into which the HPPD genes with the combinatorial mutation at positions 372 and 383 (F372A+F383W) from different species (except *Medicago sativa*) were introduced, exhibited better herbicide tolerance than the *Arabidopsis thaliana* plants into which the HPPD genes with the single position mutation F372A or F383Wwere introduced, and further achieved a synergistically enhanced effect of herbicide tolerance.
(3) From the perspective of scores, the *Arabidopsis thaliana* plants into which the HPPD genes with the combinatorial mutation at positions 372 and 383 (F372A+F383W) from *Medicago sativa* were introduced, had lower tolerance scores than those into which the HPPD genes with the single position mutation F372A or F383W were introduced. Furthermore, when treated with topramezone at four-fold field concentration, about 50% of the *Arabidopsis thaliana* plants into which the HPPD genes with the combinatorial mutation at positions 372 and 383 from *Medicago sativa* were introduced, had a damage level of grade 0 or grade 1; 25% of the *Arabidopsis thaliana* plants into which the HPPD genes with the single mutation at position 372 from *Medicago sativa* were introduced, had a damage level of grade 0 or grade 1; and the number of the *Arabidopsis thaliana* plants having a damage level of grade 0 or 1 in the *Arabidopsis thaliana* plants into which the HPPD genes with the single mutation at position 383 from *Medicago sativa* were introduced, was 0. This shows that HPPD genes with the combinatorial mutation at positions 372 and 383 (F372A+F383W) from *Medicago sativa* could confer a synergistically enhanced herbicide tolerance upon the *Arabidopsis thaliana* plants.

**TABLE 5 Isoxaflutole tolerance of Arabidopsis thaliana T₁ plants into which the mutated HPPDs from different species sources were introduced**

| Source of the gene | *Arabidopsis thaliana* genotypes | Concentratio n (g ai/ha) | Classification and statistics of the grade of pesticide damage | | | | Scores | Resistance evaluation |
|---|---|---|---|---|---|---|---|---|
| | | | Grad e 0 | Grad e 1 | Grad e2 | Grad e 3 | | |
| | CK | 0 | 16 | 0 | 0 | 0 | 0 | |
| | | 35 | 0 | 0 | 0 | 16 | 100 | Non-resistant |
| | | 70 | 0 | 0 | 0 | 16 | 100 | Non-resistant |
| | DBN11726N | 0 | 16 | 0 | 0 | 0 | 0 | |
| | | 35 | 0 | 0 | 0 | 16 | 100 | Non-resistant |
| | | 70 | 0 | 0 | 0 | 16 | 100 | Non-resistant |
| *Arabidops is thaliana* | AtHPPD-02 | 0 | 16 | 0 | 0 | 0 | 0 | |
| | | 35 | 0 | 0 | 0 | 16 | 100 | Non-resistant |
| | | 70 | 0 | 0 | 0 | 16 | 100 | Non-resistant |
| | AtHPPDm-F372A-F383W-02 | 0 | 16 | 0 | 0 | 0 | 0 | |
| | | 35 | 10 | 5 | 1 | 0 | 15 | Highly resistant |
| | | 70 | 5 | 9 | 1 | 1 | 29 | Moderately resistant |
| | AtHPPDm-F372A-02 | 0 | 16 | 0 | 0 | 0 | 0 | |
| | | 35 | 0 | 9 | 5 | 2 | 52 | Poorly resistant |
| | | 70 | 0 | 0 | 2 | 14 | 96 | Non-resistant |
| | AtHPPDm-F383W-02 | 0 | 16 | 0 | 0 | 0 | 0 | |
| | | 35 | 0 | 2 | 7 | 7 | 77 | Non-resistant |
| | | 70 | 0 | 0 | 0 | 16 | 100 | Non-resistant |
| *Medicago sativa* | MsHPPD-02 | 0 | 16 | 0 | 0 | 0 | 0 | |
| | | 35 | 0 | 0 | 0 | 16 | 100 | Non-resistant |
| | | 70 | 0 | 0 | 0 | 16 | 100 | Non-resistant |
| | MsHPPDm-F372A-F383W-02 | 0 | 16 | 0 | 0 | 0 | 0 | |
| | | 35 | 5 | 9 | 2 | 0 | 27 | Moderately resistant |
| | | 70 | 2 | 12 | 2 | 0 | 33 | Moderately resistant |
| | MsHPPDm-F372A-02 | 0 | 16 | 0 | 0 | 0 | 0 | |
| | | 35 | 0 | 6 | 10 | 0 | 54 | Poorly resistant |
| | | 70 | 0 | 0 | 0 | 16 | 100 | Non-resistant |
| | MsHPPDm-F383W-02 | 0 | 16 | 0 | 0 | 0 | 0 | |
| | | 35 | 0 | 0 | 6 | 10 | 88 | Non-resistant |
| | | 70 | 0 | 0 | 0 | 16 | 100 | Non-resistant |
| *Gossypiu* m *hirsutum* | GsHPPD-02 | 0 | 16 | 0 | 0 | 0 | 0 | |
| | | 35 | 0 | 0 | 0 | 16 | 100 | Non-resistant |
| | | 70 | 0 | 0 | 0 | 16 | 100 | Non-resistant |
| | GsHPPDm-F372A-F383W-02 | 0 | 16 | 0 | 0 | 0 | 0 | |
| | | 35 | 9 | 7 | 0 | 0 | 15 | Highly resistant |
| | | 70 | 3 | 5 | 6 | 2 | 48 | Poorly resistant |
| | GsHPPDm-F372A-02 | 0 | 16 | 0 | 0 | 0 | 0 | |
| | | 35 | 3 | 10 | 3 | 0 | 33 | Moderately resistant |
| | | 70 | 0 | 0 | 0 | 16 | 100 | Non-resistant |
| | GsHPPDm-F383W-02 | 0 | 16 | 0 | 0 | 0 | 0 | |
| | | 35 | 0 | 2 | 9 | 5 | 73 | Non-resistant |
| | | 70 | 0 | 0 | 0 | 16 | 100 | Non-resistant |
| *Brassica napus* | BnHPPD-02 | 0 | 16 | 0 | 0 | 0 | 0 | |
| | | 35 | 0 | 0 | 0 | 16 | 100 | Non-resistant |
| | | 70 | 0 | 0 | 0 | 16 | 100 | Non-resistant |
| | BnHPPDm-F372A-F383W-02 | 0 | 16 | 0 | 0 | 0 | 0 | |
| | | 35 | 5 | 8 | 3 | 0 | 29 | Moderately resistant |
| | | 70 | 3 | 10 | 3 | 0 | 33 | Moderately resistant |
| | BnHPPDm-F372A-02 | 0 | 16 | 0 | 0 | 0 | 0 | |
| | | 35 | 0 | 7 | 9 | 0 | 52 | Poorly resistant |
| | | 70 | 0 | 0 | 0 | 16 | 100 | Non-resistant |
| | BnHPPDm-F383W-02 | 0 | 16 | 0 | 0 | 0 | 0 | |
| | | 35 | 0 | 0 | 3 | 13 | 94 | Non-resistant |
| | | 70 | 0 | 0 | 0 | 16 | 100 | Non-resistant |
| *Glycine max* | GmHPPD-02 | 0 | 16 | 0 | 0 | 0 | 0 | |
| | | 35 | 0 | 0 | 0 | 16 | 100 | Non-resistant |
| | | 70 | 0 | 0 | 0 | 16 | 100 | Non-resistant |
| | GmHPPDm-F372A-F383W-02 | 0 | 16 | 0 | 0 | 0 | 0 | |
| | | 35 | 9 | 7 | 0 | 0 | 15 | Highly resistant |
| | | 70 | 4 | 6 | 5 | 1 | 40 | Poorly resistant |
| | GmHPPDm-F372A-02 | 0 | 16 | 0 | 0 | 0 | 0 | |
| | | 35 | 0 | 3 | 11 | 2 | 65 | Poorly resistant |
| | | 70 | 0 | 0 | 0 | 16 | 100 | Non-resistant |
| | GmHPPDm-F383W-02 | 0 | 16 | 0 | 0 | 0 | 0 | |
| | | 35 | 0 | 0 | 9 | 7 | 81 | Non-resistant |
| | | 70 | 0 | 0 | 0 | 16 | 100 | Non-resistant |
| *Nicotiana tabacum* | NtHPPD-02 | 0 | 16 | 0 | 0 | 0 | 0 | |
| | | 35 | 0 | 0 | 0 | 16 | 100 | Non-resistant |
| | | 70 | 0 | 0 | 0 | 16 | 100 | Non-resistant |
| | NtHPPDm-F372A-F383W-02 | 0 | 16 | 0 | 0 | 0 | 0 | |
| | | 35 | 1 | 14 | 1 | 0 | 33 | Moderately resistant |
| | | 70 | 0 | 3 | 11 | 2 | 65 | Poorly resistant |
| | NtHPPDm-F372A-02 | 0 | 16 | 0 | 0 | 0 | 0 | |
| | | 35 | 0 | 6 | 10 | 0 | 54 | Poorly resistant |
| | | 70 | 0 | 0 | 0 | 16 | 100 | Non-resistant |
| | NtHPPDm-F383W-02 | 0 | 16 | 0 | 0 | 0 | 0 | |
| | | 35 | 0 | 0 | 6 | 10 | 88 | Non-resistant |
| | | 70 | 0 | 0 | 0 | 16 | 100 | Non-resistant |
| *Oryza sativa* | OsHPPD-02 | 0 | 16 | 0 | 0 | 0 | 0 | |
| | | 35 | 0 | 0 | 0 | 16 | 100 | Non-resistant |
| | | 70 | 0 | 0 | 0 | 16 | 100 | Non-resistant |
| | OsHPPDm-F372A-F383W-02 | 0 | 16 | 0 | 0 | 0 | 0 | |
| | | 35 | 11 | 5 | 0 | 0 | 10 | Highly resistant |
| | | 70 | 9 | 7 | 0 | 0 | 15 | Highly resistant |
| | OsHPPDm-F372A-02 | 0 | 16 | 0 | 0 | 0 | 0 | |
| | | 35 | 0 | 8 | 7 | 1 | 52 | Poorly resistant |
| | | 70 | 0 | 0 | 0 | 16 | 100 | Non-resistant |
| | OsHPPDm-F383W-02 | 0 | 16 | 0 | 0 | 0 | 0 | |
| | | 35 | 0 | 0 | 3 | 13 | 94 | Non-resistant |
| | | 70 | 0 | 0 | 0 | 16 | 100 | Non-resistant |
| *Sorghum bicolor* | SbHPPD-02 | 0 | 16 | 0 | 0 | 0 | 0 | |
| | | 35 | 0 | 0 | 0 | 16 | 100 | Non-resistant |
| | | 70 | 0 | 0 | 0 | 16 | 100 | Non-resistant |
| | SbHPPDm-F372A-F383W-02 | 0 | 16 | 0 | 0 | 0 | 0 | |
| | | 35 | 12 | 4 | 0 | 0 | 8 | Highly resistant |
| | | 70 | 10 | 6 | 0 | 0 | 13 | Highly resistant |
| | SbHPPDm-F372A-02 | 0 | 16 | 0 | 0 | 0 | 0 | |
| | | 35 | 0 | 2 | 12 | 2 | 67 | Poorly resistant |
| | | 70 | 0 | 0 | 0 | 16 | 100 | Non-resistant |
| | SbHPPDm-F383W-02 | 0 | 16 | 0 | 0 | 0 | 0 | |
| | | 35 | 0 | 1 | 15 | 0 | 65 | Poorly resistant |
| | | 70 | 0 | 0 | 0 | 16 | 100 | Non-resistant |
| *Hordeum vulgare* | HvHPPD-02 | 0 | 16 | 0 | 0 | 0 | 0 | |
| | | 35 | 0 | 0 | 0 | 16 | 100 | Non-resistant |
| | | 70 | 0 | 0 | 0 | 16 | 100 | Non-resistant |
| | HvHPPDm-F372A-F383W-02 | 0 | 16 | 0 | 0 | 0 | 0 | |
| | | 35 | 4 | 12 | 0 | 0 | 25 | Moderately resistant |
| | | 70 | 1 | 14 | 1 | 0 | 33 | Moderately resistant |
| | HvHPPDm-F372A-02 | 0 | 16 | 0 | 0 | 0 | 0 | |
| | | 35 | 1 | 14 | 1 | 0 | 33 | Moderately resistant |
| | | 70 | 0 | 12 | 3 | 1 | 44 | Poorly resistant |
| | HvHPPDm-F383W-02 | 0 | 16 | 0 | 0 | 0 | 0 | |
| | | 35 | 0 | 1 | 14 | 1 | 67 | Poorly resistant |
| | | 70 | 0 | 0 | 0 | 16 | 100 | Non-resistant |
| *Zea mays* | ZmHPPD-02 | 0 | 16 | 0 | 0 | 0 | 0 | |
| | | 35 | 0 | 0 | 0 | 16 | 100 | Non-resistant |
| | | 70 | 0 | 0 | 0 | 16 | 100 | Non-resistant |
| | ZmHPPDm-F372A-F383W-02 | 0 | 16 | 0 | 0 | 0 | 0 | |
| | | 35 | 5 | 10 | 1 | 0 | 25 | Moderately resistant |
| | | 70 | 3 | 8 | 5 | 0 | 38 | Poorly resistant |
| | ZmHPPDm-F372A-02 | 0 | 16 | 0 | 0 | 0 | 0 | |
| | | 35 | 3 | 10 | 3 | 0 | 33 | Moderately resistant |
| | | 70 | 0 | 1 | 14 | 1 | 67 | Poorly resistant |
| | ZmHPPDm-F383W-02 | 0 | 16 | 0 | 0 | 0 | 0 | |
| | | 35 | 2 | 8 | 6 | 0 | 42 | Poorly resistant |
| | | 70 | 0 | 0 | 2 | 14 | 96 | Non-resistant |

The results of TABLE 5 show that (1) as compared with the *Arabidopsis thaliana* plants into which unmutated HPPD genes were introduced, the *Arabidopsis thaliana* plants into which the HPPD genes with the combinatorial mutation at positions 372 and 383 (F372A+F383W) from different species and HPPD genes with the single mutation at position 372 (F372A) from different species were introduced, had different degrees of tolerance to isoxaflutole, and only the HPPD genes with the single mutation at position 383 (F383W) from some species (*Sorghum bicolor, Hordeum vulgare* and *Zea mays*) can confer tolerance to isoxaflutole upon the *Arabidopsis thaliana* plants, while the CK plants and the control vector DBN11726N plants had no tolerance to isoxaflutole.
(2) From the perspective of resistance evaluation, as to isoxaflutole, the *Arabidopsis thaliana* plants into which the HPPD genes with the combinatorial mutation at positions 372 and 383 (F372A+F383W) from different species (except *Zea mays*) were introduced, exhibited better herbicide tolerance than the *Arabidopsis thaliana* plants into which the HPPD genes with the single position mutation F372A or F383W were introduced, and further achieved a synergistically enhanced effect of herbicide tolerance.
(3) From the perspective of scores, the *Arabidopsis thaliana* plants into which the HPPD genes with the combinatorial mutation at positions 372 and 383 (F372A+F383W) from *Zea mays* were introduced, had lower tolerance scores than those into which the HPPD genes with the single position mutation F372A or F383W were introduced. Furthermore, when treated with isoxaflutole at one-fold field concentration, about 69% of the *Arabidopsis thaliana* plants into which the HPPD genes with the combinatorial mutation at positions 372 and 383 from *Zea mays* were introduced, had a damage level of grade 0 or grade 1; 6% of the *Arabidopsis thaliana* plants into which the HPPD genes with the single mutation at position 372 from *Zea mays* were introduced, had a damage level of grade 0 or grade 1; and the number of the *Arabidopsis thaliana* plants having a damage level of grade 0 or grade 1 in the *Arabidopsis thaliana* plants into which the HPPD genes with the single mutation at position 383 from *Zea mays* were introduced, was 0. This shows that HPPD genes with the combinatorial mutation at positions 372 and 383 (F372A+F383W) from *Zea mays* can confer confer a synergistically enhanced herbicide tolerance upon the *Arabidopsis thaliana* plants.

**TABLE 6 Mesotrione tolerance of Arabidopsis thaliana T₁ plants into which the mutated HPPDs from different species sources were introduced**

| Source of the gene | *Arabidopsis thaliana* genotypes | Concentrati on (g ai/ha) | Classification and statistics of the grade of pesticide damage | | | | Sco res | Resistance evaluation |
|---|---|---|---|---|---|---|---|---|
| | | | Grad e 0 | Grad e 1 | Grad e 2 | Grade 3 | | |
| | CK | 0 | 16 | 0 | 0 | 0 | 0 | |
| | | 52.5 | 0 | 0 | 0 | 16 | 100 | Non-resistant |
| | | 105 | 0 | 0 | 0 | 16 | 100 | Non-resistant |
| | DBN11726N | 0 | 16 | 0 | 0 | 0 | 0 | |
| | | 52.5 | 0 | 0 | 0 | 16 | 100 | Non-resistant |
| | | 105 | 0 | 0 | 0 | 16 | 100 | Non-resistant |
| *Arabidopsi s thaliana* | AtHPPD-02 | 0 | 16 | 0 | 0 | 0 | 0 | |
| | | 52.5 | 0 | 0 | 0 | 16 | 100 | Non-resistant |
| | | 105 | 0 | 0 | 0 | 16 | 100 | Non-resistant |
| | AtHPPDm-F372A-F383W-02 | 0 | 16 | 0 | 0 | 0 | 0 | |
| | | 52.5 | 8 | 6 | 2 | 0 | 21 | Moderately resistant |
| | | 105 | 2 | 8 | 5 | 1 | 44 | Poorly resistant |
| | AtHPPDm-F372A-02 | 0 | 16 | 0 | 0 | 0 | 0 | |
| | | 52.5 | 0 | 5 | 8 | 3 | 63 | Poorly resistant |
| | | 105 | 0 | 0 | 0 | 16 | 100 | Non-resistant |
| | AtHPPDm-F383W-02 | 0 | 16 | 0 | 0 | 0 | 0 | |
| | | 52.5 | 0 | 0 | 0 | 16 | 100 | Non-resistant |
| | | 105 | 0 | 0 | 0 | 16 | 100 | Non-resistant |
| *Medicago sativa* | MsHPPD-02 | 0 | 16 | 0 | 0 | 0 | 0 | |
| | | 52.5 | 0 | 0 | 0 | 16 | 100 | Non-resistant |
| | | 105 | 0 | 0 | 0 | 16 | 100 | Non-resistant |
| | MsHPPDm-F372A-F383W-02 | 0 | 16 | 0 | 0 | 0 | 0 | |
| | | 52.5 | 3 | 10 | 3 | 0 | 33 | Moderately resistant |
| | | 105 | 0 | 0 | 10 | 6 | 79 | Non-resistant |
| | MsHPPDm-F372A-02 | 0 | 16 | 0 | 0 | 0 | 0 | |
| | | 52.5 | 0 | 9 | 5 | 2 | 52 | Poorly resistant |
| | | 105 | 0 | 0 | 0 | 16 | 100 | Non-resistant |
| | MsHPPDm-F383W-02 | 0 | 16 | 0 | 0 | 0 | 0 | |
| | | 52.5 | 0 | 0 | 0 | 16 | 100 | Non-resistant |
| | | 105 | 0 | 0 | 0 | 16 | 100 | Non-resistant |
| *Gossypium hirsutum* | GsHPPD-02 | 0 | 16 | 0 | 0 | 0 | 0 | |
| | | 52.5 | 0 | 0 | 0 | 16 | 100 | Non-resistant |
| | | 105 | 0 | 0 | 0 | 16 | 100 | Non-resistant |
| | GsHPPDm-F372A-F383W-02 | 0 | 16 | 0 | 0 | 0 | 0 | |
| | | 52.5 | 3 | 10 | 3 | 0 | 33 | Moderately resistant |
| | | 105 | 0 | 0 | 11 | 5 | 77 | Non-resistant |
| | GsHPPDm-F372A-02 | 0 | 16 | 0 | 0 | 0 | 0 | |
| | | 52.5 | 0 | 8 | 6 | 2 | 54 | Poorly resistant |
| | | 105 | 0 | 0 | 0 | 16 | 100 | Non-resistant |
| | GsHPPDm-F383W-02 | 0 | 16 | 0 | 0 | 0 | 0 | |
| | | 52.5 | 0 | 0 | 0 | 16 | 100 | Non-resistant |
| | | 105 | 0 | 0 | 0 | 16 | 100 | Non-resistant |
| *Brassica napus* | BnHPPD-02 | 0 | 16 | 0 | 0 | 0 | 0 | |
| | | 52.5 | 0 | 0 | 0 | 16 | 100 | Non-resistant |
| | | 105 | 0 | 0 | 0 | 16 | 100 | Non-resistant |
| | BnHPPDm-F372A-F383W-02 | 0 | 16 | 0 | 0 | 0 | 0 | |
| | | 52.5 | 5 | 8 | 2 | 1 | 31 | Moderately resistant |
| | | 105 | 0 | 13 | 3 | 0 | 40 | Poorly resistant |
| | BnHPPDm-F372A-02 | 0 | 16 | 0 | 0 | 0 | 0 | |
| | | 52.5 | 0 | 4 | 10 | 2 | 63 | Poorly resistant |
| | | 105 | 0 | 0 | 0 | 16 | 100 | Non-resistant |
| | BnHPPDm-F383W-02 | 0 | 16 | 0 | 0 | 0 | 0 | |
| | | 52.5 | 0 | 0 | 0 | 16 | 100 | Non-resistant |
| | | 105 | 0 | 0 | 0 | 16 | 100 | Non-resistant |
| *Glycine max* | GmHPPD-02 | 0 | 16 | 0 | 0 | 0 | 0 | |
| | | 52.5 | 0 | 0 | 0 | 16 | 100 | Non-resistant |
| | | 105 | 0 | 0 | 0 | 16 | 100 | Non-resistant |
| | GmHPPDm-F372A-F383W-02 | 0 | 16 | 0 | 0 | 0 | 0 | |
| | | 52.5 | 6 | 9 | 1 | 0 | 23 | Moderately resistant |
| | | 105 | 2 | 7 | 6 | 1 | 46 | Poorly resistant |
| | GmHPPDm-F372A-02 | 0 | 16 | 0 | 0 | 0 | 0 | |
| | | 52.5 | 0 | 2 | 12 | 2 | 67 | Poorly resistant |
| | | 105 | 0 | 0 | 0 | 16 | 100 | Non-resistant |
| | GmHPPDm-F383W-02 | 0 | 16 | 0 | 0 | 0 | 0 | |
| | | 52.5 | 0 | 0 | 0 | 16 | 100 | Non-resistant |
| | | 105 | 0 | 0 | 0 | 16 | 100 | Non-resistant |
| *Nicotiana tabacum* | NtHPPD-02 | 0 | 16 | 0 | 0 | 0 | 0 | |
| | | 52.5 | 0 | 0 | 0 | 16 | 100 | Non-resistant |
| | | 105 | 0 | 0 | 0 | 16 | 100 | Non-resistant |
| | NtHPPDm-F372A-F383W-02 | 0 | 16 | 0 | 0 | 0 | 0 | |
| | | 52.5 | 1 | 13 | 2 | 0 | 35 | Poorly resistant |
| | | 105 | 0 | 1 | 13 | 2 | 69 | Non-resistant |
| | NtHPPDm-F372A-02 | 0 | 16 | 0 | 0 | 0 | 0 | |
| | | 52.5 | 0 | 2 | 13 | 1 | 65 | Poorly resistant |
| | | 105 | 0 | 0 | 0 | 16 | 100 | Non-resistant |
| | NtHPPDm-F383W-02 | 0 | 16 | 0 | 0 | 0 | 0 | |
| | | 52.5 | 0 | 0 | 0 | 16 | 100 | Non-resistant |
| | | 105 | 0 | 0 | 0 | 16 | 100 | Non-resistant |
| *Oryza sativa* | OsHPPD-02 | 0 | 16 | 0 | 0 | 0 | 0 | |
| | | 52.5 | 0 | 0 | 0 | 16 | 100 | Non-resistant |
| | | 105 | 0 | 0 | 0 | 16 | 100 | Non-resistant |
| | OsHPPDm-F372A-F383W-02 | 0 | 16 | 0 | 0 | 0 | 0 | |
| | | 52.5 | 9 | 7 | 0 | 0 | 15 | Highly resistant |
| | | 105 | 0 | 14 | 2 | 0 | 38 | Poorly resistant |
| | OsHPPDm-F372A-02 | 0 | 16 | 0 | 0 | 0 | 0 | |
| | | 52.5 | 0 | 5 | 8 | 3 | 63 | Poorly resistant |
| | | 105 | 0 | 0 | 0 | 16 | 100 | Non-resistant |
| | OsHPPDm-F383W-02 | 0 | 16 | 0 | 0 | 0 | 0 | |
| | | 52.5 | 0 | 0 | 7 | 9 | 85 | Non-resistant |
| | | 105 | 0 | 0 | 0 | 16 | 100 | Non-resistant |
| *Sorghum bicolor* | SbHPPD-02 | 0 | 16 | 0 | 0 | 0 | 0 | |
| | | 52.5 | 0 | 0 | 0 | 16 | 100 | Non-resistant |
| | | 105 | 0 | 0 | 0 | 16 | 100 | Non-resistant |
| | SbHPPDm-F372A-F383W-02 | 0 | 16 | 0 | 0 | 0 | 0 | |
| | | 52.5 | 10 | 6 | 0 | 0 | 13 | Highly resistant |
| | | 105 | 0 | 14 | 2 | 0 | 38 | Poorly resistant |
| | SbHPPDm-F372A-02 | 0 | 16 | 0 | 0 | 0 | 0 | |
| | | 52.5 | 0 | 6 | 7 | 3 | 60 | Poorly resistant |
| | | 105 | 0 | 0 | 0 | 16 | 100 | Non-resistant |
| | SbHPPDm-F383W-02 | 0 | 16 | 0 | 0 | 0 | 0 | |
| | | 52.5 | 0 | 5 | 5 | 6 | 69 | Non-resistant |
| | | 105 | 0 | 0 | 0 | 16 | 100 | Non-resistant |
| *Hordeum vulgare* | HvHPPD-02 | 0 | 16 | 0 | 0 | 0 | 0 | |
| | | 52.5 | 0 | 0 | 0 | 16 | 100 | Non-resistant |
| | | 105 | 0 | 0 | 0 | 16 | 100 | Non-resistant |
| | HvHPPDm-F372A-F383W-02 | 0 | 16 | 0 | 0 | 0 | 0 | |
| | | 52.5 | 12 | 4 | 0 | 0 | 8 | Highly resistant |
| | | 105 | 2 | 14 | 0 | 0 | 29 | Moderately resistant |
| | HvHPPDm-F372A-02 | 0 | 16 | 0 | 0 | 0 | 0 | |
| | | 52.5 | 9 | 7 | 0 | 0 | 15 | Highly resistant |
| | | 105 | 0 | 9 | 3 | 4 | 56 | Poorly resistant |
| | HvHPPDm-F383W-02 | 0 | 16 | 0 | 0 | 0 | 0 | |
| | | 52.5 | 2 | 12 | 2 | 0 | 33 | Moderately resistant |
| | | 105 | 0 | 0 | 0 | 16 | 100 | Non-resistant |
| *Zea mays* | ZmHPPD-02 | 0 | 16 | 0 | 0 | 0 | 0 | |
| | | 52.5 | 0 | 0 | 0 | 16 | 100 | Non-resistant |
| | | 105 | 0 | 0 | 0 | 16 | 100 | Non-resistant |
| | ZmHPPDm-F372A-F383W-02 | 0 | 16 | 0 | 0 | 0 | 0 | |
| | | 52.5 | 9 | 7 | 0 | 0 | 15 | Highly resistant |
| | | 105 | 3 | 10 | 3 | 0 | 33 | Moderately resistant |
| | ZmHPPDm-F372A-02 | 0 | 16 | 0 | 0 | 0 | 0 | |
| | | 52.5 | 3 | 11 | 2 | 0 | 31 | Moderately resistant |
| | | 105 | 0 | 1 | 14 | 1 | 67 | Poorly resistant |
| | ZmHPPDm-F383W-02 | 0 | 16 | 0 | 0 | 0 | 0 | |
| | | 52.5 | 0 | 3 | 12 | 1 | 63 | Poorly resistant |
| | | 105 | 0 | 0 | 1 | 15 | 98 | Non-resistant |
| *Pseudomo nas fluorescen* s | PfHPPD-02 | 0 | 16 | 0 | 0 | 0 | 0 | |
| | | 52.5 | 0 | 0 | 0 | 16 | 100 | Non-resistant |
| | | 105 | 0 | 0 | 0 | 16 | 100 | Non-resistant |
| | PfHPPDm-F372A-F383W-02 | 0 | 16 | 0 | 0 | 0 | 0 | |
| | | 52.5 | 7 | 8 | 1 | 0 | 21 | Moderately resistant |
| | | 105 | 5 | 6 | 5 | 0 | 33 | Moderately resistant |
| | PfHPPDm-F372A-02 | 0 | 16 | 0 | 0 | 0 | 0 | |
| | | 52.5 | 6 | 7 | 1 | 2 | 31 | Moderately resistant |
| | | 105 | 0 | 4 | 12 | 0 | 58 | Poorly resistant |
| | PfHPPDm-F383W-02 | 0 | 16 | 0 | 0 | 0 | 0 | |
| | | 52.5 | 0 | 0 | 11 | 5 | 77 | Non-resistant |
| | | 105 | 0 | 0 | 0 | 16 | 100 | Non-resistant |

The results of TABLE 6 show that (1) as compared with the *Arabidopsis thaliana* plants into which unmutated HPPD genes were introduced, the *Arabidopsis thaliana* plants into which the HPPD genes with the combinatorial mutation at positions 372 and 383 (F372A+F383W) from different species and HPPD genes with the single mutation at position 372 (F372A) from different species were introduced, had different degrees of tolerance to mesotrione, and only the HPPD genes with the single mutation at position 383 (F383W) from some species (*Hordeum vulgare and Zea mays*) can confer tolerance to mesotrione upon the *Arabidopsis thaliana* plants, while the CK plants and the control vector DBN11726N plants had no tolerance to mesotrione.
(2) From the perspective of resistance evaluation, as to mesotrione, the *Arabidopsis thaliana* plants into which the HPPD genes with the combinatorial mutation at positions 372 and 383 (F372A+F383W) from different species (except *Nicotiana tabacum*) were introduced, exhibited better herbicide tolerance than the *Arabidopsis thaliana* plants into which the HPPD genes with the single position mutation F372A or F383W were introduced, and further achieved a synergistically enhanced effect of herbicide tolerance.
(3) From the perspective of scores, when treated with mesotrione at half-fold or one-fold field concentration, the *Arabidopsis thaliana* plants into which the HPPD genes with the combinatorial mutation at positions 372 and 383 (F372A+F383W) from *Nicotiana tabacum* had lower tolerance scores than those of the single position mutation F372A or F383W, and further showed a synergistically enhanced effect of herbicide tolerance.

In view of the foregoing, the results of TABLEs 4-6 show that all the HPPD genes with the combinatorial mutation at positions 372 and 383 (F372A+F383W) from different species can confer a synergistically enhanced effect of herbicide tolerance upon the plants.

### Example 3: Different mutations at positions 372 and 383 (the combinatorial mutation F372G+F383W or F372V+F383W) of the HPPD amino acid sequence and verification of the mutation effects

### 1. Acquisition of the genes AsHPPDm-F372G-F383W and AsHPPDm-F372V-F383W

(1) The amino acid at position 372 of the AsHPPD amino acid sequence was mutated from the original phenylalanine (F) to glycine (G), to obtain the AsHPPDm-F372G amino acid sequence as set forth in SEQ ID NO: 155 in the SEQUENCE LISTING; the AsHPPDm-F372G-01 nucleotide sequence encoding the AsHPPDm-F372G amino acid sequence is set forth as SEQ ID NO: 156 in the SEQUENCE LISTING; and the AsHPPDm-F372G-02 nucleotide sequence encoding the AsHPPDm-F372G amino acid sequence, which was obtained based on the *Arabidopsis* thaliana/soybean common codon usage bias, is set forth as SEQ ID NO: 157 in the SEQUENCE LISTING. The amino acid at position 372 of the AsHPPD amino acid sequence was mutated from the original phenylalanine (F) to glycine (G), and the amino acid at position 383 was mutated from the original phenylalanine (F) to tryptophan (W), to obtain the AsHPPDm-F372G-F383W amino acid sequence as set forth in SEQ ID NO: 158 in the SEQUENCE LISTING; the AsHPPDm-F372G-F383W-01 nucleotide sequence encoding the AsHPPDm-F372G-F383W amino acid sequence is set forth as SEQ ID NO: 159 in the SEQUENCE LISTING; and the AsHPPDm-F372G-F383W-02 nucleotide sequence encoding the AsHPPDm-F372G-F383W amino acid sequence, which was obtained based on the *Arabidopsis thaliana*/soybean common codon usage bias, is set forth as SEQ ID NO: 160 in the SEQUENCE LISTING.
(2) The amino acid at position 372 of the AsHPPD amino acid sequence was mutated from the original phenylalanine (F) to valine (V), to obtain the AsHPPDm-F372V amino acid sequence as set forth in SEQ ID NO: 161 in the SEQUENCE LISTING; the AsHPPDm-F372V-01 nucleotide sequence encoding the AsHPPDm-F372V amino acid sequence is set forth as SEQ ID NO: 162 in the SEQUENCE LISTING; and the AsHPPDm-F372V-02 nucleotide sequence encoding the AsHPPDm-F372V amino acid sequence, which was obtained based on the *Arabidopsis thaliana*/soybean common codon usage bias, is set forth as SEQ ID NO: 163 in the SEQUENCE LISTING.

The amino acid at position 372 of the AsHPPD amino acid sequence was mutated from the original phenylalanine (F) to valine (V), and the amino acid at position 383 was mutated from the original phenylalanine (F) to tryptophan (W), to obtain the AsHPPDm-F372V-F383W amino acid sequence as set forth in SEQ ID NO: 164 in the SEQUENCE LISTING; the AsHPPDm-F372V-F383W-01 nucleotide sequence encoding the AsHPPDm-F372V-F383W amino acid sequence is set forth as SEQ ID NO: 165 in the SEQUENCE LISTING; and the AsHPPDm-F372V-F383W-02 nucleotide sequence encoding the AsHPPDm-F372V-F383W amino acid sequence, which was obtained based on the *Arabidopsis thaliana*/soybean common codon usage bias, is set forth as SEQ ID NO: 166 in the SEQUENCE LISTING.

### 2. Construction of recombinant expression vectors containing Avena sativa HPPD genes (372G+F383W or F372V+F383W) for Arabidopsis thaliana

According to the method of constructing the recombinant expression vector DBN11726 containing the AsHPPDm-F372A-F383W-02 nucleotide sequence as described above in point 3 of Example 1, the AsHPPDm-F372G-02 nucleotide sequence, AsHPPDm-F372G-F383W-02 nucleotide sequence, AsHPPDm-F372V-02 nucleotide sequence, and AsHPPDm-F372V-F383W-02 nucleotide sequence which were linked to the universal adapter primer 1 were respectively subjected to a recombination reaction with the linearized DBNBC-01 expression vector backbone to obtain the recombinant expression vectors DBN11774 to DBN11777 in sequence. Sequencing verified that the aforementioned nucleotide sequences were inserted correctly in the recombinant expression vectors DBN11774 to DBN11777.

### 3. Transformation of Agrobacterium with the recombinant expression vectors for Arabidopsis thaliana

According to the method of transforming *Agrobacterium* with the recombinant expression vectors for *Arabidopsis thaliana* as described above in point 4 of Example 1, the recombinant expression vectors DBN11774 to DBN11777 which had been constructed correctly, and the recombinant expression vector DBN11727 containing the AsHPPD-02 nucleotide sequence in point 3 of Example 1, the recombinant expression vector DBN11729 containing the AsHPPDm-F383W-02 nucleotide sequence in point 3 of Example 1, and the control recombinant expression vector DBN11726N constructed in point 3 of Example 1 were transformed into *Agrobacterium* GV3101 respectively using a liquid nitrogen method. The results were verified by sequencing, showing that the structures of the recombinant expression vectors DBN11774 to DBN11777 and DBN1172, DBN11729, and DBN11726N were completely correct.

### 4. Detection of the herbicide tolerance of the transgenic Arabidopsis thaliana plants into which the AsHPPDm-F372G-F383W-02 or AsHPPDm-F372V-F383W-02 nucleotide sequence was introduced

According to the method as described above in point 5 of Example 1, *Arabidopsis thaliana* inflorescences were immersed in the *Agrobacterium* solution as described in Example 3 so as to introduce the T-DNA in the recombinant expression vectors DBN11774 to DBN11777 constructed in Example 2, the recombinant expression vector DBN11727 containing the AsHPPD-02 nucleotide sequence in point 3 of Example 1, the recombinant expression vector DBN11729 containing the AsHPPDm-F383W-02 nucleotide sequence in point 3 of Example 1, and the control recombinant expression vector DBN11726N constructed in point 3 of Example 1 into the *Arabidopsis thaliana* chromosomes, thereby obtaining the corresponding transgenic *Arabidopsis thaliana* plants, i.e., *Arabidopsis thaliana* T₁ plants into which the AsHPPDm-F372G-02 nucleotide sequence was introduced (AsHPPDm-F372G-02), *Arabidopsis thaliana* T₁ plants into which the AsHPPDm-F372G-F383W-02 nucleotide sequence was introduced (AsHPPDm-F372G-F383W-02), *Arabidopsis thaliana* T₁ plants into which the AsHPPDm-F372V-02 nucleotide sequence was introduced (AsHPPDm-F372V-02), *Arabidopsis thaliana* T₁ plants into which the AsHPPDm-F372V-F383W-02 nucleotide sequence was introduced (AsHPPDm-F372V-F383W-02), *Arabidopsis thaliana* T₁ plants into which the AsHPPD-02 nucleotide sequence was introduced, *Arabidopsis thaliana* T₁ plants into which the AsHPPDm-F383W-02 nucleotide sequence was introduced, and *Arabidopsis thaliana* T₁ plants into which the control recombinant expression vector DBN11726N was introduced.

### 5. Verification of the synergistic effect of F372G+F383W or F372V+F383W

According to the method as described above in point 6 of Example 1, the aforementioned *Arabidopsis thaliana* T₁ plants and wild-type *Arabidopsis thaliana* plants (CK) (18 days after sowing) were sprayed with topramezone at three different concentrations (i.e., 100 g ai/ha (four-fold field concentration, 4×), 200 g ai/ha (eight-fold field concentration, 8×) and 0 g ai/ha (water, 0×)), isoxaflutole at three different concentrations (i.e., 140 g ai/ha (two-fold field concentration, 2×), 280 g ai/ha (four-fold field concentration, 4×) and 0 g ai/ha (water, 0×)), and mesotrione at three different concentrations (i.e., 210 g ai/ha (two-fold field concentration, 2×), 420 g ai/ha (four-fold field concentration, 4×) and 0 g ai/ha (water, 0×)) respectively to detect the herbicide tolerance of *Arabidopsis thaliana.* The experimental results are shown in TABLE 7 to TABLE 9.

**TABLE 7 Topramezone tolerance of transgenic Arabidopsis thaliana T₁ plants Arabidopsis thaliana genotypes**

| | Concentration (g ai/ha) | Classification and statistics of the grade of pesticide damage | | | | Scores | Resistance evaluation |
|---|---|---|---|---|---|---|---|
| | | Grade 0 | Grade 1 | Grade 2 | Grade 3 | | |
| CK | 0 | 16 | 0 | 0 | 0 | 0 | |
| | 100 | 0 | 0 | 0 | 16 | 100 | Non-resistant |
| | 200 | 0 | 0 | 0 | 16 | 100 | Non-resistant |
| DBN11726N | 0 | 16 | 0 | 0 | 0 | 0 | |
| | 100 | 0 | 0 | 0 | 16 | 100 | Non-resistant |
| | 200 | 0 | 0 | 0 | 16 | 100 | Non-resistant |
| AsHPPD-02 | 0 | 16 | 0 | 0 | 0 | 0 | |
| | 100 | 0 | 0 | 4 | 12 | 92 | Non-resistant |
| | 200 | 0 | 0 | 0 | 16 | 100 | Non-resistant |
| AsHPPDm-F372G-02 | 0 | 16 | 0 | 0 | 0 | 0 | |
| | 100 | 16 | 0 | 0 | 0 | 0 | Highly resistant |
| | 200 | 16 | 0 | 0 | 0 | 0 | Highly resistant |
| AsHPPDm-F383W-02 | 0 | 16 | 0 | 0 | 0 | 0 | |
| | 100 | 16 | 0 | 0 | 0 | 0 | Highly resistant |
| | 200 | 16 | 0 | 0 | 0 | 0 | Highly resistant |
| AsHPPDm-F372G-F383W-02 | 0 | 16 | 0 | 0 | 0 | 0 | |
| | 100 | 16 | 0 | 0 | 0 | 0 | Highly resistant |
| | 200 | 16 | 0 | 0 | 0 | 0 | Highly resistant |
| AsHPPDm-F372V-02 | 0 | 16 | 0 | 0 | 0 | 0 | |
| | 100 | 16 | 0 | 0 | 0 | 0 | Highly resistant |
| | 200 | 16 | 0 | 0 | 0 | 0 | Highly resistant |
| AsHPPDm-F372V-F383W-02 | 0 | 16 | 0 | 0 | 0 | 0 | |
| | 100 | 16 | 0 | 0 | 0 | 0 | Highly resistant |
| | 200 | 16 | 0 | 0 | 0 | 0 | Highly resistant |

The results of TABLE 7 show that as compared with CK, when treated with topramezone at four-fold or eight-fold field concentration, the *Arabidopsis thaliana* genotypes AsHPPDm-F372G-02, AsHPPDm-F383W-02, AsHPPDm-F372G-F383W-02, AsHPPDm-F372V-02, and AsHPPDm-F372V-F383W-02 all exhibited highly-resistant tolerance, while AsHPPD-02 and the control vector DBN11726N plants exhibited no tolerance to topramezone.

**TABLE 8 Isoxaflutole tolerance of transgenic Arabidopsis thaliana T₁ plants**

| *Arabidopsis thaliana* genotypes | Concentration (g ai/ha) | Classification and statistics of the grade of pesticide damage | | | | Scores | Resistance evaluation |
|---|---|---|---|---|---|---|---|
| | | Grade 0 | Grade 1 | Grade 2 | Grade 3 | | |
| CK | 0 | 16 | 0 | 0 | 0 | 0 | |
| | 140 | 0 | 0 | 0 | 16 | 100 | Non-resistant |
| | 280 | 0 | 0 | 0 | 16 | 100 | Non-resistant |
| DBN11726N | 0 | 16 | 0 | 0 | 0 | 0 | |
| | 140 | 0 | 0 | 0 | 16 | 100 | Non-resistant |
| | 280 | 0 | 0 | 0 | 16 | 100 | Non-resistant |
| AsHPPD-02 | 0 | 16 | 0 | 0 | 0 | 0 | |
| | 140 | 0 | 1 | 4 | 11 | 88 | Non-resistant |
| | 280 | 0 | 0 | 0 | 16 | 100 | Non-resistant |
| AsHPPDm-F372G-02 | 0 | 16 | 0 | 0 | 0 | 0 | |
| | 140 | 6 | 7 | 2 | 1 | 29 | Moderately resistant |
| | 280 | 0 | 0 | 16 | 0 | 67 | Poorly resistant |
| AsHPPDm-F383W-02 | 0 | 16 | 0 | 0 | 0 | 0 | |
| | 140 | 6 | 10 | 0 | 0 | 21 | Moderately resistant |
| | 280 | 4 | 4 | 8 | 0 | 42 | Poorly resistant |
| AsHPPDm-F372G-F383W-02 | 0 | 16 | 0 | 0 | 0 | 0 | |
| | 140 | 16 | 0 | 0 | 0 | 0 | Highly resistant |
| | 280 | 12 | 4 | 0 | 0 | 8 | Highly resistant |
| AsHPPDm-F372V-02 | 0 | 16 | 0 | 0 | 0 | 0 | |
| | 140 | 6 | 5 | 4 | 1 | 33 | Moderately resistant |
| | 280 | 0 | 0 | 16 | 0 | 67 | Poorly resistant |
| AsHPPDm-F372V-F383W-02 | 0 | 16 | 0 | 0 | 0 | 0 | |
| | 140 | 16 | 0 | 0 | 0 | 0 | Highly resistant |
| | 280 | 12 | 3 | 1 | 0 | 10 | Highly resistant |

**TABLE 9 Mesotrione tolerance of transgenic Arabidopsis thaliana T₁ plants**

| *Arabidopsis thaliana* genotypes | Concentration (g ai/ha) | Classification and statistics of the grade of pesticide damage | | | | Scores | Resistance evaluation |
|---|---|---|---|---|---|---|---|
| | | Grade 0 | Grade 1 | Grade 2 | Grade 3 | | |
| CK | 0 | 16 | 0 | 0 | 0 | 0 | |
| | 210 | 0 | 0 | 0 | 16 | 100 | Non-resistant |
| | 420 | 0 | 0 | 0 | 16 | 100 | Non-resistant |
| DBN11726N | 0 | 16 | 0 | 0 | 0 | 0 | |
| | 210 | 0 | 0 | 0 | 16 | 100 | Non-resistant |
| | 420 | 0 | 0 | 0 | 16 | 100 | Non-resistant |
| AsHPPD-02 | 0 | 16 | 0 | 0 | 0 | 0 | |
| | 210 | 0 | 0 | 0 | 16 | 100 | Non-resistant |
| | 420 | 0 | 0 | 0 | 16 | 100 | Non-resistant |
| AsHPPDm-F372G-02 | 0 | 16 | 0 | 0 | 0 | 0 | |
| | 210 | 5 | 8 | 2 | 1 | 31 | Moderately resistant |
| | 420 | 0 | 8 | 8 | 0 | 50 | Poorly resistant |
| AsHPPDm-F383W-02 | 0 | 16 | 0 | 0 | 0 | 0 | |
| | 210 | 4 | 9 | 2 | 1 | 33 | Moderately resistant |
| | 420 | 0 | 2 | 14 | 0 | 63 | Poorly resistant |
| AsHPPDm-F372G-F383W-02 | 0 | 16 | 0 | 0 | 0 | 0 | |
| | 210 | 16 | 0 | 0 | 0 | 0 | Highly resistant |
| | 420 | 13 | 3 | 0 | 0 | 6 | Highly resistant |
| AsHPPDm-F372V-02 | 0 | 16 | 0 | 0 | 0 | 0 | |
| | 210 | 5 | 7 | 4 | 0 | 31 | Moderately resistant |
| | 420 | 0 | 8 | 7 | 1 | 52 | Poorly resistant |
| AsHPPDm-F372V-F383W-02 | 0 | 16 | 0 | 0 | 0 | 0 | |
| | 210 | 16 | 0 | 0 | 0 | 0 | Highly resistant |
| | 420 | 15 | 1 | 0 | 0 | 2 | Highly resistant |

The results of TABLE 8 and TABLE 9 show that (1) as compared with CK, the *Arabidopsis thaliana* plants into which the HPPD genes with the combinatorial mutation at positions 372 and 383 (F372G+F383W or F372V+F383W) from *Avena sativa,* HPPD genes with the single mutation at position 372 (F372G or F372V) from *Avena sativa,* and HPPD genes with the single mutation at position 383 (F383W) from *Avena sativa* were introduced, had different degrees of tolerance to both isoxaflutole and mesotrione, while the *Arabidopsis thaliana* plants into which unmutated HPPD genes and the control vector DBN11726N were introduced had no tolerance to both isoxaflutole and mesotrione.

(2) From the perspective of resistance evaluation, when treated with isoxaflutole or mesotrione at two-fold field concentration, the *Arabidopsis thaliana* plants into which the HPPD genes with the combinatorial mutation at positions 372 and 383 (F372G+F383W or F372V+F383W) from *Avena sativa* exhibited better herbicide tolerance (highly resistant) to than the *Arabidopsis thaliana* plants into which the HPPD genes with the single mutation at position 372 (moderately resistant) or the single mutation at position 383 (moderately resistant) were introduced; when treated with isoxaflutole or mesotrione at four-fold field concentration, the *Arabidopsis thaliana* plants into which the HPPD genes with the combinatorial mutation at positions 372 and 383 (F372G+F383W or F372V+F383W) from *Avena sativa* exhibited better herbicide tolerance (highly resistant) than the *Arabidopsis thaliana* plants into which the HPPD genes with the single mutation at position 372 (poorly resistant) or the single mutation at position 383 (poorly resistant) were introduced, and further achieved a synergistically enhanced effect of herbicide tolerance.

The above Tables 8 and 9 demonstrate that different mutations at positions 372 and 383 (the combinatorial mutation F372G+F383W or F372V+F383W) of the wild-type HPPD amino acid sequence also achieved a synergistically enhanced tolerance to HPPD-inhibitor herbicides.

### Example 4: Combination of the combinatorial mutations at positions 372 and 383 with the mutation at other position in the HPPD amino acid sequence and the mutation effect thereof

### 1. Acquisition of the sequence with a combinatorial mutation at multiple positions

### (1) Acquisition of the HPPDm-1 amino acid sequence (AsHPPDm-A107-F372A-F383W amino acid sequence)

The original alanine (A) at position 107 of the AsHPPD amino acid sequence was deleted, and the amino acid at position 372 was mutated from the original phenylalanine (F) to alanine (A), to obtain the AsHPPDm-A107-F372A amino acid sequence as set forth in SEQ ID NO: 167 in the SEQUENCE LISTING; the AsHPPDm-A107-F372A-01 nucleotide sequence encoding the AsHPPDm-A107-F372A amino acid sequence is set forth in SEQ ID NO: 168 in the SEQUENCE LISTING; and the AsHPPDm-A107-F372A-02 nucleotide sequence encoding the AsHPPDm-A107-F372A amino acid sequence, which was obtained based on the *Arabidopsis thaliana*/soybean common codon usage bias, is set forth in SEQ ID NO:169 in the SEQUENCE LISTING.

The original alanine (A) at position 107 of the AsHPPD amino acid sequence was deleted, and the amino acid at position 383 was mutated from the original phenylalanine (F) to tryptophan (W), to obtain the AsHPPDm-A107-F383W amino acid sequence as set forth in SEQ ID NO: 170 in the SEQUENCE LISTING; the AsHPPDm-A107-F383W-01 nucleotide sequence encoding the AsHPPDm-A107-F383W amino acid sequence is set forth in SEQ ID NO: 171 in the SEQUENCE LISTING; and the AsHPPDm-A107-F383W-02 nucleotide sequence encoding the AsHPPDm-A107-F383W amino acid sequence, which was obtained based on the *Arabidopsis thaliana*/soybean common codon usage bias, is set forth in SEQ ID NO: 172 in the SEQUENCE LISTING.

The original alanine (A) at position 107 of the AsHPPD amino acid sequence was deleted, the amino acid at position 372 was mutated from the original phenylalanine (F) to alanine (A), and the amino acid at position 383 was mutated from the original phenylalanine (F) to tryptophan (W), to obtain the HPPDm-1 amino acid sequence (AsHPPDm-A107-F372A-F383W amino acid sequence) as set forth in SEQ ID NO: 173 in the SEQUENCE LISTING; the HPPDm-1-01 nucleotide sequence encoding the HPPDm-1 amino acid sequence is set forth in SEQ ID NO: 174 in the SEQUENCE LISTING; and the HPPDm-1-02 nucleotide sequence encoding the HPPDm-1 amino acid sequence, which was obtained based on the *Arabidopsis thaliana*/soybean common codon usage bias, is set forth in SEQ ID NO: 175 in the SEQUENCE LISTING.

### (2) Acquisition of the HPPDm-2 amino acid sequence (AsHPPDm-A111T-F372A-F383W amino acid sequence)

The amino acid at position 111 of the AsHPPD amino acid sequence was mutated from the original alanine (A) to threonine (T), and the amino acid at position 372 was mutated from the original phenylalanine (F) to alanine (A), to obtain the AsHPPDm-A111T-F372A amino acid sequence as set forth in SEQ ID NO: 176 in the SEQUENCE LISTING; the AsHPPDm-A111T-F372A-01 nucleotide sequence encoding the AsHPPDm-A111T-F372A amino acid sequence is set forth in SEQ ID NO: 177 in the SEQUENCE LISTING; and the AsHPPDm-A111T-F372A-02 nucleotide sequence encoding the AsHPPDm-A111T-F372A amino acid sequence, which was obtained based on the *Arabidopsis thaliana*/soybean common codon usage bias, is set forth in SEQ ID NO: 178 in the SEQUENCE LISTING.

The amino acid at position 111 of the AsHPPD amino acid sequence was mutated from the original alanine (A) to threonine (T), and the amino acid at position 383 was mutated from the original phenylalanine (F) to tryptophan (W), to obtain the AsHPPDm-A111T-F383W amino acid sequence as set forth in SEQ ID NO: 179 in the SEQUENCE LISTING; the AsHPPDm-A111T-F383W-01 nucleotide sequence encoding the AsHPPDm-A111T-F383W amino acid sequence is set forth in SEQ ID NO: 180 in the SEQUENCE LISTING; and the AsHPPDm-A111T-F383W-02 nucleotide sequence encoding the AsHPPDm-A111T-F383W amino acid sequence, which was obtained based on the *Arabidopsis thaliana*/soybean common codon usage bias, is set forth in SEQ ID NO: 181 in the SEQUENCE LISTING.

The amino acid at position 111 of the AsHPPD amino acid sequence was mutated from the original alanine (A) to threonine (T), the amino acid at position 372 was mutated from the original phenylalanine (F) to alanine (A), and the amino acid at position 383 was mutated from the original phenylalanine (F) to tryptophan (W), to obtain the HPPDm-2 amino acid sequence (AsHPPDm-A111T-F372A-F383W amino acid sequence) as set forth in SEQ ID NO: 182 in the SEQUENCE LISTING; the HPPDm-2-01 nucleotide sequence encoding the HPPDm-2 amino acid sequence is set forth in SEQ ID NO: 183 in the SEQUENCE LISTING; and the HPPDm-2-02 nucleotide sequence encoding the HPPDm-2 amino acid sequence, which was obtained based on the *Arabidopsis thaliana*/soybean common codon usage bias, is set forth in SEQ ID NO: 184 in the SEQUENCE LISTING.

### (3) Acquisition of the HPPDm-3 amino acid sequence (AsHPPDm-A106G-F372A-F383W amino acid sequence)

The amino acid at position 106 of the AsHPPD amino acid sequence was mutated from the original alanine (A) to glycine (G), the amino acid at position 372 was mutated from the original phenylalanine (F) to alanine (A), and the amino acid at position 383 was mutated from the original phenylalanine (F) to tryptophan (W), to obtain the HPPDm-3 amino acid sequence (AsHPPDm-A106G-F372A-F383W amino acid sequence) as set forth in SEQ ID NO: 185 in the SEQUENCE LISTING; the HPPDm-3-01 nucleotide sequence encoding the HPPDm-3 amino acid sequence is set forth in SEQ ID NO: 186 in the SEQUENCE LISTING; and the HPPDm-3-02 nucleotide sequence encoding the HPPDm-3 amino acid sequence, which was obtained based on the *Arabidopsis thaliana*/soybean common codon usage bias, is set forth in SEQ ID NO: 187 in the SEQUENCE LISTING.

### (4) Acquisition of the HPPDm-4 amino acid sequence (AsHPPDm-A107-K351N-F372A-F383W amino acid sequence)

The original alanine (A) at position 107 of the AsHPPD amino acid sequence was deleted, the amino acid at position 351 was mutated from the original lysine (K) to asparagine (N), the amino acid at position 372 was mutated from the original phenylalanine (F) to alanine (A), and the amino acid at position 383 was mutated from the original phenylalanine (F) to tryptophan (W), to obtain the HPPDm-4 amino acid sequence (AsHPPDm-A107-K351N-F372A-F383W amino acid sequence) as set forth in SEQ ID NO: 188 in the SEQUENCE LISTING; the HPPDm-4-01 nucleotide sequence encoding the HPPDm-4 amino acid sequence is set forth in SEQ ID NO: 189 in the SEQUENCE LISTING; and the HPPDm-4-02 nucleotide sequence encoding the HPPDm-4 amino acid sequence, which was obtained based on the *Arabidopsis thaliana*/soybean common codon usage bias, is set forth in SEQ ID NO: 190 in the SEQUENCE LISTING.

### (5) Acquisition of the HPPDm-5 amino acid sequence (AsHPPDm-A111T-K351N-F372A-F383W amino acid sequence)

The amino acid at position 111 of the AsHPPD amino acid sequence was mutated from the original alanine (A) to threonine (T), the amino acid at position 351 was mutated from the original lysine (K) to asparagine (N), the amino acid at position 372 was mutated from the original phenylalanine (F) to alanine (A), and the amino acid at position 383 was mutated from the original phenylalanine (F) to tryptophan (W), to obtain the HPPDm-5 amino acid sequence (AsHPPDm-A111T-K351N-F372A-F383W amino acid sequence) as set forth in SEQ ID NO: 191 in the SEQUENCE LISTING; the HPPDm-5-01 nucleotide sequence encoding the HPPDm-5 amino acid sequence is set forth in SEQ ID NO: 192 in the SEQUENCE LISTING; and the HPPDm-5-02 nucleotide sequence encoding the HPPDm-5 amino acid sequence, which was obtained based on the *Arabidopsis thaliana*/soybean common codon usage bias, is set forth in SEQ ID NO: 193 in the SEQUENCE LISTING.

### (6) Acquisition of the HPPDm-6 amino acid sequence (AsHPPDm-A106G-K351N-F372A-F383W amino acid sequence)

The amino acid at position 106 of the AsHPPD amino acid sequence was mutated from the original alanine (A) to threonine (G), the amino acid at position 351 was mutated from the original lysine (K) to asparagine (N), the amino acid at position 372 was mutated from the original phenylalanine (F) to alanine (A), and the amino acid at position 383 was mutated from the original phenylalanine (F) to tryptophan (W), to obtain the HPPDm-6 amino acid sequence (AsHPPDm-A106G-K351N-F372A-F383W amino acid sequence) as set forth in SEQ ID NO: 194 in the SEQUENCE LISTING; the HPPDm-6-01 nucleotide sequence encoding the HPPDm-6 amino acid sequence is set forth in SEQ ID NO: 195 in the SEQUENCE LISTING; and the HPPDm-6-02 nucleotide sequence encoding the HPPDm-6 amino acid sequence, which was obtained based on the *Arabidopsis thaliana*/soybean common codon usage bias, is set forth in SEQ ID NO: 196 in the SEQUENCE LISTING.

### (7) Acquisition of the HPPDm-7 amino acid sequence

The HPPDm-7 amino acid sequence is obtained by mutating the C-terminal amino acid of the HPPD amino acid sequence from *Avena sativa* on the basis of the AsHPPDm-A107-K351N-F372A-F383W amino acid sequence, and the HPPDm-7 amino acid sequence is set forth in SEQ ID NO: 197 in the SEQUENCE LISTING; the HPPDm-7-01 nucleotide sequence encoding the HPPDm-7 amino acid sequence is set forth in SEQ ID NO: 198 in the SEQUENCE LISTING; and the HPPDm-7-02 nucleotide sequence encoding the HPPDm-7 amino acid sequence, which was obtained based on the *Arabidopsis thaliana*/soybean common codon usage bias, is set forth in SEQ ID NO: 199 in the SEQUENCE LISTING.

### (8) Acquisition of the HPPDm-8 amino acid sequence

The HPPDm-8 amino acid sequence is obtained by mutating the C-terminal amino acid of the HPPD amino acid sequence from *Avena sativa* on the basis of the AsHPPDm-A111T-K351N-F372A-F383W amino acid sequence, and the HPPDm-8 amino acid sequence is set forth in SEQ ID NO: 200 in the SEQUENCE LISTING; the HPPDm-8-01 nucleotide sequence encoding the HPPDm-8 amino acid sequence is set forth in SEQ ID NO: 201 in the SEQUENCE LISTING; and the HPPDm-8-02 nucleotide sequence encoding the HPPDm-8 amino acid sequence, which was obtained based on the *Arabidopsis thaliana*/soybean common codon usage bias, is set forth in SEQ ID NO: 202 in the SEQUENCE LISTING.

### (9) Acquisition of the HPPDm-9 amino acid sequence

The HPPDm-9 amino acid sequence is obtained by mutating the C-terminal amino acid of the HPPD amino acid sequence from *Avena sativa* on the basis of the AsHPPDm-A106G-K351N-F372A-F383W amino acid sequence, and the HPPDm-9 amino acid sequence is set forth in SEQ ID NO: 203 in the SEQUENCE LISTING; the HPPDm-9-01 nucleotide sequence encoding the HPPDm-9 amino acid sequence is set forth in SEQ ID NO: 204 in the SEQUENCE LISTING; and the HPPDm-9-02 nucleotide sequence encoding the HPPDm-9 amino acid sequence, which was obtained based on the *Arabidopsis thaliana*/soybean common codon usage bias, is set forth in SEQ ID NO: 205 in the SEQUENCE LISTING.

### 2. Construction of recombinant expression vectors containing HPPDs with a combinatorial mutation at multiple positions for Arabidopsis thaliana

According to the method of constructing the recombinant expression vector DBN11726 containing the AsHPPDm-F372A-F383W-02 nucleotide sequence as described above in point 3 of Example 1, the AsHPPDm-A107-F372A-02 nucleotide sequence, AsHPPDm-A107-F383W-02 nucleotide sequence, HPPDm-1-02 nucleotide sequence, AsHPPDm-A111T-F372A-02 nucleotide sequence, AsHPPDm-A111T-F383W-02 nucleotide sequence, HPPDm-2-02 nucleotide sequence, HPPDm-3-02 nucleotide sequence, HPPDm-4-02 nucleotide sequence, HPPDm-5-02 nucleotide sequence, HPPDm-6-02 nucleotide sequence, HPPDm-7-02 nucleotide sequence, HPPDm-8-02 nucleotide sequence, and HPPDm-9-02 nucleotide sequence which were linked to the universal adapter primer 1 were respectively subjected to a recombination reaction with the linearized DBNBC-01 expression vector backbone, to obtain the recombinant expression vectors DBN11778 to DBN11790 in sequence. Sequencing verified that the aforementioned nucleotide sequences were inserted correctly in the recombinant expression vectors DBN11778 to DBN11790.

### 3. Transformation of Agrobacterium with the recombinant expression vectors for Arabidopsis thaliana

According to the method of transforming *Agrobacterium* with the recombinant expression vectors for *Arabidopsis thaliana* as described above in point 4 of Example 1, the recombinant expression vectors DBN11778 to DBN11790 which had been constructed correctly, and the recombinant expression vector DBN11727 containing the AsHPPD-02 nucleotide sequence in point 3 of Example 1, and the control recombinant expression vector DBN11726N in point 3 of Example 1 were transformed into *Agrobacterium* GV3101 respectively using a liquid nitrogen method. The results were verified by sequencing, showing that the structures of the recombinant expression vectors DBN11778 to DBN11790, DBN11727 and DBN11726N were completely correct.

### 4. Detection of the herbicide tolerance of the Arabidopsis thaliana plants into which the HPPDs with a combinatorial mutation at multiple positions were introduced

According to the method as described above in point 5 of Example 1, *Arabidopsis thaliana* inflorescences were immersed in the *Agrobacterium* solution as described in Example 3, so as to introduce the T-DNA in the recombinant expression vectors DBN11778 to DBN11790 constructed in Example 2, the recombinant expression vector DBN11727 containing the AsHPPD-02 nucleotide sequence in point 3 of Example 1, and the control recombinant expression vector DBN11726N in point 3 of Example 1 into the *Arabidopsis thaliana* chromosomes, thereby obtaining the corresponding transgenic *Arabidopsis thaliana* plants, i.e., *Arabidopsis thaliana* T₁ plants into which the AsHPPDm-A107-F372A-02 nucleotide sequence was introduced (AsHPPDm-A107-F372A-02), *Arabidopsis thaliana* T₁ plants into which the AsHPPDm-A107-F383W-02 nucleotide sequence was introduced (AsHPPDm-A107-F383W-02), *Arabidopsis thaliana* T₁ plants into which the HPPDm-1-02 nucleotide sequence was introduced (HPPDm-1-02), *Arabidopsis thaliana* T₁ plants into which the AsHPPDm-A111T-F372A-02 nucleotide sequence was introduced (AsHPPDm-A111T-F372A-02), *Arabidopsis thaliana* T₁ plants into which the AsHPPDm-A111T-F383W-02 nucleotide sequence was introduced (AsHPPDm-A111T-F383W-02), *Arabidopsis thaliana* T₁ plants into which the HPPDm-2-02 nucleotide sequence was introduced (HPPDm-2-02), *Arabidopsis thaliana* T₁ plants into which the HPPDm-3-02 nucleotide sequence was introduced (HPPDm-3-02), *Arabidopsis thaliana* T₁ plants into which the HPPDm-4-02 nucleotide sequence was introduced (HPPDm-4-02), *Arabidopsis thaliana* T₁ plants into which the HPPDm-5-02 nucleotide sequence was introduced (HPPDm-5-02), *Arabidopsis thaliana* T₁ plants into which the HPPDm-6-02 nucleotide sequence was introduced (HPPDm-6-02), *Arabidopsis thaliana* T₁ plants into which the HPPDm-7-02 nucleotide sequence was introduced (HPPDm-7-02), *Arabidopsis thaliana* T₁ plants into which the HPPDm-8-02 nucleotide sequence was introduced (HPPDm-8-02), and *Arabidopsis thaliana* T₁ plants into which the HPPDm-9-02 nucleotide sequence was introduced (HPPDm-9-02), *Arabidopsis thaliana* T₁ plants into which the AsHPPD-02 nucleotide sequence was introduced (AsHPPD-02), and *Arabidopsis thaliana* T₁ plants into which the control recombinant expression vector was introduced (DBN11726N).

According to the method as described above in point 6 of Example 1, the aforementioned *Arabidopsis thaliana* T₁ plants and wild-type *Arabidopsis thaliana* plants (CK) (18 days after sowing) were sprayed with topramezone at three different concentrations (i.e., 100 g ai/ha (four-fold field concentration, 4×), 200 g ai/ha (eight-fold field concentration, 8×) and 0 g ai/ha (water, 0×)), isoxaflutole at three different concentrations (i.e., 140 g ai/ha (two-fold field concentration, 2×), 280 g ai/ha (four-fold field concentration, 4×) and 0 g ai/ha (water, 0×)), and mesotrione at three different concentrations (i.e., 210 g ai/ha (two-fold field concentration, 2×), 420 g ai/ha (four-fold field concentration, 4×) and 0 g ai/ha (water, 0×)) respectively to detect the herbicide tolerance of *Arabidopsis thaliana.* The experimental results are shown in TABLE 10 to TABLE 12.

**TABLE 10 Topramezone tolerance of Arabidopsis thaliana T₁ plants into which the HPPD genes with a combinatorial mutation at multiple positions were introduced**

| *Arabidopsis thaliana* genotypes | Concentration (g ai/ha) | Classification and statistics of the grade of pesticide damage | | | | Scores | Resistance evaluation |
|---|---|---|---|---|---|---|---|
| | | Grade 0 | Grade 1 | Grade 2 | Grade 3 | | |
| CK | 0 | 16 | 0 | 0 | 0 | 0 | |
| | 100 | 0 | 0 | 0 | 16 | 100 | Non-resistant |
| | 200 | 0 | 0 | 0 | 16 | 100 | Non-resistant |
| DBN11726N | 0 | 16 | 0 | 0 | 0 | 0 | |
| | 100 | 0 | 0 | 0 | 16 | 100 | Non-resistant |
| | 200 | 0 | 0 | 0 | 16 | 100 | Non-resistant |
| AsHPPD-02 | 0 | 16 | 0 | 0 | 0 | 0 | |
| | 100 | 0 | 0 | 4 | 12 | 92 | Non-resistant |
| | 200 | 0 | 0 | 0 | 16 | 100 | Non-resistant |
| HPPDm-1-02 | 0 | 16 | 0 | 0 | 0 | 0 | |
| | 100 | 16 | 0 | 0 | 0 | 0 | Highly resistant |
| | 200 | 16 | 0 | 0 | 0 | 0 | Highly resistant |
| HPPDm-2-02 | 0 | 16 | 0 | 0 | 0 | 0 | |
| | 100 | 16 | 0 | 0 | 0 | 0 | Highly resistant |
| | 200 | 16 | 0 | 0 | 0 | 0 | Highly resistant |
| HPPDm-3-02 | 0 | 16 | 0 | 0 | 0 | 0 | |
| | 100 | 16 | 0 | 0 | 0 | 0 | Highly resistant |
| | 200 | 16 | 0 | 0 | 0 | 0 | Highly resistant |
| HPPDm-4-02 | 0 | 16 | 0 | 0 | 0 | 0 | |
| | 100 | 16 | 0 | 0 | 0 | 0 | Highly resistant |
| | 200 | 16 | 0 | 0 | 0 | 0 | Highly resistant |
| HPPDm-5-02 | 0 | 16 | 0 | 0 | 0 | 0 | |
| | 100 | 16 | 0 | 0 | 0 | 0 | Highly resistant |
| | 200 | 16 | 0 | 0 | 0 | 0 | Highly resistant |
| HPPDm-6-02 | 0 | 16 | 0 | 0 | 0 | 0 | |
| | 100 | 16 | 0 | 0 | 0 | 0 | Highly resistant |
| | 200 | 16 | 0 | 0 | 0 | 0 | Highly resistant |
| HPPDm-7-02 | 0 | 16 | 0 | 0 | 0 | 0 | |
| | 100 | 16 | 0 | 0 | 0 | 0 | Highly resistant |
| | 200 | 16 | 0 | 0 | 0 | 0 | Highly resistant |
| HPPDm-8-02 | 0 | 16 | 0 | 0 | 0 | 0 | |
| | 100 | 16 | 0 | 0 | 0 | 0 | Highly resistant |
| | 200 | 16 | 0 | 0 | 0 | 0 | Highly resistant |
| HPPDm-9-02 | 0 | 16 | 0 | 0 | 0 | 0 | |
| | 100 | 16 | 0 | 0 | 0 | 0 | Highly resistant |
| | 200 | 16 | 0 | 0 | 0 | 0 | Highly resistant |

The results of TABLE 10 show that (1) as compared with CK and the *Arabidopsis thaliana* plants into which unmutated HPPD genes were introduced, when treated with topramezone at four-fold or eight-fold field concentration, all the HPPD genes with the combination of the combinatorial mutation at positions 372 and 383 and the mutations at other positions (including A107 deletion, A1 1 1T, A106G, A107+K351N, A111T+K351N, A106G+K351N, A107+K351 N+C-terminal mutation, A111T+K351N+C-terminal mutation, or A106G+K351N+C-terminal mutation) can confer highly-resistant tolerance to topramezone upon the plants. This shows that the combination of the combinatorial mutation at positions 372+383 and the mutations at other positions of the HPPD amino acid sequence did not affect the topramezone tolerance of the combinatorial mutation at positions 372+383 alone, and also shows the importance and stability of the tolerance to HPPD-inhibitor herbicides of the plants conferred by the combinatorial mutation at positions 372 and 383 of the HPPD amino acid sequence. In contrast, the *Arabidopsis thaliana* T₁ plants into which the control recombinant expression vector DBN11726N was introduced had no tolerance to topramezone.

**TABLE 11 Isoxaflutole tolerance of Arabidopsis thaliana T₁ plants into which the HPPD genes with a combinatorial mutation at multiple positions were introduced**

| *Arabidopsis thaliana* genotypes | Concentration (g ai/ha) | Classification and statistics of the grade of pesticide damage | | | | Scores | Resistance evaluation |
|---|---|---|---|---|---|---|---|
| | | Grade 0 | Grade 1 | Grade 2 | Grade 3 | | |
| CK | 0 | 16 | 0 | 0 | 0 | 0 | |
| | 140 | 0 | 0 | 0 | 16 | 100 | Non-resistant |
| | 280 | 0 | 0 | 0 | 16 | 100 | Non-resistant |
| DBN11726N | 0 | 16 | 0 | 0 | 0 | 0 | |
| | 140 | 0 | 0 | 0 | 16 | 100 | Non-resistant |
| | 280 | 0 | 0 | 0 | 16 | 100 | Non-resistant |
| AsHPPD-02 | 0 | 16 | 0 | 0 | 0 | 0 | |
| | 140 | 0 | 1 | 4 | 11 | 88 | Non-resistant |
| | 280 | 0 | 0 | 0 | 16 | 100 | Non-resistant |
| AsHPPDm-A107-F372A-02 | 0 | 16 | 0 | 0 | 0 | 0 | |
| | 140 | 5 | 7 | 4 | 0 | 31 | Moderately resistant |
| | 280 | 0 | 1 | 15 | 0 | 65 | Poorly resistant |
| AsHPPDm-A107-F383W-02 | 0 | 16 | 0 | 0 | 0 | 0 | |
| | 140 | 7 | 7 | 1 | 1 | 25 | Moderately resistant |
| | 280 | 4 | 4 | 7 | 1 | 44 | Poorly resistant |
| HPPDm-1-02 | 0 | 16 | 0 | 0 | 0 | 0 | |
| | 140 | 16 | 0 | 0 | 0 | 0 | Highly resistant |
| | 280 | 12 | 4 | 0 | 0 | 8 | Highly resistant |
| AsHPPDm-A111T-F372A-02 | 0 | 16 | 0 | 0 | 0 | 0 | |
| | 140 | 5 | 8 | 1 | 2 | 33 | Moderately resistant |
| | 280 | 0 | 0 | 16 | 0 | 67 | Poorly resistant |
| AsHPPDm-A111T-F383W-02 | 0 | 16 | 0 | 0 | 0 | 0 | |
| | 140 | 6 | 8 | 2 | 0 | 25 | Moderately resistant |
| | 280 | 4 | 4 | 8 | 0 | 42 | Poorly resistant |
| HPPDm-2-02 | 0 | 16 | 0 | 0 | 0 | 0 | |
| | 140 | 16 | 0 | 0 | 0 | 0 | Highly resistant |
| | 280 | 12 | 3 | 1 | 0 | 10 | Highly resistant |
| HPPDm-3-02 | 0 | 16 | 0 | 0 | 0 | 0 | |
| | 140 | 16 | 0 | 0 | 0 | 0 | Highly resistant |
| | 280 | 12 | 4 | 0 | 0 | 8 | Highly resistant |
| HPPDm-4-02 | 0 | 16 | 0 | 0 | 0 | 0 | |
| | 140 | 16 | 0 | 0 | 0 | 0 | Highly resistant |
| | 280 | 13 | 3 | 0 | 0 | 6 | Highly resistant |
| HPPDm-5-02 | 0 | 16 | 0 | 0 | 0 | 0 | |
| | 140 | 16 | 0 | 0 | 0 | 0 | Highly resistant |
| | 280 | 14 | 1 | 1 | 0 | 6 | Highly resistant |
| HPPDm-6-02 | 0 | 16 | 0 | 0 | 0 | 0 | |
| | 140 | 16 | 0 | 0 | 0 | 0 | Highly resistant |
| | 280 | 13 | 3 | 0 | 0 | 6 | Highly resistant |
| HPPDm-7-02 | 0 | 16 | 0 | 0 | 0 | 0 | |
| | 140 | 16 | 0 | 0 | 0 | 0 | Highly resistant |
| | 280 | 16 | 0 | 0 | 0 | 0 | Highly resistant |
| HPPDm-8-02 | 0 | 16 | 0 | 0 | 0 | 0 | |
| | 140 | 16 | 0 | 0 | 0 | 0 | Highly resistant |
| | 280 | 16 | 0 | 0 | 0 | 0 | Highly resistant |
| HPPDm-9-02 | 0 | 16 | 0 | 0 | 0 | 0 | |
| | 140 | 16 | 0 | 0 | 0 | 0 | Highly resistant |
| | 280 | 16 | 0 | 0 | 0 | 0 | Highly resistant |

**TABLE 12 Mesotrione tolerance of Arabidopsis thaliana T₁ plants into which the HPPD genes with a combinatorial mutation at multiple positions were introduced**

| *Arabidopsis thaliana* genotypes | Concentration (g ai/ha) | Classification and statistics of the grade of pesticide damage | | | | Scores | Resistance evaluation |
|---|---|---|---|---|---|---|---|
| | | Grade 0 | Grade 1 | Grade 2 | Grade 3 | | |
| CK | 0 | Grade 0 | Grade 1 | Grade 2 | Grade 3 | 0 | |
| | 210 | 0 | 0 | 0 | 16 | 100 | Non-resistant |
| | 420 | 0 | 0 | 0 | 16 | 100 | Non-resistant |
| DBN11726N | 0 | 16 | 0 | 0 | 0 | 0 | |
| | 210 | 0 | 0 | 0 | 16 | 100 | Non-resistant |
| | 420 | 0 | 0 | 0 | 16 | 100 | Non-resistant |
| AsHPPD-02 | 0 | 16 | 0 | 0 | 0 | 0 | |
| | 210 | 0 | 0 | 0 | 16 | 100 | Non-resistant |
| | 420 | 0 | 0 | 0 | 16 | 100 | Non-resistant |
| AsHPPDm-A107-F372A-02 | 0 | 16 | 0 | 0 | 0 | 0 | |
| | 210 | 6 | 7 | 1 | 2 | 31 | Moderately resistant |
| | 420 | 1 | 7 | 8 | 0 | 48 | Poorly resistant |
| AsHPPDm-A107-F383W-02 | 0 | 16 | 0 | 0 | 0 | 0 | |
| | 210 | 4 | 9 | 3 | 0 | 31 | Moderately resistant |
| | 420 | 0 | 2 | 14 | 0 | 63 | Poorly resistant |
| HPPDm-1-02 | 0 | 16 | 0 | 0 | 0 | 0 | |
| | 210 | 16 | 0 | 0 | 0 | 0 | Highly resistant |
| | 420 | 15 | 1 | 0 | 0 | 2 | Highly resistant |
| AsHPPDm-A111T-F372A-02 | 0 | 16 | 0 | 0 | 0 | 0 | |
| | 210 | 5 | 8 | 3 | 0 | 29 | Moderately resistant |
| | 420 | 0 | 8 | 7 | 1 | 52 | Poorly resistant |
| AsHPPDm-A111T-F383W-02 | 0 | 16 | 0 | 0 | 0 | 0 | |
| | 210 | 4 | 10 | 2 | 0 | 29 | Moderately resistant |
| | 420 | 0 | 2 | 13 | 1 | 65 | Poorly resistant |
| HPPDm-2-02 | 0 | 16 | 0 | 0 | 0 | 0 | |
| | 210 | 16 | 0 | 0 | 0 | 0 | Highly resistant |
| | 420 | 14 | 2 | 0 | 0 | 4 | Highly resistant |
| HPPDm-3-02 | 0 | 16 | 0 | 0 | 0 | 0 | |
| | 210 | 16 | 0 | 0 | 0 | 0 | Highly resistant |
| | 420 | 14 | 2 | 0 | 0 | 4 | Highly resistant |
| HPPDm-4-02 | 0 | 16 | 0 | 0 | 0 | 0 | |
| | 210 | 16 | 0 | 0 | 0 | 0 | Highly resistant |
| | 420 | 15 | 1 | 0 | 0 | 2 | Highly resistant |
| HPPDm-5-02 | 0 | 16 | 0 | 0 | 0 | 0 | |
| | 210 | 16 | 0 | 0 | 0 | 0 | Highly resistant |
| | 420 | 16 | 0 | 0 | 0 | 0 | Highly resistant |
| HPPDm-6-02 | 0 | 16 | 0 | 0 | 0 | 0 | |
| | 210 | 16 | 0 | 0 | 0 | 0 | Highly resistant |
| | 420 | 16 | 0 | 0 | 0 | 0 | Highly resistant |
| HPPDm-7-02 | 0 | 16 | 0 | 0 | 0 | 0 | |
| | 210 | 16 | 0 | 0 | 0 | 0 | Highly resistant |
| | 420 | 15 | 1 | 0 | 0 | 2 | Highly resistant |
| HPPDm-8-02 | 0 | 16 | 0 | 0 | 0 | 0 | |
| | 210 | 16 | 0 | 0 | 0 | 0 | Highly resistant |
| | 420 | 16 | 0 | 0 | 0 | 0 | Highly resistant |
| HPPDm-9-02 | 0 | 16 | 0 | 0 | 0 | 0 | |
| | 210 | 16 | 0 | 0 | 0 | 0 | Highly resistant |
| | 420 | 16 | 0 | 0 | 0 | 0 | Highly resistant |

The results of Tables 11 and 12 show that (1) as compared with CK and the *Arabidopsis thaliana* plants into which unmutated HPPD genes were introduced, when treated with isoxaflutole and mesotrione at two-fold or four-fold field concentration, all the HPPD genes with the combination of the combinatorial mutation at positions 372 and 383 and the mutations at other positions (including A107 deletion, A111T, A106G, A107+K351N, A111T+K351N, A106G+K351N, A107+K351 N+C-terminal mutation, A111T+K351N+C-terminal mutation, or A106G+K351N+C-terminal mutation) can confer highly-resistant tolerance to isoxaflutole and mesotrione upon the plants, while the *Arabidopsis thaliana* T₁ plants into which the control recombinant expression vector DBN11726N was introduced had no tolerance to isoxaflutole and mesotrione.
(2) From the perspective of resistance evaluation, when treated with isoxaflutole or mesotrione at four-fold field concentration, the *Arabidopsis thaliana* plants into which HPPDm-1-02 (AsHPPDm-A107-F372A-F383W-02) genes was introduced exhibited better herbicide tolerance (highly resistant) than the *Arabidopsis thaliana* plants into which AsHPPDm-A107-F372A-02 genes (poorly resistant) or AsHPPDm-A107-F383W-02 genes (poorly resistant) were introduced, and further achieved a synergistically enhanced effect of herbicide tolerance. Similarly, the *Arabidopsis thaliana* plants into which HPPDm-2-02 (AsHPPDm-A111T-F372A-F383W) genes was introduced exhibited better herbicide tolerance (highly resistant) than the *Arabidopsis thaliana* plants into which AsHPPDm-A111T-F372A-02 genes (poorly resistant) or AsHPPDm-A111T-F383W-02 genes (poorly resistant) were introduced, and further achieved a synergistically enhanced effect of herbicide tolerance. Therefore, the combination of the combinatorial mutation at positions 372+383 and the mutations at other positions of the HPPD amino acid sequence did not affect the synergistically enhanced herbicide tolerance to HPPD-inhibitor herbicides of of the combinatorial mutation at positions 372+383 alone, and further shows the importance and stability of the tolerance to HPPD-inhibitor herbicides of the plants conferred by the combinatorial mutation at positions 372 and 383 of the HPPD amino acid sequence.
(3) From the perspective of resistance scores, when treated with isoxaflutole at four-fold field concentration, all the *Arabidopsis thaliana* T₁ plants into which HPPDm-7-02 to HPPDm-9-02 nucleotide sequences were introduced had higher resistance scores (0) than the *Arabidopsis thaliana* T₁ plants into which HPPDm-1-02 to HPPDm-6-02 nucleotide sequences were introduced, indicating that optimizing the C-terminal of the HPPD amino acid sequence would be advantageous to improving the tolerance of the plants to isoxaflutole.

### Example 5: A combinatorial mutation (not the combinatorial mutation F372A (F372G/F372V)+F383W) of the HPPD amino acid sequence and verification of the mutation effect

### 1. Acquisition of mutant genes (not the combinatorial mutation F372A (F372G/F372V)+F383W) of the HPPD amino acid sequence from Avena sativa and Arabidopsis thaliana

### (1) A combinatorial mutation gene (F372A+F415W) of HPPD from Avena sativa

The amino acid at position 415 of the AsHPPD amino acid sequence was mutated from the original phenylalanine (F) to tryptophan (W), to obtain the AsHPPDm-F415W amino acid sequence as set forth in SEQ ID NO: 206 in the SEQUENCE LISTING; the AsHPPDm-F415W-01 nucleotide sequence encoding the AsHPPDm-F415W amino acid sequence is set forth in SEQ ID NO: 207 in the SEQUENCE LISTING; and the AsHPPDm-F415W-02 nucleotide sequence encoding the AsHPPDm-F415W amino acid sequence, which was obtained based on the *Arabidopsis thaliana*/soybean common codon usage bias, is set forth in SEQ ID NO: 208 in the SEQUENCE LISTING.

The amino acid at position 372 of the AsHPPD amino acid sequence was mutated from the original phenylalanine (F) to alanine (A), and the amino acid at position 415 was mutated from the original phenylalanine (F) to tryptophan (W), to obtain the AsHPPDm-F372A-F415W amino acid sequence as set forth in SEQ ID NO: 209 in the SEQUENCE LISTING; the AsHPPDm-F372A-F415W-01 nucleotide sequence encoding the AsHPPDm-F372A-F415W amino acid sequence is set forth in SEQ ID NO: 210 in the SEQUENCE LISTING; and the AsHPPDm-F372A-F415W-02 nucleotide sequence encoding the AsHPPDm-F372A-F415W amino acid sequence, which was obtained based on the *Arabidopsis thaliana*/soybean common codon usage bias, is set forth in SEQ ID NO: 211 in the SEQUENCE LISTING.

### (2) A combinatorial mutation gene (F372A+F415W) of HPPD from Arabidopsis thaliana

The amino acid at position 424 of the AtHPPD amino acid sequence (corresponding to position 415 of the amino acid sequence as set forth in SEQ ID NO:1, that is, position 415) was mutated from the original phenylalanine (F) to tryptophan (W), to obtain the AtHPPDm-F415W amino acid sequence as set forth in SEQ ID NO: 212 in the SEQUENCE LISTING; the AtHPPDm-F415W-01 nucleotide sequence encoding the AtHPPDm-F415W amino acid sequence is set forth in SEQ ID NO: 213 in the SEQUENCE LISTING; and the AtHPPDm-F415W-02 nucleotide sequence encoding the AtHPPDm-F415W amino acid sequence, which was obtained based on the *Arabidopsis thaliana*/soybean common codon usage bias, is set forth in SEQ ID NO: 214 in the SEQUENCE LISTING.

The amino acid at position 372 of the AtHPPD amino acid sequence was mutated from the original phenylalanine (F) to alanine (A), and the amino acid at position 415 was mutated from the original phenylalanine (F) to tryptophan (W), to obtain the AtHPPDm-F372A-F415W amino acid sequence as set forth in SEQ ID NO: 215 in the SEQUENCE LISTING; the AtHPPDm-F372A-F415W-01 nucleotide sequence encoding the AtHPPDm-F372A-F415W amino acid sequence is set forth in SEQ ID NO: 216 in the SEQUENCE LISTING; and the AtHPPDm-F372A-F415W-02 nucleotide sequence encoding the AtHPPDm-F415W amino acid sequence, which was obtained based on the *Arabidopsis thaliana*/soybean common codon usage bias, is set forth in SEQ ID NO: 217 in the SEQUENCE LISTING.

### (3) A combinatorial mutation gene (F372A+F383Y) of HPPD from Avena sativa

The amino acid at position 383 of the AsHPPD amino acid sequence was mutated from the original phenylalanine (F) to tyrosine (Y), to obtain the AsHPPDm-F383Y amino acid sequence as set forth in SEQ ID NO: 218 in the SEQUENCE LISTING; the AsHPPDm-F383Y-01 nucleotide sequence encoding the AsHPPDm-F383Y amino acid sequence is set forth in SEQ ID NO: 219 in the SEQUENCE LISTING; and the AsHPPDm-F383Y-02 nucleotide sequence encoding the AsHPPDm-F383Y amino acid sequence, which was obtained based on the *Arabidopsis thaliana*/soybean common codon usage bias, is set forth in SEQ ID NO: 220 in the SEQUENCE LISTING.

The amino acid at position 372 position of the AsHPPD amino acid sequence was mutated from the original phenylalanine (F) to alanine (A), and amino acid at position 383 was mutated from the original phenylalanine (F) to tyrosine (Y), to obtain the AsHPPDm-F372A-F383Y amino acid sequence as set forth in SEQ ID NO: 221 in the SEQUENCE LISTING; the AsHPPDm-F372A-F383Y-01 nucleotide sequence encoding the AsHPPDm-F372A-F383Y amino acid sequence is set forth in SEQ ID NO: 222 in the SEQUENCE LISTING; and the AsHPPDm-F372A-F383Y-02 nucleotide sequence encoding the AsHPPDm-F372A-F383Y amino acid sequence, which was obtained based on the *Arabidopsis thaliana*/*soybean* common codon usage bias, is set forth in SEQ ID NO: 223 in the SEQUENCE LISTING.

### (4) A combinatorial mutation gene (F372A+F383Y) of HPPD from Arabidopsis thaliana

The amino acid at position 392 of the AtHPPD amino acid sequence (corresponding to position 383 of the amino acid sequence as set forth in SEQ ID NO:1, that is, position 383) was mutated from the original phenylalanine (F) to tyrosine (Y), to obtain the AtHPPDm-F383Y amino acid sequence as set forth in SEQ ID NO: 224 in the SEQUENCE LISTING; the AtHPPDm-F383Y-01 nucleotide sequence encoding the AtHPPDm-F383Y amino acid sequence is set forth in SEQ ID NO: 225 in the SEQUENCE LISTING; and the AtHPPDm-F383Y-02 nucleotide sequence encoding the AtHPPDm-F383Y amino acid sequence, which was obtained based on the *Arabidopsis thaliana*/soybean common codon usage bias, is set forth in SEQ ID NO: 226 in the SEQUENCE LISTING.

The amino acid at position 372 of the AtHPPD amino acid sequence was mutated from the original phenylalanine (F) to alanine (A), and the amino acid at position 383 was mutated from the original phenylalanine (F) to tyrosine (Y), to obtain the AtHPPDm-F372A-F383Y amino acid sequence as set forth in SEQ ID NO: 227 in the SEQUENCE LISTING; the AtHPPDm-F372A-F383Y-01 nucleotide sequence encoding the AtHPPDm-F372A-F383Y amino acid sequence is set forth in SEQ ID NO: 228 in the SEQUENCE LISTING; and the AtHPPDm-F372A-F383Y-02 nucleotide sequence encoding the AtHPPDm-F372A-F383Y amino acid sequence, which was obtained based on the *Arabidopsis thaliana*/*soybean* common codon usage bias, is set forth in SEQ ID NO: 229 in the SEQUENCE LISTING.

### 2. Construction of recombinant expression vectors containing a mutant HPPD gene (not the combinatorial mutation F372A (F372G/F372V)+F383W) for Arabidopsis thaliana

According to the method of constructing the recombinant expression vector DBN11726 containing the AsHPPDm-F372A-F383W-02 nucleotide sequence as described above in point 3 of Example 1, the AsHPPDm-F415W-02 nucleotide sequence, AsHPPDm-F372A-F415W-02 nucleotide sequence, AtHPPDm-F415W-02 nucleotide sequence, AtHPPDm-F372A-F415W-02 nucleotide sequence, AsHPPDm-F383Y-02 nucleotide sequence, AsHPPDm-F372A-F383Y-02 nucleotide sequence, AtHPPDm-F383Y-02 nucleotide sequence, and AtHPPDm-F372A-F383Y-02 nucleotide sequence which were linked to the universal adapter primer 1 were respectively subjected to a recombination reaction with the linearized DBNBC-01 expression vector backbone, to obtain the recombinant expression vectors DBN11791 to DBN11798 in sequence. Sequencing verified that the aforementioned nucleotide sequences were inserted correctly in the recombinant expression vectors DBN11791 to DBN11798.

### 3. Transformation of Agrobacterium with the recombinant expression vectors for Arabidopsis thaliana

According to the method of transforming *Agrobacterium* with the recombinant expression vectors for *Arabidopsis thaliana* as described above in point 4 of Example 1, the recombinant expression vectors DBN11791 to DBN11798 which had been constructed correctly, the recombinant expression vector DBN11727 containing the AsHPPD-02 nucleotide sequence in point 3 of Example 1, the recombinant expression vector DBN11728 containing the AsHPPDm-F372A-02 nucleotide sequence in point 3 of Example 1, the recombinant expression vector DBN11730 containing the AtHPPDm-02 nucleotide sequence in point 2 of Example 2, and the recombinant expression vector DBN11731 containing the AtHPPDm-F372A-02 nucleotide sequence in point 2 of Example 2 were transformed into *Agrobacterium* GV3101 respectively using a liquid nitrogen method. The results were verified by sequencing, showing that the structures of the recombinant expression vectors DBN11791 to DBN11798, DBN11727, DBN11728, DBN11730, and DBN11731 were completely correct.

### 4. Detection of the herbicide tolerance of the Arabidopsis thaliana plants into which a mutant HPPD gene (not the combinatorial mutation F372A (F372G/F372V)+F383W) was introduced

According to the method as described above in point 5 of Example 1, *Arabidopsis thaliana* inflorescences were immersed in the *Agrobacterium* solution as described in Example 3, so as to introduce the T-DNA in the recombinant expression vectors DBN11791 to DBN11798, DBN11727, DBN11728, DBN11730, and DBN11731 constructed in Example 2 into the *Arabidopsis thaliana* chromosomes, thereby obtaining the corresponding transgenic *Arabidopsis thaliana* plants, i.e., *Arabidopsis thaliana* T₁ plants into which the AsHPPDm-F415W-02 nucleotide sequence was introduced (AsHPPDm-F415W-02), *Arabidopsis thaliana* T₁ plants into which the AsHPPDm-F372A-F415W-02 nucleotide sequence was introduced (AsHPPDm-F372A-F415W-02), *Arabidopsis thaliana* T₁ plants into which the AtHPPDm-F415W-02 nucleotide sequence was introduced (AtHPPDm-F415W-02), *Arabidopsis thaliana* T₁ plants into which the AtHPPDm-F372A-F415W-02 nucleotide sequence was introduced (AtHPPDm-F372A-F415W-02), *Arabidopsis thaliana* T₁ plants into which the AsHPPDm-F383Y-02 nucleotide sequence was introduced (AsHPPDm-F383Y-02), *Arabidopsis thaliana* T₁ plants into which the AsHPPDm-F372A-F383Y-02 nucleotide sequence was introduced (AsHPPDm-F372A-F383Y-02), *Arabidopsis thaliana* T₁ plants into which the AtHPPDm-F383Y-02 nucleotide sequence was introduced (AtHPPDm-F383Y-02), *Arabidopsis thaliana* T₁ plants into which the AtHPPDm-F372A-F383Y-02 nucleotide sequence was introduced (AtHPPDm-F372A-F383Y-02), *Arabidopsis thaliana* T₁ plants into which the AsHPPD-02 nucleotide sequence was introduced (AsHPPD-02), *Arabidopsis thaliana* T₁ plants into which the AsHPPDm-F372A-02 nucleotide sequence was introduced (AsHPPDm-F372A-02), *Arabidopsis thaliana* T₁ plants into which the AtHPPDm-02 nucleotide sequence was introduced (AtHPPDm-02), and *Arabidopsis thaliana* T₁ plants into which the AtHPPDm-F372A-02 nucleotide sequence was introduced (AtHPPDm-F372A-02).

According to the method as described above in point 6 of Example 1, the aforementioned *Arabidopsis thaliana* T₁ plants and wild-type *Arabidopsis thaliana* plants (CK) (18 days after sowing) were sprayed with topramezone at three different concentrations (i.e., 25 g ai/ha (one-fold field concentration, 1×), 100 g ai/ha (four-fold field concentration, 4×) and 0 g ai/ha (water, 0×)), isoxaflutole at five different concentrations (i.e., 35 g ai/ha (half-fold field concentration, 0.5×), 70 g ai/ha (one-fold field concentration, 1×), 140 g ai/ha (two-fold field concentration, 2×), 280 g ai/ha (four-fold field concentration, 4×) and 0 g ai/ha (water, 0×)) (among them, isoxaflutole at 0×, 0.5× and 1× concentrations were used to spray *Arabidopsis thaliana* T₁ plants into which the mutant HPPDs from *Arabidopsis thaliana* were introduced; and isoxaflutole at 0×, 2× and 4× concentrations were used to spray *Arabidopsis thaliana* T₁ plants into which the mutant HPPDs from *Avena sativa* were introduced), and mesotrione at five different concentrations (i.e., 52.5 g ai/ha (half-fold field concentration, 0.5×), 105 g ai/ha (one-fold field concentration, 1×), 210 g ai/ha (two-fold field concentration, 2×), 420 g ai/ha (four-fold field concentration, 4×) and 0 g ai/ha (water, 0×)) (among them, mesotrione at 0×, 0.5× and 1 × concentrations were used to spray *Arabidopsis thaliana* T₁ plants into which the mutant HPPDs from *Arabidopsis thaliana* were introduced; and mesotrione at 0×, 2× and 4× concentrations were used to spray *Arabidopsis thaliana* T₁ plants into which the mutant HPPDs from *Avena sativa* were introduced) respectively, to detect the herbicide tolerance of *Arabidopsis thaliana.* The experimental results are shown in TABLE 13 to TABLE 15.

**TABLE 13 Topramezone tolerance of Arabidopsis thaliana T₁ plants into which the mutant HPPDs (not the combinatorial mutation F372A (F372G/F372V)+F383W) were introduced**

| Source of the genes and sequences to be verified | *Arabidopsis thaliana* genotypes | Concentration (g ai/ha) | Classification and statistics of the grade of pesticide damage | | | | Scores | Resistance evaluation |
|---|---|---|---|---|---|---|---|---|
| | | | Grade 0 | Grade 1 | Grade 2 | Grade 3 | | |
| | CK | 0 | 16 | 0 | 0 | 0 | 0 | |
| | | 25 | 0 | 0 | 0 | 16 | 100 | Non-resistant |
| | | 100 | 0 | 0 | 0 | 16 | 100 | Non-resistant |
| | AtHPPD-02 | 0 | 16 | 0 | 0 | 0 | 0 | |
| | | 25 | 0 | 0 | 0 | 16 | 100 | Non-resistant |
| | | 100 | 0 | 0 | 0 | 16 | 100 | Non-resistant |
| | AtHPPDm-F372A-02 | 0 | 16 | 0 | 0 | 0 | 0 | |
| | | 25 | 4 | 4 | 8 | 0 | 42 | Poorly resistant |
| | | 100 | 0 | 0 | 4 | 12 | 92 | Non-resistant |
| *Arabidopsis thaliana* (F372A+F415W) | AtHPPDm-F415W-02 | 0 | 16 | 0 | 0 | 0 | 0 | |
| | | 25 | 0 | 0 | 0 | 16 | 100 | Non-resistant |
| | | 100 | 0 | 0 | 0 | 16 | 100 | Non-resistant |
| | AtHPPDm-F372A-F415W-02 | 0 | 16 | 0 | 0 | 0 | 0 | |
| | | 25 | 4 | 4 | 7 | 1 | 44 | Poorly resistant |
| | | 100 | 0 | 0 | 3 | 13 | 94 | Non-resistant |
| *Arabidopsis thaliana* (F372A+F383Y) | AtHPPDm-F383Y-02 | 0 | 16 | 0 | 0 | 0 | 0 | |
| | | 25 | 0 | 0 | 0 | 16 | 100 | Non-resistant |
| | | 100 | 0 | 0 | 0 | 16 | 100 | Non-resistant |
| | AtHPPDm-F372A-F383Y-02 | 0 | 16 | 0 | 0 | 0 | 0 | |
| | | 25 | 4 | 4 | 8 | 0 | 42 | Poorly resistant |
| | | 100 | 0 | 0 | 4 | 12 | 92 | Non-resistant |

**TABLE 14 Isoxazole tolerance of Arabidopsis thaliana T₁ plants into which the mutant HPPDs (not the combinatorial mutation F372A (F372G/F372V)+F383W) were introduced**

| Source of the genes and sequences to be verified | *Arabidopsis thaliana* genotypes | Concentration (g ai/ha) | Classification and statistics of the grade of pesticide damage | | | | Scores | Resistance evaluation |
|---|---|---|---|---|---|---|---|---|
| | | | Grade 0 | Grade 1 | Grade 2 | Grade 3 | | |
| | CK | 0 | 16 | 0 | 0 | 0 | 0 | |
| | | 140 | 0 | 0 | 0 | 16 | 100 | Non-resistant |
| | | 280 | 0 | 0 | 0 | 16 | 100 | Non-resistant |
| | AsHPPD-02 | 0 | 16 | 0 | 0 | 0 | 0 | |
| | | 140 | 0 | 1 | 4 | 11 | 88 | Non-resistant |
| | | 280 | 0 | 0 | 0 | 16 | 100 | Non-resistant |
| | AsHPPDm-F372A-02 | 0 | 16 | 0 | 0 | 0 | 0 | |
| | | 140 | 5 | 6 | 5 | 0 | 33 | Moderately resistant |
| | | 280 | 0 | 0 | 16 | 0 | 67 | Poorly resistant |
| *Avena sativa* (F372A+F415W) | AsHPPDm-F415W-02 | 0 | 16 | 0 | 0 | 0 | 0 | |
| | | 140 | 5 | 7 | 4 | 0 | 31 | Moderately resistant |
| | | 280 | 0 | 2 | 14 | 0 | 63 | Poorly resistant |
| | AsHPPDm-F372A-F415W-02 | 0 | 16 | 0 | 0 | 0 | 0 | |
| | | 140 | 5 | 7 | 4 | 0 | 31 | Moderately resistant |
| | | 280 | 0 | 3 | 12 | 1 | 63 | Poorly resistant |
| *Avena sativa* (F372A+F383Y) | AsHPPDm-F383Y-02 | 0 | 16 | 0 | 0 | 0 | 0 | |
| | | 140 | 0 | 0 | 0 | 16 | 100 | Non-resistant |
| | | 280 | 0 | 0 | 0 | 16 | 100 | Non-resistant |
| | AsHPPDm-F372A-F383Y-02 | 0 | 16 | 0 | 0 | 0 | 0 | |
| | | 140 | 5 | 6 | 5 | 0 | 33 | Moderately resistant |
| | | 280 | 0 | 0 | 16 | 0 | 67 | Poorly resistant |
| | AtHPPD-02 | 0 | 16 | 0 | 0 | 0 | 0 | |
| | | 35 | 0 | 0 | 0 | 16 | 100 | Non-resistant |
| | | 70 | 0 | 0 | 0 | 16 | 100 | Non-resistant |
| | AtHPPDm-F372A-02 | 0 | 16 | 0 | 0 | 0 | 0 | |
| | | 35 | 0 | 9 | 5 | 2 | 52 | Poorly resistant |
| | | 70 | 0 | 0 | 2 | 14 | 96 | Non-resistant |
| *Arabidopsis thaliana* (F372A+F415W) | AtHPPDm-F415W-02 | 0 | 16 | 0 | 0 | 0 | 0 | |
| | | 35 | 0 | 0 | 0 | 16 | 100 | Non-resistant |
| | | 70 | 0 | 0 | 0 | 16 | 100 | Non-resistant |
| | AtHPPDm-F372A-F415W-02 | 0 | 16 | 0 | 0 | 0 | 0 | |
| | | 35 | 0 | 9 | 4 | 3 | 54 | Poorly resistant |
| | | 70 | 0 | 0 | 0 | 16 | 100 | Non-resistant |
| *Arabidopsis thaliana* (F372A+F383Y) | AtHPPDm-F383Y-02 | 0 | 16 | 0 | 0 | 0 | 0 | |
| | | 35 | 0 | 0 | 0 | 16 | 100 | Non-resistant |
| | | 70 | 0 | 0 | 0 | 16 | 100 | Non-resistant |
| | AtHPPDm-F372A-F383Y-02 | 0 | 16 | 0 | 0 | 0 | 0 | |
| | | 35 | 0 | 9 | 3 | 4 | 56 | Poorly resistant |
| | | 70 | 0 | 0 | 0 | 16 | 100 | Non-resistant |

**TABLE 15 Mesotrione tolerance of Arabidopsis thaliana T₁ plants into which the mutant HPPDs (not the combinatorial mutation F372A (F372G/F372V)+F383W) were introduced**

| Source of the genes and sequences to be verified | *Arabidopsis thaliana* genotypes | Concentration (g ai/ha) | Classification and statistics of the grade of pesticide damage | | | | Scores | Resistance evaluation |
|---|---|---|---|---|---|---|---|---|
| | | | Grade 0 | Grade 1 | Grade 2 | Grade 3 | | |
| | CK | 0 | 16 | 0 | 0 | 0 | 0 | |
| | | 210 | 0 | 0 | 0 | 16 | 100 | Non-resistant |
| | | 420 | 0 | 0 | 0 | 16 | 100 | Non-resistant |
| | AsHPPD-02 | 0 | 16 | 0 | 0 | 0 | 0 | |
| | | 210 | 0 | 0 | 0 | 16 | 100 | Non-resistant |
| | | 420 | 0 | 0 | 0 | 16 | 100 | Non-resistant |
| | AsHPPDm-F372A-02 | 0 | 16 | 0 | 0 | 0 | 0 | |
| | | 210 | 5 | 7 | 4 | 0 | 31 | Moderately resistant |
| | | 420 | 0 | 8 | 8 | 0 | 50 | Poorly resistant |
| *Avena sativa* (F372A+F415W) | AsHPPDm-F415W-02 | 0 | 16 | 0 | 0 | 0 | 0 | |
| | | 210 | 0 | 0 | 9 | 7 | 81 | Non-resistant |
| | | 420 | 0 | 0 | 0 | 16 | 100 | Non-resistant |
| | AsHPPDm-F372A-F415W-02 | 0 | 16 | 0 | 0 | 0 | 0 | |
| | | 210 | 6 | 6 | 3 | 1 | 31 | Moderately resistant |
| | | 420 | 0 | 8 | 8 | 0 | 50 | Poorly resistant |
| *Avena sativa* (F372A+F383Y) | AsHPPDm-F383Y-02 | 0 | 16 | 0 | 0 | 0 | 0 | |
| | | 210 | 0 | 0 | 0 | 16 | 100 | Non-resistant |
| | | 420 | 0 | 0 | 0 | 16 | 100 | Non-resistant |
| | AsHPPDm-F372A-F383Y-02 | 0 | 16 | 0 | 0 | 0 | 0 | |
| | | 210 | 5 | 6 | 5 | 0 | 33 | Moderately resistant |
| | | 420 | 0 | 8 | 7 | 1 | 52 | Poorly resistant |
| | AtHPPD-02 | 0 | 16 | 0 | 0 | 0 | 0 | |
| | | 52.5 | 0 | 0 | 0 | 16 | 100 | Non-resistant |
| | | 105 | 0 | 0 | 0 | 16 | 100 | Non-resistant |
| | AtHPPDm-F372A-02 | 0 | 16 | 0 | 0 | 0 | 0 | |
| | | 52.5 | 0 | 5 | 8 | 3 | 63 | Poorly resistant |
| | | 105 | 0 | 0 | 0 | 16 | 100 | Non-resistant |
| *Arabidopsis thaliana* (F372A+F415W) | AtHPPDm-F415W-02 | 0 | 16 | 0 | 0 | 0 | 0 | |
| | | 52.5 | 0 | 0 | 0 | 16 | 100 | Non-resistant |
| | | 105 | 0 | 0 | 0 | 16 | 100 | Non-resistant |
| | AtHPPDm-F372A-F415W-02 | 0 | 16 | 0 | 0 | 0 | 0 | |
| | | 52.5 | 0 | 4 | 9 | 3 | 65 | Poorly resistant |
| | | 105 | 0 | 0 | 0 | 16 | 100 | Non-resistant |
| *Arabidopsis thaliana* (F372A+F383Y) | AtHPPDm-F383Y-02 | 0 | 16 | 0 | 0 | 0 | 0 | |
| | | 52.5 | 0 | 0 | 0 | 16 | 100 | Non-resistant |
| | | 105 | 0 | 0 | 0 | 16 | 100 | Non-resistant |
| | AtHPPDm-F372A-F383Y-02 | 0 | 16 | 0 | 0 | 0 | 0 | |
| | | 52.5 | 0 | 5 | 8 | 3 | 63 | Poorly resistant |
| | | 105 | 0 | 0 | 0 | 16 | 100 | Non-resistant |

The results of TABLE 13 to TABLE 15 show that the *Arabidopsis thaliana* T₁ plants into which the HPPD genes with a combinatorial mutation F372A+F415W or F372A+F383Y were introduced and the *Arabidopsis thaliana* T₁ plants into which the HPPD genes with the single position mutation F372A were introduced did not showed substantially different tolerance to HPPD-inhibitor herbicides. It can thus be seen that not any combinatorial mutation at any two positions of the HPPD amino acid sequence can confer a synergistically enhanced tolerance to HPPD-inhibitor herbicides upon plants, and it also shows that the synergistically enhanced technical effect produced by the mutations at positions 372+383 of the HPPD amino acid sequence of the present invention is unexpected.

### Example 6: Acquisition and verification of transgenic soybean plants

### 1. Transformation of Agrobacterium with the recombinant expression vectors

The recombinant expression vectors DBN11758 containing the SbHPPD-02 nucleotide sequence, the recombinant expression vector DBN11759 containing the SbHPPDm-F372A-02 nucleotide sequence, the recombinant expression vector DBN11760 containing the SbHPPDm-F383W-02 nucleotide sequence, and the recombinant expression vector DBN11761 containing the SbHPPDm-F372A-F383W-02 nucleotide sequence in point 2 of Example 2, and the control recombinant expression vectors DBN11726N in point 3 of Example 1 were transformed into the *Agrobacterium* LBA4404 (Invitrogen, Chicago, USA, CAT: 18313-015) respectively using a liquid nitrogen method, under the following transformation conditions: 100 µL of *Agrobacterium* LBA4404, and 3 µL of plasmid DNA (recombinant expression vector) were placed in liquid nitrogen for 10 minutes, and bathed in warm water at 37°C for 10 minutes; the transformed *Agrobacterium* LBA4404 were inoculated into an LB tube, cultured under the conditions of a temperature of 28°C and a rotation speed of 200 rpm for 2 hours, and then spread on the LB plate containing 50 mg/L of rifampicin and 50 mg/L of spectinomycin until positive single clones were grown, and single clones were picked out for culturing and the plasmids thereof were extracted. The extracted plasmids were identified by sequencing. The results showed that the structures of the recombinant expression vectors DBN11758, DBN11759, DBN11760, DBN11761 and DBN11726N were completely correct.

### 2. Acquisition of transgenic soybean plants

According to the conventional *Agrobacterium* infection method, the cotyledonary node tissue of a sterilely cultured soybean variety Zhonghuang13 was co-cultured with the *Agrobacterium* as described in point 1 of this Example, so as to introduce the T-DNA (including the figwort mosaic virus 34S enhancer sequence, the oilseed rape eukaryotic elongation factor gene 1α (Tsf1) promoter sequence, the *Arabidopsis thaliana* chloroplast transit peptide sequence, a 5-enolpyruvylshikimate-3-phosphate synthase gene, the pea RbcS gene terminator sequence, the *Arabidopsis thaliana* Ubiquitin10 gene promoter sequence, SbHPPD-02 nucleotide sequence, SbHPPDm-F372A-02 nucleotide sequence, SbHPPDm-F383W-02 nucleotide sequence, SbHPPDm-F372A-F383W-02 nucleotide sequence, the nopaline synthetase gene terminator sequence, the cauliflower mosaic virus 35S promoter sequence, phosphinothricin-N-acetyl-transferase gene, and the cauliflower mosaic virus 35S terminator sequence) of the recombinant expression vectors DBN11758, DBN11759, DBN11760, DBN11761 and DBN11726N into the soybean chromosomes, thereby obtaining soybean plants into which the SbHPPD-02 nucleotide sequence was introduced, soybean plants into which the SbHPPDm-F372A-02 nucleotide sequence was introduced, soybean plants into which the SbHPPDm-F383W-02 nucleotide sequence was introduced, soybean plants into which the SbHPPDm-F372A-F383W-02 nucleotide sequence was introduced, and soybean plants into which the control vector DBN11726N was introduced.

For the *Agrobacterium-mediated* soybean transformation, briefly, mature soybean seeds were germinated in a soybean germination culture medium (3.1 g/L of B5 salt, B5 vitamin, 20 g/L of sucrose, and 8 g/L of agar, pH 5.6), and then cultured under the conditions of a temperature of 25 ± 1°C; and a photoperiod (light/dark) of 16 h/8 h. After 4-6 days of germination, soybean sterile seedlings swelling at bright green cotyledonary nodes were taken, hypocotyledonary axes were cut off 3-4 millimeters below the cotyledonary nodes, the cotyledons were cut longitudinally, and apical buds, lateral buds and seminal roots were removed. A wound was created at a cotyledonary node using the knife back of a scalpel, and the wounded cotyledonary node tissues were contacted with an *Agrobacterium* suspension, wherein the *Agrobacterium* can transfer the SbHPPD-02 nucleotide sequence, SbHPPDm-F372A-02 nucleotide sequence, SbHPPDm-F383W-02 nucleotide sequence, or SbHPPDm-F372A-F383W-02 nucleotide sequence to the wounded cotyledonary node tissues (step 1: the infection step). In this step, the cotyledonary node tissues were preferably immersed in the *Agrobacterium* suspension (OD₆₆₀ = 0.5-0.8, an infection culture medium (2.15 g/L of MS salt, B5 vitamin, 20 g/L of sucrose, 10 g/L of glucose, 40 mg/L of acetosyringone (AS), 4 g/L of 2-morpholine ethanesulfonic acid (MES), and 2 mg/L of zeatin (ZT), pH 5.3)) to initiate the inoculation. The cotyledonary node tissues were co-cultured with *Agrobacterium* for a period of time (3 days) (step 2: the co-culturing step). Preferably, after the infection step, the cotyledonary node tissues were cultured in a solid culture medium (4.3 g/L of MS salt, B5 vitamin, 20 g/L of sucrose, 10 g/L of glucose, 4 g/L of MES, 2 mg/L of ZT, and 8 g/L of agar, pH 5.6). After this co-culturing stage, there can be an optional "recovery" step in which a recovery culture medium (3.1 g/L of B5 salt, B5 vitamin, 1 g/L of MES, 30 g/L of sucrose, 2 mg/L of ZT, 8 g/L of agar, 150 mg/L of cephalosporin, 100 mg/L of glutamic acid, and 100 mg/L of aspartic acid, pH 5.6) with the addition of at least one antibiotic (150-250 mg/L of cephalosporin) for inhibiting the growth of *Agrobacterium,* and without the addition of a selective agent for a plant transformant, was used (step 3: the recovery step). Preferably, tissue blocks regenerated from the cotyledonary nodes were cultured in a solid culture medium containing the antibiotic and no selective agent, to eliminate *Agrobacterium* and provide a recovery stage for the infected cells. Subsequently, the tissue blocks regenerated from the cotyledonary nodes were cultured in a culture medium containing a selective agent (glyphosate), and on-growing transformed calli were selected (step 4: the selection step). Preferably, the tissue blocks regenerated from the cotyledonary nodes were cultured in a screening solid culture medium (3.1 g/L of B5 salt, B5 vitamin, 1 g/L of MES, 30 g/L of sucrose, 1 mg/L of 6-benzyladenine (6-BAP), 8 g/L of agar, 150 mg/L of cephalosporin, 100 mg/L of glutamic acid, 100 mg/L of aspartic acid, and 0.25 mol/L of N-(phosphonomethyl)glycine, pH 5.6) containing a selective agent, thus resulting in selective growth of the transformed cells. Then, plants were regenerated from the transformed cells (step 5: the regeneration step). Preferably, the tissue blocks regenerated from the cotyledonary nodes grown in a culture medium containing a selective agent were cultured in solid culture media (a B5 differentiation culture medium and B5 rooting culture medium) to regenerate plants.

The screened out resistant tissues were transferred onto the B5 differentiation culture medium (3.1 g/L of B5 salt, B5 vitamin, 1 g/L of MES, 30 g/L of sucrose, 1 mg/L of ZT, 8 g/L of agar, 150 mg/L of cephalosporin, 50 mg/L of glutamic acid, 50 mg/L of aspartic acid, 1 mg/L of gibberellin, 1 mg/L of auxin, and 0.25 mol/L of N-(phosphonomethyl)glycine, pH 5.6), and cultured at 25°C for differentiation. The differentiated seedlings were transferred onto the B5 rooting culture medium (3.1 g/L of B5 salt, B5 vitamin, 1 g/L of MES, 30 g/L of sucrose, 8 g/L of agar, 150 mg/L of cephalosporin, and 1 mg/L of indole-3-butyric acid (IBA)), cultured in the rooting culture medium at 25°C until a height of about 10 cm, and then transferred to a glasshouse until fruiting. In the greenhouse, the plants were cultured at 26°C for 16 hours, and then cultured at 20°C for 8 hours per day.

The soybean T₀ plants into which the SbHPPD-02 nucleotide sequence was introduced, soybean T₀ plants into which the SbHPPDm-F372A-02 nucleotide sequence was introduced, soybean T₀ plants into which the SbHPPDm-F383W-02 nucleotide sequence was introduced, soybean T₀ plants into which the SbHPPDm-F372A-F383W-02 nucleotide sequence was introduced, and soybean T₀ plants into which the control vector DBN11726N was introduced were transplanted into the greenhouse for cultivation and propagation to obtain corresponding transgenic T₁ plants.

### 3. Verification of the transgenic soybean plants using TaqMan

About 100 mg of leaves from the soybean T₁ plants into which the SbHPPD-02 nucleotide sequence was introduced, soybean T₁ plants into which the SbHPPDm-F372A-02 nucleotide sequence was introduced, soybean T₁ plants into which the SbHPPDm-F383W-02 nucleotide sequence was introduced, soybean T₁ plants into which the SbHPPDm-F372A-F383W-02 nucleotide sequence was introduced, and soybean T₁ plants into which the control vector DBN11726N was introduced, were taken as samples, and the genomic DNA thereof was extracted with a DNeasy Plant Maxi Kit of Qiagen, and copy numbers of an *EPSPS* gene were detected by the Taqman probe fluorescence quantitative PCR method so as to determine the copy numbers of the mutant HPPD gene. At the same time, wild-type soybean plants were used as controls, and detected and analyzed according to the above-mentioned method. Triple repeats were set for the experiments, and were averaged.

The specific method for detecting the copy number of the *EPSPS* gene was as follows:
Step 11: 100 mg of leaves of the soybean T₁ plants into which the SbHPPD-02 nucleotide sequence was introduced, soybean T₁ plants into which the SbHPPDm-F372A-02 nucleotide sequence was introduced, soybean T₁ plants into which the SbHPPDm-F383W-02 nucleotide sequence was introduced, soybean T₁ plants into which the SbHPPDm-F372A-F383W-02 nucleotide sequence was introduced, soybean T₁ plants into which the control vector DBN11726N nucleotide sequence was introduced, and wild-type soybean plants were taken, and ground into a homogenate using liquid nitrogen in a mortar, and triple repeats were taken for each sample;
Step 12: The genomic DNA of the above-mentioned samples was extracted using a DNeasy Plant Mini Kit of Qiagen, with the particular method as described in the product manual;
Step 13: The concentrations of the genomic DNA of the above-mentioned samples were detected using NanoDrop 2000 (Thermo Scientific);
Step 14: The concentrations of the genomic DNA of the above-mentioned samples were adjusted to a same value in the range of from 80 to 100 ng/µL;
Step 15: The copy numbers of the samples were identified using the Taqman probe fluorescence quantitative PCR method, wherein samples for which the copy numbers were known and had been identified were taken as standards, the samples of the wild-type soybean plants were taken as the control, and triple repeats were taken for each sample, and were averaged; the sequences of fluorescence quantitative PCR primers and a probe were as follows:
   the following primers and probe were used to detect the *EPSPS* gene sequence:
   primer 1:ctggaaggcgaggacgtcatcaata, as set forth in SEQ ID NO: 232 in the SEQUENCE LISTING;
   primer 2: tggcggcattgccgaaatcgag, as set forth in SEQ ID NO: 233 in the SEQUENCE LISTING;
   probe 1: atgcaggcgatgggcgcccgcatccgta, as set forth in SEQ ID NO: 234 in the SEQUENCE LISTING;

### PCR reaction system:

| | |
|---|---|
| JumpStart^{™} Taq ReadyMix^{™} (Sigma) | 10 µL |
| 50× primer/probe mixture | 1 µL |
| genomic DNA | 3 µL |
| water (ddH₂O) | 6 µL |

The 50× primer/probe mixture comprises 45 µL of each primer at a concentration of 1 mM, 50 µL of the probe at a concentration of 100 µM, and 860 µL of 1× TE buffer, and was stored at 4°C in an amber tube.

### PCR reaction conditions:

| Step | Temperature | Time |
|---|---|---|
| 21 | 95°C | 5 min |
| 22 | 95°C | 30 s |
| 23 | 60°C | 1 min |
| 24 | go back to step 22, and repeat 40 times | |

### Data was analyzed using software SDS2.3 (Applied Biosystems).

By analyzing the experimental results of the copy number of the *EPSPS* gene, it was further demonstrated that the SbHPPD-02 nucleotide sequence, SbHPPDm-F372A-02 nucleotide sequence, SbHPPDm-F383W-02 nucleotide sequence, SbHPPDm-F372A-F383W-02 nucleotide sequence, and the control vector DBN11726N had all been incorporated into the chromosome of the detected soybean plants, and all of the soybean T₁ plants into which the SbHPPD-02 nucleotide sequence was introduced, soybean T₁ plants into which the SbHPPDm-F372A-02 nucleotide sequence was introduced, soybean T₁ plants into which the SbHPPDm-F383W-02 nucleotide sequence was introduced, soybean T₁ plants into which the SbHPPDm-F372A-F383W-02 nucleotide sequence was introduced, soybean T₁ plants into which the control vector DBN11726N was introduced, resulted in single-copy transgenic soybean plants.

### 4. Detection of the herbicide tolerance of the transgenic soybean plants to HPPD-inhibitor herbicides

The soybean T₁ plants into which the SbHPPD-02 nucleotide sequence was introduced, soybean T₁ plants into which the SbHPPDm-F372A-02 nucleotide sequence was introduced, soybean T₁ plants into which the SbHPPDm-F383W-02 nucleotide sequence was introduced, soybean T₁ plants into which the SbHPPDm-F372A-F383W-02 nucleotide sequence was introduced, soybean T₁ plants into which the control vector DBN11726N nucleotide sequence was introduced, and wild-type soybean plants (V3-V4 at seedling stage) were sprayed with topramezone at three different concentrations (i.e., 25 g ai/ha (one-fold field concentration, 1×), 100 g ai/ha (four-fold field concentration, 4×) and 0 g ai/ha (water, 0×)), isoxaflutole at three different concentrations (i.e., 70 g ai/ha (one-fold field concentration, 1×), 280 g ai/ha (four-fold field concentration, 4×) and 0 g ai/ha (water, 0×)), and mesotrione at three different concentrations (i.e., 105 g ai/ha (one-fold field concentration, 1×), 420 g ai/ha (four-fold field concentration, 4×) and 0 g ai/ha (water, 0×)) respectively, to detect the herbicide tolerance of soybean plants. According to the method in point 6 of Example 1, after 7 days of spraying (7 DAT), the damage degree of each plant by the herbicide was statistically analyzed, and the scoring and resistance evaluation were carried out accordingly. The soybean plants into which the SbHPPD-02 nucleotide sequence was introduced were of two strains in total (S1 and S2), the soybean plants into which the SbHPPDm-F372A-02 nucleotide sequence was introduced were of two strains in total (S3 and S4), the soybean plants into which the SbHPPDm-F383W-02 nucleotide sequence was introduced were of two strains in total (S5 and S6), the soybean plants into which the SbHPPDm-F372A-F383W-02 nucleotide sequence was introduced were of two strains in total (S7 and S8), and the soybean plants into which the control vector DBN11726N was introduced were of one strain in total (S9), and the wild-type soybean plants were of one strain in total (CK1); and 8 plants were selected from each strain and detected. The results were shown in TABLE 16 to TABLE 18.

**TABLE 16 Topramezone tolerance of transgenic soybean T₁ plants**

| Source of the gene | Strains | Concentrati on (g ai/ha) | Classification and statistics of the grade of pesticide damage | | | | Scores | Resistance evaluation |
|---|---|---|---|---|---|---|---|---|
| | | | Grad e 0 | Grad e 1 | Grad e2 | Grad e 3 | | |
| | CK1 | 25 | 0 | 0 | 0 | 8 | 100 | Non-resistant |
| | | 100 | 0 | 0 | 0 | 8 | 100 | Non-resistant |
| DBN11726N | S9 | 25 | 0 | 0 | 0 | 8 | 100 | Non-resistant |
| | | 100 | 0 | 0 | 0 | 8 | 100 | Non-resistant |
| SbHPPD-02 | S1 | 25 | 0 | 0 | 0 | 8 | 100 | Non-resistant |
| | | 100 | 0 | 0 | 0 | 8 | 100 | Non-resistant |
| | S2 | 25 | 0 | 0 | 0 | 8 | 100 | Non-resistant |
| | | 100 | 0 | 0 | 0 | 8 | 100 | Non-resistant |
| SbHPPDm-F372A-02 | S3 | 25 | 8 | 0 | 0 | 0 | 0 | Highly resistant |
| | | 100 | 0 | 7 | 1 | 0 | 38 | Poorly resistant |
| | S4 | 25 | 8 | 0 | 0 | 0 | 0 | Highly resistant |
| | | 100 | 0 | 6 | 2 | 0 | 42 | Poorly resistant |
| SbHPPDm-F383W-02 | S5 | 25 | 4 | 2 | 2 | 0 | 25 | Moderately resistant |
| | | 100 | 0 | 2 | 3 | 3 | 71 | Non-resistant |
| | S6 | 25 | 4 | 2 | 2 | 0 | 25 | Moderately resistant |
| | | 100 | 0 | 2 | 2 | 4 | 75 | Non-resistant |
| SbHPPDm-F372A-F383W-02 | S7 | 25 | 8 | 0 | 0 | 0 | 0 | Highly resistant |
| | | 100 | 5 | 3 | 0 | 0 | 13 | Highly resistant |
| | S8 | 25 | 8 | 0 | 0 | 0 | 0 | Highly resistant |
| | | 100 | 6 | 1 | 1 | 0 | 13 | Highly resistant |

**TABLE 17 Isoxaflutole tolerance of transgenic soybean T₁ plants**

| Source of the gene | Strains | Concentrati on (g ai/ha) | Classification and statistics of the grade of pesticide damage | | | | Scores | Resistance evaluation |
|---|---|---|---|---|---|---|---|---|
| | | | Grad e 0 | Grad e 1 | Grad e2 | Grad e 3 | | |
| | CK1 | 70 | 0 | 0 | 0 | 8 | 100 | Non-resistant |
| | | 280 | 0 | 0 | 0 | 8 | 100 | Non-resistant |
| DBN11726N | S9 | 70 | 0 | 0 | 0 | 8 | 100 | Non-resistant |
| | | 280 | 0 | 0 | 0 | 8 | 100 | Non-resistant |
| SbHPPD-02 | S1 | 70 | 0 | 0 | 0 | 8 | 100 | Non-resistant |
| | | 280 | 0 | 0 | 0 | 8 | 100 | Non-resistant |
| | S2 | 70 | 0 | 0 | 0 | 8 | 100 | Non-resistant |
| | | 280 | 0 | 0 | 0 | 8 | 100 | Non-resistant |
| SbHPPDm-F372A-02 | S3 | 70 | 0 | 4 | 2 | 2 | 58 | Poorly resistant |
| | | 280 | 0 | 2 | 3 | 3 | 71 | Non-resistant |
| | S4 | 70 | 0 | 4 | 1 | 3 | 63 | Poorly resistant |
| | | 280 | 0 | 1 | 4 | 3 | 75 | Non-resistant |
| SbHPPDm-F383W-02 | S5 | 70 | 0 | 6 | 2 | 0 | 42 | Poorly resistant |
| | | 280 | 0 | 0 | 6 | 2 | 75 | Non-resistant |
| | S6 | 70 | 0 | 6 | 1 | 1 | 46 | Poorly resistant |
| | | 280 | 0 | 0 | 5 | 3 | 79 | Non-resistant |
| SbHPPDm-F372A-F383W-02 | S7 | 70 | 7 | 1 | 0 | 0 | 4 | Highly resistant |
| | | 280 | 4 | 4 | 0 | 0 | 17 | Moderately resistant |
| | S8 | 70 | 7 | 1 | 0 | 0 | 4 | Highly resistant |
| | | 280 | 4 | 3 | 1 | 0 | 21 | Moderately resistant |

**TABLE 18 Mesotrione tolerance of transgenic soybean T₁ plants**

| Source of the gene | Strains | Concentrati on (g ai/ha) | Classification and statistics of the grade of pesticide damage | | | | Scores | Resistance evaluation |
|---|---|---|---|---|---|---|---|---|
| | | | Grad e 0 | Grad e 1 | Grad e2 | Grad e3 | | |
| | CK1 | 105 | 0 | 0 | 0 | 8 | 100 | Non-resistant |
| | | 420 | 0 | 0 | 0 | 8 | 100 | Non-resistant |
| DBN11726N | S9 | 105 | 0 | 0 | 0 | 8 | 100 | Non-resistant |
| | | 420 | 0 | 0 | 0 | 8 | 100 | Non-resistant |
| SbHPPD-02 | S1 | 105 | 0 | 0 | 0 | 8 | 100 | Non-resistant |
| | | 420 | 0 | 0 | 0 | 8 | 100 | Non-resistant |
| | S2 | 105 | 0 | 0 | 0 | 8 | 100 | Non-resistant |
| | | 420 | 0 | 0 | 0 | 8 | 100 | Non-resistant |
| SbHPPDm-F372A-02 | S3 | 105 | 0 | 7 | 1 | 0 | 38 | Poorly resistant |
| | | 420 | 0 | 2 | 3 | 3 | 71 | Non-resistant |
| | S4 | 105 | 0 | 7 | 1 | 0 | 38 | Poorly resistant |
| | | 420 | 0 | 2 | 3 | 3 | 71 | Non-resistant |
| SbHPPDm-F383W-02 | S5 | 105 | 0 | 0 | 7 | 1 | 71 | Non-resistant |
| | | 420 | 0 | 0 | 4 | 4 | 83 | Non-resistant |
| | S6 | 105 | 0 | 0 | 7 | 1 | 71 | Non-resistant |
| | | 420 | 0 | 0 | 4 | 4 | 83 | Non-resistant |
| SbHPPDm-F372A-F383W-02 | S7 | 105 | 5 | 3 | 0 | 0 | 13 | Highly resistant |
| | | 420 | 0 | 3 | 4 | 1 | 58 | Poorly resistant |
| | S8 | 105 | 5 | 3 | 0 | 0 | 13 | Highly resistant |
| | | 420 | 0 | 5 | 1 | 2 | 54 | Poorly resistant |

The results in TABLE 16 to TABLE 18 shows that (1) as compared with the soybean plants into which unmutated HPPD genes were introduced and the wild-type soybean plants, the soybean plants SbHPPDm-F372A-02, SbHPPDm-F383W-02 and SbHPPDm-F372A-F383W-02 were able to produce different degrees of tolerance to HPPD-inhibitor herbicides at different concentrations, while DBN11726N had no tolerance to HPPD-inhibitor herbicides; (2) the soybean plants into which the HPPD genes with a combinatorial mutation at positions 372 and 383 (F372A+F383W) exhibited better herbicide tolerance than the soybean plants into which the HPPD genes with a single position mutation F372A or F383W were introduced, and further achieved a synergistically enhanced effect of herbicide tolerance. This indicates that the mutated HPPD (F372A+F383W) can confer synergistically enhanced tolerance to HPPD-inhibitor herbicides upon transgenic soybeans plants, and it further shows that the importance and stability of the herbicide tolerance to HPPD-inhibitor herbicides of the plants conferred by the combinatorial mutation at positions 372 and 383 of the HPPD amino acid sequence.

In conclusion, the present invention discloses for the first time that the combinatorial mutation at positions 372 and 383 of hydroxyphenyl pyruvate dioxygenase polypeptides from different species can confer synergistically enhanced tolerance to HPPD inhibitor herbicides pyrazolinates, isoxazoles and triketones upon plants, and, in particular, can confer tolerance to topramezone, isoxaflutole or mesotrione at four-fold field concentration upon transgenic soybean plants. Therefore, the present invention has a broad application prospect in plants.

At last, it should be noted that all the above Examples are only used to illustrate the embodiments of the present invention rather than to limit the present invention. Although the present invention is described in detail with reference to the preferred Examples, those skilled in the art should understand that the embodiments of the present invention could be modified or substituted equivalently without departing from the spirit and scope of the technical solutions of the present invention.

## Claims

1. A mutant hydroxyphenylpyruvate dioxygenase polypeptide, which retains the activity of catalyzing the conversion of 4-hydroxyphenylpyruvic acid into homogentisic acid or homogentisate and is less sensitive to an HPPD-inhibitor herbicide than the wild-type HPPD, **characterized by** comprising the following amino acid mutations at the positions corresponding to the positions of the amino acid sequence as set forth in SEQ ID NO: 1: substitutions of F with A, G or V at position 372, and substitution of F with W at position 383;
preferably, the mutant hydroxyphenylpyruvate dioxygenase polypeptide comprises amino acid mutations at the following positions corresponding to those of the amino acid sequence as set forth in SEQ ID NO: 1: substitutionof F with A at position 372, and substitution of F with W at position 383.

2. The mutant hydroxyphenylpyruvate dioxygenase polypeptide according to claim 1, **characterized in that** the mutant hydroxyphenylpyruvate dioxygenase polypeptide further comprises a second mutation;
preferably, the second mutation comprises at least one of the following amino acid mutation at the positions corresponding to the positions of the amino acid sequence as set forth in SEQ ID NO: 1: A106G, A107 deletion, A111T, or K351N.

3. The mutant hydroxyphenylpyruvate dioxygenase polypeptide according to claim 1 or 2, **characterized in that** the mutant hydroxyphenylpyruvate dioxygenase polypeptide comprises a polypeptide having an amino acid sequence as set forth in SEQ ID NO: 173, SEQ ID NO: 182, SEQ ID NO: 185, SEQ ID NO: 188, SEQ ID NO: 191, SEQ ID NO: 194, SEQ ID NO: 197, SEQ ID NO: 200, or SEQ ID NO: 203.

4. The mutant hydroxyphenylpyruvate dioxygenase polypeptide according to any of claims 1-3, **characterized in that** the mutant hydroxyphenylpyruvate dioxygenase polypeptide is derived from wild-type HPPDs in plants or microorganisms.

5. A polynucleotide encoding the mutant hydroxyphenylpyruvate dioxygenase polypeptide according to any of claims 1-4.

6. An expression cassette or a recombinant vector, **characterized by** comprising the polynucleotide according to claim 5 under the regulation of effectively-linked regulatory sequences.

7. A method for expanding the scope of herbicides to which the plants are tolerant, **characterized by** comprising expressing the mutant hydroxyphenylpyruvate dioxygenase polypeptide according to any of claims 1-4 together with at least one herbicide-tolerant protein other than the mutant hydroxyphenylpyruvate dioxygenase polypeptide according to any of claims 1-4.

8. The method for expanding the scope of herbicides to which the plants are tolerant according to claim 7, **characterized in that** the herbicide-tolerant protein is 5-enolpyruvylshikimate-3-phosphate synthase (EPSPS), glyphosate oxidoreductase, glyphosate-N-acetyltransferase, glyphosate decarboxylase, glufosinate acetyltransferase, alpha-ketoglutarate-dependent dioxygenase, dicamba monooxygenase, acetolactate synthase, cytochrome-like protein and/or protoporphyrinogen oxidase.

9. A method for selecting transformed plant cells, **characterized by** comprising transforming a plurality of plant cells with the polynucleotide according to claim 5, and cultivating the cells under a concentration of the HPPD-inhibitor herbicide that allows the growth of the transformed cells expressing the polynucleotide, while killing the untransformed cells or inhibiting the growth of the untransformed cells;
preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of isoxazoles is isoxaflutole, and the HPPD-inhibitor herbicide of triketones is mesotrione.

10. A method for controlling weeds, **characterized by** comprising applying an effective dose of the HPPD-inhibitor herbicide to a field planting with a target plant, wherein the target plant contains the polynucleotide according to claim 5;
preferably, the target plant is glyphosate-tolerant plant, and the weeds are glyphosate-resistant weeds;
preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of isoxazoles is isoxaflutole, and the HPPD-inhibitor herbicide of triketones is mesotrione.

11. A method for protecting a plant from damages caused by an HPPD-inhibitor herbicide or for conferring tolerance to HPPD-inhibitor herbicide upon a plant, **characterized by** comprising introducing the polynucleotide according to claim 5 or the expression cassette or the recombinant vector according to claim 6 into a plant to make the post-introduction plant produce a sufficient amount of the mutant hydroxyphenylpyruvate dioxygenase polypeptide
to protect the plant from damages caused by the HPPD-inhibitor herbicide;
preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of isoxazoles is isoxaflutole, and the HPPD-inhibitor herbicide of triketones is mesotrione.

12. A method for producing a plant which is tolerant to an HPPD-inhibitor herbicide, **characterized by** comprising introducing the polynucleotide according to claim 5 into the genome of the plant;
preferably, the introduction method comprises genetic transformation, genome editing or gene mutation methods.
preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of isoxazoles is isoxaflutole, and the HPPD-inhibitor herbicide of triketones is mesotrione.

13. A method for cultivating a plant which is tolerant to an HPPD-inhibitor herbicide, **characterized by** comprising:
planting at least one plant propagule, the genome of which contains the polynucleotide according to claim 5; growing the plant propagule into a plant; and
applying an effective dose of the HPPD-inhibitor herbicide to a plant growing environment comprising at least the plant, and harvesting the plant having a reduced plant damage and/or increased plant yield compared to other plants without the polynucleotide according to claim 5;
preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of isoxazoles is isoxaflutole, and the HPPD-inhibitor herbicide of triketones is mesotrione.

14. A method for obtaining a processed agricultural product, **characterized by** comprising treating harvested product of the HPPD-inhibitor herbicide-tolerant plant obtained by the method according to claim 13 to obtain the processed agricultural product.

15. A planting system for controlling the growth of weeds, **characterized by** comprising an HPPD-inhibitor herbicide and a plant growing environment in which at least one target plant is present, wherein the target plant contains the polynucleotide according to claim 5;
preferably, the target plant is glyphosate-tolerant plant, and the weeds are glyphosate-resistant weeds;
preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of isoxazoles is isoxaflutole, and the HPPD-inhibitor herbicide of triketones is mesotrione.

16. Use of the mutant hydroxyphenylpyruvate dioxygenase polypeptide according to any of claims 1-4 for conferring tolerance to an HPPD-inhibitor herbicide upon a plant;
preferably, the HPPD-inhibitor herbicide comprises an HPPD-inhibitor herbicide from the class of pyrazolinates, triketones and/or isoxazoles; more preferably, the HPPD-inhibitor herbicide of pyrazolinates is topramezone, the HPPD-inhibitor herbicide of isoxazoles is isoxaflutole, and the HPPD-inhibitor herbicide of triketones is mesotrione.
